(19) Europäisches Patentamt
European Patent Office
Office européen des brevets

(11) EP 1 402 887 A1

# (12) EUROPEAN PATENT APPLICATION

(43) Date of publication:
31.03.2004 Bulletin 2004/14

(51) Int Cl.[7]: **A61K 31/17**, A61P 43/00

(21) Application number: **02020922.7**

(22) Date of filing: **18.09.2002**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(71) Applicant: **Jerini AG**
**10115 Berlin (DE)**

(72) Inventors:
- **Knolle, Jochen**
**10115 Berlin (DE)**
- **Schutkowski, Mike**
**06268 Ziegelroda (DE)**
- **Hummel, Gerd**
**13357 Berlin (DE)**

(74) Representative: **Bohmann, Armin K., Dr.**
**Bohmann & Loosen,**
**Anwaltssozietät,**
**Sonnenstrasse 8**
**80331 München (DE)**

## (54) New compounds for the inhibition of undesired cell proliferation and use thereof

(57) The present invention is related to the use of a compound for the manufacture of a medicament for the treatment of a disease, whereby the disease involves an abnormal cell proliferation, an undesired cell proliferation, an abnormal mitosis and/or an undesired mitosis.
whereby the compound has the structure:

$$A\text{-}X\text{-}Y \quad (I)$$

wherein A is cycloalkyl, substituted cycloalkyl, heterocyclyl, substituted heterocyclyl, aryl, substituted aryl, heteroaryl or substituted heteroaryl;
X is a spacer and is selected from $X_1$ or $X_2$,
wherein $X_1$ is selected from the group comprising $-[(CR^aR^b)_n-NR^c-CO-NR^d-(CR^aR^b)_m]_t-$, $[(CR^aR^b)_n-NR^c-CS-NR^d-(CR^aR^b)_m]_t-$, $-[(CR^aR^b)_n-NR^c-C(N-CN)-NR^d-(CR^aR^b)_m]_t-$, $-[(CR^aR^b)_n-NR^c-C(N-R^e)-NR^d-(CR^aR^b)_m]_t-$, $-[(CR^aR^b)_n-CO-NR^c-(CR^aR^b)_m]_t-$, $-[(CR^aR^b)_n-NR^c-(CR^aR^b)_m]_t-$, $-[(CR^aR^b)_n-NR^c-CO-O-(CR^aR^b)_m]_t-$, $-[(CR^aR^b)_n-O-CO-NR^c-(CR^aR^b)_m]_t-$, $-[(CR^aR^b)_n-O-(CR^aR^b)_m]_t-$, $-[(CR^aR^b)_n-SR^c-(CR^aR^b)_m]_t-$;
wherein $X_2$ is selected from the group comprising $-[(CR^aR^b)_n-CO-(CR^aR^b)_m]_t-$, $-[(CR^aR^b)_n-CS-(CR^aR^b)_m]_t-$, $-[(CR^aR^b)_n-NR^c-CO-(CR^aR^b)_m]_t-$, $-[(CR^aR^b)_n-CO-NR^c-(CR^aR^b)_m]_t-$, $-[(CR^aR^b)_n-NR^c-CS-(CR^aR^b)_m]_t-$, $-[(CR^aR^b)_n-CS-NR^c-(CR^aR^b)_m]_t-$, $-[(CR^aR^b)_n-NR^c-SO_2-(CR^aR^b)_m]_t-$, $-[(CR^aR^b)_n-SO_2-NR^c-(CR^aR^b)_m]_t-$, $-[(CR^aR^b)_n-SO-(CR^aR^b)_m]_t-$, $-[(CR^aR^b)_n-SO_2-(CR^aR^b)_m]_t-$, $-[(CR^aR^b)_n]_t-$;
wherein n and m are independently selected from each other and are any integer between 0 and 10 provided that if n is 0, m is different from 0, and if m is 0, n is different from 0;
wherein t is independently selected from n and/or m and is any integer between 0 and 10;
wherein
$R^a$, $R^b$, $R^c$, $R^d$ and $R^e$ are independently from each other selected from the group comprising H, alkyl, cycloalkyl, aryl, heteroaryl and heterocyclyl;
and wherein Y is selected from the group comprising alkyl, substituted alkyl, cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, aryl, substituted aryl, heteroaryl and substituted heteroaryl, wherein Y is different from a peptide.

EP 1 402 887 A1

## Description

The present invention is related to new compounds and the use of said compounds for the manufacture of medicaments.

Cell proliferation is a prerequisite for any form of life which is based on cells. Cell proliferation, i. e. increasing the cell number starting from a limited number of cells, is thus relevant for any monocellular and multicellular organism. Cell proliferation as such is a process which is highly regulated by the cell. Cell proliferation under circumstances which are not favourable to support the life of the proliferating cell has to be avoided from the biological point of view. To allow the survival of the cell complex regulation systems including sensoring mechanisms were developed in the evolution of life.

Apart from the mere increase in biomass of monocellular and multicellular organisms, multicellular organisms have to control cell proliferation in order to maintain the highly organized interaction of the cells forming the body of the multicellular organism. Any deregulation of cell proliferation represents or results in a pathological condition. Deregulated cell proliferation is the cause for a number of diseases, including the class of diseases generally referred to as cancer.

Taken the multiplicity of biological processes where cell proliferation has to be controlled, there is a need in the art to provide compounds which are suitable to control cell proliferation. The problem underlying the present invention is thus to provide new compounds which are effective in inhibiting cell proliferation, more particularly undesired cell proliferation.

In a first aspect the problem underlying the present invention is solved by a compound having the structure

$$A-X-Y \qquad (I)$$

wherein A is selected from the group comprising cycloalkyl, substituted cycloalkyl, heterocyclyl, substituted heterocyclyl, aryl, substituted aryl, heteroaryl and substituted heteroaryl;
X is a spacer and is selected from the group comprising
$-[(CR^aR^b)_n-CO-(CR^aR^b)_m]_t-$, $-[(CR^aR^b)_n-CS-(CR^aR^b)_m]_t-$, $-[(CR^aR^b)_n-CS-(CR^aR^b)_m]_t-$, $-[(CR^aR^b)_n-NR^c-CO-(CR^aR^b)_m]_t-$, $-[(CR^aR^b)_n-NR^c-CS-(CR^aR^b)_m]_t-$, $-[(CR^aR^b)_n-NR^c-CO-NR^d-(CR^aR^b)_m]_t-$, $-[(CR^aR^b)_n-NR^c-CS-NR^d-(CR^aR^b)_m]_t-$, $-[(CR^aR^b)_n-NR^c-C(N-CN)-NR^d-(CR^aR^b)_m]_t-$, $-[(CR^aR^b)_n-NR^c-C(N-R^e)-NR^d-(CR^aR^b)_m]_t-$, $-[(CR^aR^b)_n-CO-NR^c-(CR^aR^b)_m]_t-$, $-](CR^aR^b)_n-CS-NR^c-(CR^aR^b)_m]_t-$, $-[(CR^aR^b)_n-NR^c-(CR^aR^b)_m]_t-$, $-[(CR^aR^b)_n-NR^c-SO_2-(CR^aR^b)_m]_t-$, $-[(CR^aR^b)_n-SO_2-NR^c-(CR^aR^b)_m]_t-$, $-[(CR^aR^b)_n-NR^c-CO-O-(CR^aR^b)_m]_t-$,$-[(CR^aR^b)_n-O-CO-NR^c-(CR^aR^b)_m]_t-$, $-[(CR^aR^b)_n-O-(CR^aR^b)_m]_t-$, $-[(CR^aR^b)_n-SR^c-(CR^aR^b)_m]_t-$,$-[(CR^aR^b)_n-SO-(CR^aR^b)_m]_t-$, $-[(CR^aR^b)_n-SO_2-(CR^aR^b)_m]_t-$, $-[(CR^aR^b)_n]_t-$;
wherein:

$R^a$ is selected from the group comprising H, alkyl, cycloalkyl, aryl, heteroaryl and heterocyclyl;
$R^b$ is selected from the group comprising H, alkyl, cycloalkyl, aryl, heteroaryl and heterocyclyl;
$R^c$ is selected from the group comprising H, alkyl, cycloalkyl, aryl, heteroaryl and heterocyclyl;
$R^d$ is selected from the group comprising H, alkyl, cycloalkyl, aryl, heteroaryl and heterocyclyl;
$R^e$ is selected from the group comprising H, alkyl, cycloalkyl, aryl, heteroaryl and heterocyclyl;
n, m and t are independently selected from each other and are any integer between 0 and 10, i. e. 0, 1, 2, 3, 4 , 5, 6, 7, 8, 9 or 10;
Y is selected from the group comprising H, alkyl, substituted alkyl, cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, substituted poly-unsaturated heterocyclyl, aryl, substituted aryl, heteroaryl, and substituted heteroaryl.

In a second aspect which is actually an embodiment of the first aspect of the invention, the problem is solved by a compound which has the structure

$$A-X-Y \qquad (I)$$

wherein A is cycloalkyl, substituted cycloalkyl, heterocyclyl, substituted heterocyclyl, aryl, substituted aryl, heteroaryl or substituted heteroaryl;
X is a spacer and is selected from $X_1$ or $X_2$,
wherein $X_1$ is selected from the group comprising

$-[(CR^aR^b)_n-NR^c-CO-NR^d-(CR^aR^b)_m]_t-$, $-[(CR^aR^b)_n-NR^c-CS-NR^d-(CR^aR^b)_m]_t-$, $-[(CR^aR^b)_n-NR^c-C(N-CN)-NR^d-(CR^aR^b)_m]_t-$, $-[(CR^aR^b)_n-NR^c-C(N-R^e)-NR^d-(CR^aR^b)_m]_t-$, $-[(CR^aR^b)_n-CO-NR^c-(CR^aR^b)_m]_t-$, $-[(CR^aR^b)_n-NR^c-(CR^aR^b)_m]_t-$, $-[(CR^aR^b)_n-NR^c-CO-O-(CR^aR^b)_m]_t-$, $-[(CR^aR^b)_n-O-CO-NR^c-(CR^aR^b)_m]_t-$, $-[(CR^aR^b)_n-O-(CR^aR^b)_m]_t-$, $-[(CR^aR^b)_n-SR^c-(CR^aR^b)_m]_t-$;

$X_2$ is selected from the group comprising $-[(CR^aR^b)_n-CO-(CR^aR^b)_m]_t-$, $-[(CR^aR^b)_n-CS-(CR^aR^b)_m]t-$, $-[(CR^aR^b)_n-NR^c-CO-(CR^aR^b)_m]_t-$, $-[(CR^aR^b)_n-CO-NR^c-(CR^aR^b)_m]_t-$, $-[(CR^aR^b)_n-NR^c-CS-(CR^aR^b)_m]_t-$, $-[(CR^aR^b)_n-CS-NR^c-(CR^aR^b)_m]_t-$, $-[(CR^aR^b)_n-NR^c-SO_2-(CR^aR^b)_m]_t-$, $-[(CR^aR^b)_n-SO_2-NR^c-(CR^aR^b)_m]_t-$, $-[(CR^aR^b)_n-SO-(CR^aR^b)_m]_t-[(CR^aR^b)_n-SO_2-(CR^aR^b)_m]_t-$, $-[(CR^aR^b)_n]_t-$;

wherein n and m are independently selected from each other and are any integer between 0 and 10 provided that if n is 0, m is different from 0, and if m is 0, n is different from 0;

wherein t is independently selected from n and/or m and is any integer between 0 and 10;

wherein

$R^a$, $R^6$, $R^c$, $R^d$ and $R^e$ are independently selected from the group comprising H, alkyl, cycloalkyl, aryl, heteroaryl and heterocyclyl;

and wherein Y is selected from the group comprising alkyl, substituted alkyl, cycloalkyl, substituted cycloalkyl, cycloalkenyl, substituted cycloalkenyl, heterocyclyl, substituted heterocyclyl, mono- or poly-unsaturated heterocyclyl or mono- or substituted poly-unsaturated heterocyclyl, aryl, substituted aryl, heteroaryl, and substituted heteroaryl.

As used herein, any integer between, e. g., 0 and 10 means 1, 2, 3, 4, 5, 6, 7, 8, 9 and 10.

In an embodiment of any aspect of the present invention Y is different from a peptide. As used herein peptide means a polymer of at least two amino acids which are linked by an amide bond. Any of the amino acids may be a natural or a non natural acid.

In a preferred embodiment of any aspect of the present invention m, n and t are independently selected from each other and are any integer between 0 and 5; more preferably if n is 0, m is different from 0 and if m is 0, n is different from 0.

Also as used herein the term compound(s) according to the present invention means any compound(s) according to any aspect of the present invention. If not indicated to the contrary, any embodiment of the present invention is an embodiment of any aspect of the present invention.

In a preferred embodiment of the inventive compound, A is

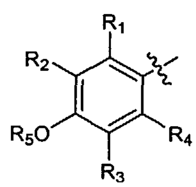

or

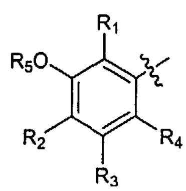

or

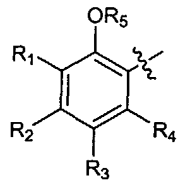

wherein $R_1$, $R_2$, $R_3$ and $R_4$ are each independently selected from the group comprising H, O, S, $NR^e$, halogen, alkyl, cycloalkyl, aryl, heterocyclyl and heteroaryl;
$R_5$ is selected from selected from the group comprising H, alkyl, cycloalkyl, aryl, heterocyclyl and heteroaryl; and
$R^e$ is selected from the group comprising H, alkyl, aryl, alkoxy, aryloxy, alkylamino and arylamino.

In an even more preferred embodiment of the inventive compound $R_1$, $R_2$, $R_3$ $R_4$ and/or $R_5$ have independently from each other one or more groups of the formula $R^f$; whereby $R^f$ is selected from the group comprising alkyl, cycloalkyl, aryl, heterocyclyl, heteroaryl, alkoxy, aryloxy, arylalkoxy, alkoxycarbonyl, aryloxycarbonyl, alkanoyl, aroyl, alkanoyloxy, aroyloxy, carbamoyl, alkanoylamino, aroylamino, alkylthio, arylthio, ureido and amine.

In a further preferred embodiment
the alkylthio group is derivatized, preferably the sulfur atom of the alkylthio group is oxidized to a sulfoxide or sulfone;
the arylthio group is derivatized, preferably the sulfur atom of the arylthio group is oxidized to a sulfoxide or sulfone,
the ureido group is derivatized, preferably the nitrogen atom of the ureido group is independently mono- or di-substituted, more preferably the substitution is selected from the group comprising alkyl, aryl, heterocyclyl, heteroaryl, alkoxycarbonylamino, aryloxycarbonylamino, alkylcarbamoyloxy, arylcarbamoyloxy, alkylsulfonylamino, arylsulfonylamino, alkylaminosulfonyl, and arylaminosulfonyl; and/or
the amino group is derivatized, preferably the nitrogen atom is independently mono- or di-substituted by alkly, aryl, heterocyclyl, heteroalyl, halogen, hydroxy, oxo, carboxy, cyano, nitro, amidino and guanidino.

In a still more preferred embodiment $R^f$ is further substituted by one ore more groups $R^g$, whereby $R^g$ is selected from the group comprising alkyl, cycloalkyl, aryl, arylalkyl, alkoxy, aryloxy, arylalkoxy, alkanoyl, aroyl, amino, halogen, hydroxy, oxo, carboxy, cyano, nitro, amidino and guanidino.

Particularly preferred compounds according to the present invention are the compounds specified in the following table 1:

| | Structure | chemical name | Formula | MolWeight | MS data |
|---|---|---|---|---|---|
| 1 | 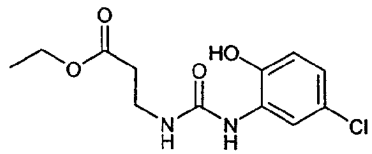 | 3-[3-(5-Chloro-2-hydroxy-phenyl)-ureido]-propionic acid ethyl ester | C12H15ClN2O4 | 286.71 | 286.9 |
| 2 | 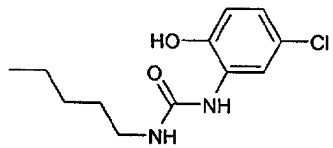 | 1-(5-Chloro-2-hydroxy-phenyl)-3-pentyl-urea | C12H17ClN2O2 | 256.73 | 256.9 |
| 3 | 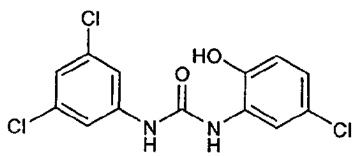 | 1-(5-Chloro-2-hydroxy-phenyl)-3-(3,5-dichloro-phenyl)-urea | C13H9Cl3N2O2 | 331.59 | 331.9 |
| 4 | 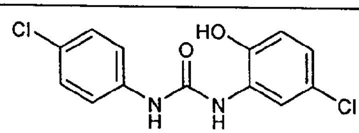 | 1-(5-Chloro-2-hydroxy-phenyl)-3-(4-chloro-phenyl)-urea | C13H10Cl2N2O2 | 297.14 | 297.7 |
| 5 | 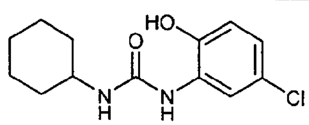 | 1-(5-Chloro-2-hydroxy-phenyl)-3-cyclohexyl-urea | C13H17ClN2O2 | 268.74 | 269.1 |
| 6 | 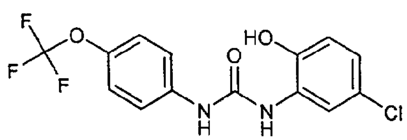 | 1-(5-Chloro-2-hydroxy-phenyl)-3-(4-trifluoromethoxy-phenyl)-urea | C14H10ClF3N2O3 | 346.69 | 346.9 |

| | | | | | |
|---|---|---|---|---|---|
| 7 | 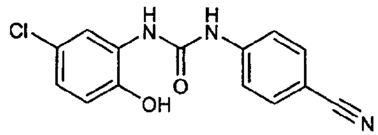 | 1-(5-Chloro-2-hydroxy-phenyl)-3-(4-cyano-phenyl)-urea | C14H10ClN3O2 | 287.70 | 287.9 |
| 8 | 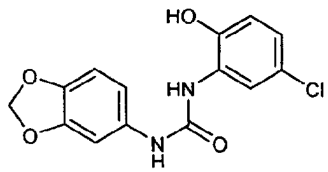 | 1-Benzo[1,3]dioxol-5-yl-3-(5-chloro-2-hydroxy-phenyl)-urea | C14H11ClN2O4 | 306.70 | 306.9 |
| 9 | 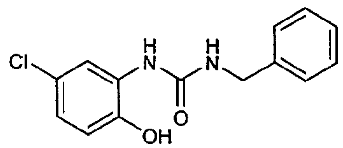 | 1-Benzyl-3-(5-chloro-2-hydroxy-phenyl)-urea | C14H13ClN2O2 | 276.72 | 276.9 |
| 10 | 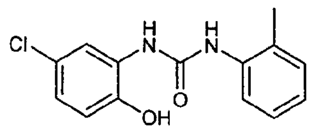 | 1-(5-Chloro-2-hydroxy-phenyl)-3-o-tolyl-urea | C14H13ClN2O2 | 276.72 | 276.9 |
| 11 | 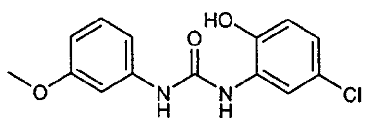 | 1-(5-Chloro-2-hydroxy-phenyl)-3-(3-methoxy-phenyl)-urea | C14H13ClN2O3 | 292.72 | 292.9 |
| 12 | 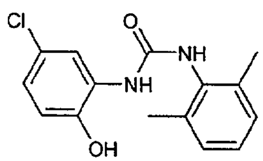 | 1-(5-Chloro-2-hydroxy-phenyl)-3-(2,6-dimethyl-phenyl)-urea | C15H15ClN2O2 | 290.75 | 290.9 |
| 13 | 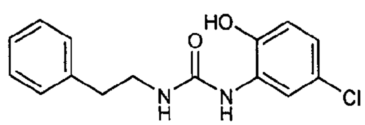 | 1-(5-Chloro-2-hydroxy-phenyl)-3-phenethyl-urea | C15H15ClN2O2 | 290.75 | 291.2 |

| | | | | | |
|---|---|---|---|---|---|
| 14 | 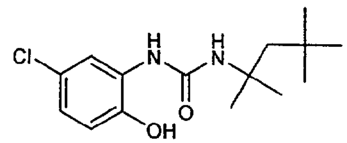 | 1-(5-Chloro-2-hydroxy-phenyl)-3-(1,1,3,3-tetramethyl-butyl)-urea | C15H23ClN2O2 | 298.81 | 299.4 |
| 15 | 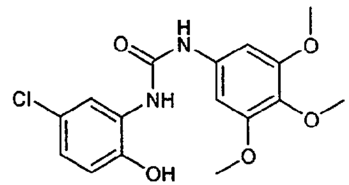 | 1-(5-Chloro-2-hydroxy-phenyl)-3-(3,4,5-trimethoxy-phenyl)-urea | C16H17ClN2O5 | 352.77 | 353.2 |
| 16 | 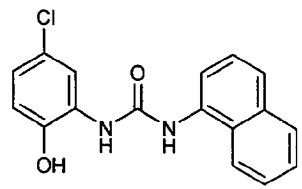 | 1-(5-Chloro-2-hydroxy-phenyl)-3-naphthalen-1-yl-urea | C17H13ClN2O2 | 312.75 | 313.1 |
| 17 | 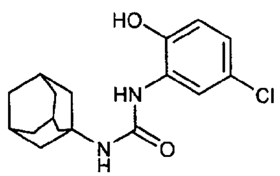 | 1-Adamantan-1-yl-3-(5-chloro-2-hydroxy-phenyl)-urea | C17H21ClN2O2 | 320.82 | 321.4 |
| 18 | 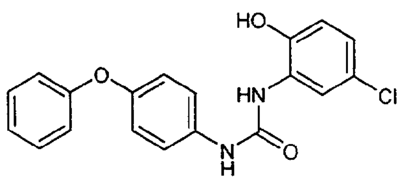 | 1-(5-Chloro-2-hydroxy-phenyl)-3-(4-phenoxy-phenyl)-urea | C19H15ClN2O3 | 354.79 | 354.9 |
| 19 | 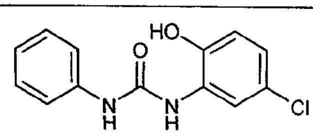 | 1-(5-Chloro-2-hydroxy-phenyl)-3-phenyl-urea | C13H11ClN2O2 | 262.70 | 263.2 |

| | | | | | |
|---|---|---|---|---|---|
| 20 | 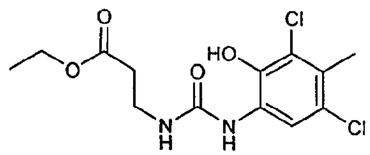 | 3-[3-(3,5-Dichloro-2-hydroxy-4-methyl-phenyl)-ureido]-propionic acid ethyl ester | C13H16Cl2N2O4 | 335.19 | 335.9 |
| 21 | 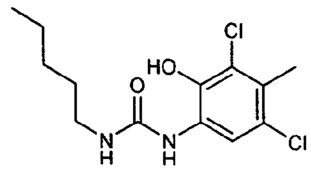 | 1-(3,5-Dichloro-2-hydroxy-4-methyl-phenyl)-3-pentyl-urea | C13H18Cl2N2O2 | 305.20 | 305.9 |
| 22 | 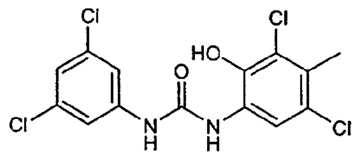 | 1-(3,5-Dichloro-2-hydroxy-4-methyl-phenyl)-3-(3,5-dichloro-phenyl)-urea | C14H10Cl4N2O2 | 380.06 | 380.2 |
| 23 | 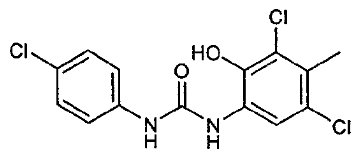 | 1-(4-Chloro-phenyl)-3-(3,5-dichloro-2-hydroxy-4-methyl-phenyl)-urea | C14H11Cl3N2O2 | 345.61 | 345.9 |
| 24 | 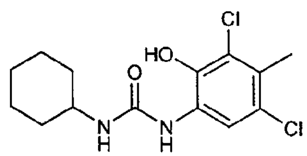 | 1-Cyclohexyl-3-(3,5-dichloro-2-hydroxy-4-methyl-phenyl)-urea | C14H18Cl2N2O2 | 317.21 | 317.8 |
| 25 | 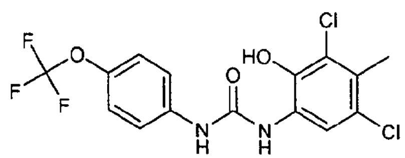 | 1-(3,5-Dichloro-2-hydroxy-4-methyl-phenyl)-3-(4-trifluoromethoxy-phenyl)-urea | C15H11Cl2F3N2O3 | 395.16 | 395.4 |

| | | | | | |
|---|---|---|---|---|---|
| 26 | 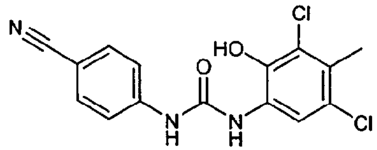 | 1-(4-Cyano-phenyl)-3-(3,5-dichloro-2-hydroxy-4-methyl-phenyl)-urea | C15H11Cl2N3O2 | 336.18 | 336.4 |
| 27 | 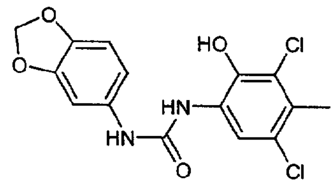 | 1-Benzo[1,3]dioxol-5-yl-3-(3,5-dichloro-2-hydroxy-4-methyl-phenyl)-urea | C15H12Cl2N2O4 | 355.18 | 355.2 |
| 28 | 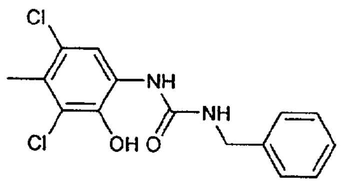 | 1-Benzyl-3-(3,5-dichloro-2-hydroxy-4-methyl-phenyl)-urea | C15H14Cl2N2O2 | 325.19 | 325.5 |
| 29 | 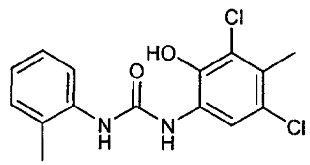 | 1-(3,5-Dichloro-2-hydroxy-4-methyl-phenyl)-3-o-tolyl-urea | C15H14Cl2N2O2 | 325.19 | 325.5 |
| 30 | 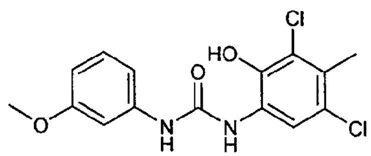 | 1-(3,5-Dichloro-2-hydroxy-4-methyl-phenyl)-3-(3-methoxy-phenyl)-urea | C15H14Cl2N2O3 | 341.19 | 341.4 |
| 31 | 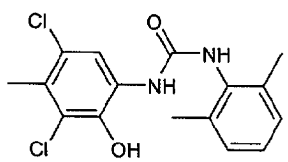 | 1-(3,5-Dichloro-2-hydroxy-4-methyl-phenyl)-3-(2,6-dimethyl-phenyl)-urea | C16H16Cl2N2O2 | 339.22 | 339.4 |

| | | | | | |
|---|---|---|---|---|---|
| 32 | 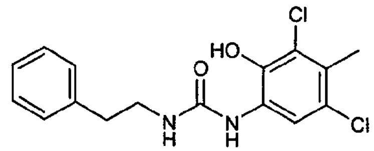 | 1-(3,5-Dichloro-2-hydroxy-4-methyl-phenyl)-3-phenethyl-urea | C16H16Cl2N2O2 | 339.22 | 339.4 |
| 33 | 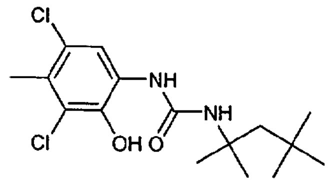 | 1-(3,5-Dichloro-2-hydroxy-4-methyl-phenyl)-3-(1,1,3,3-tetramethyl-butyl)-urea | C16H24Cl2N2O2 | 347.28 | 347.4 |
| 34 | 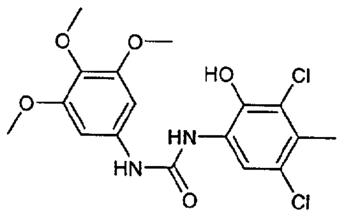 | 1-(3,5-Dichloro-2-hydroxy-4-methyl-phenyl)-3-(3,4,5-trimethoxy-phenyl)-urea | C17H18Cl2N2O5 | 401.25 | 401.3 |
| 35 | 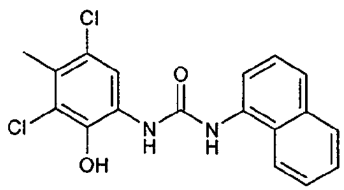 | 1-(3,5-Dichloro-2-hydroxy-4-methyl-phenyl)-3-naphthalen-1-yl-urea | C18H14Cl2N2O2 | 361.23 | 361.3 |
| 36 | 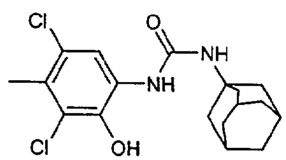 | 1-Adamantan-1-yl-3-(3,5-dichloro-2-hydroxy-4-methyl-phenyl)-urea | C18H22Cl2N2O2 | 369.29 | 369.3 |

| | | | | | |
|---|---|---|---|---|---|
| 37 | 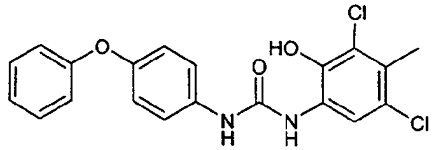 | 1-(3,5-Dichloro-2-hydroxy-4-methyl-phenyl)-3-(4-phenoxy-phenyl)-urea | C20H16Cl2N2O3 | 403.26 | 403.3 |
| 38 | 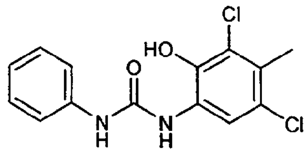 | 1-(3,5-Dichloro-2-hydroxy-4-methyl-phenyl)-3-phenyl-urea | C14H12Cl2N2O2 | 311.17 | 311.3 |
| 39 | 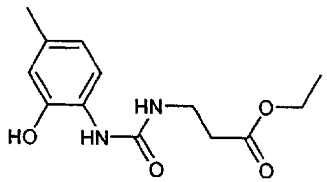 | 3-[3-(2-Hydroxy-4-methyl-phenyl)-ureido]-propionic acid ethyl ester | C13H18N2O4 | 266.30 | 266.5 |
| 40 | 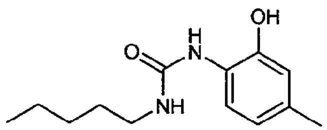 | 1-(2-Hydroxy-4-methyl-phenyl)-3-pentyl-urea | C13H20N2O2 | 236.31 | 236.5 |
| 41 | 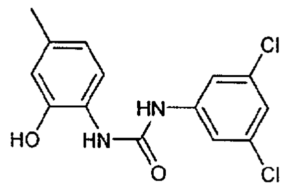 | 1-(3,5-Dichloro-phenyl)-3-(2-hydroxy-4-methyl-phenyl)-urea | C14H12Cl2N2O2 | 311.17 | 311.5 |
| 42 | 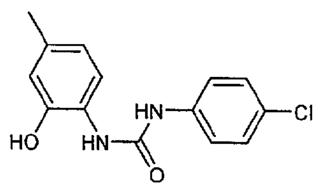 | 1-(4-Chloro-phenyl)-3-(2-hydroxy-4-methyl-phenyl)-urea | C14H13ClN2O2 | 276.72 | 276.8 |

| | | | | | |
|---|---|---|---|---|---|
| 43 | 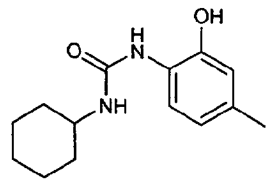 | 1-Cyclohexyl-3-(2-hydroxy-4-methyl-phenyl)-urea | C14H20N2O2 | 248.32 | 248.5 |
| 44 | 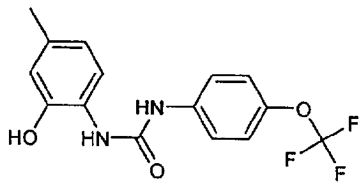 | 1-(2-Hydroxy-4-methyl-phenyl)-3-(4-trifluoromethoxy-phenyl)-urea | C15H13F3N2O3 | 326.27 | 326.6 |
| 45 | 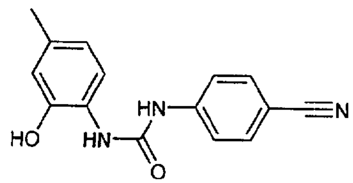 | 1-(4-Cyano-phenyl)-3-(2-hydroxy-4-methyl-phenyl)-urea | C15H13N3O2 | 267.29 | 267.7 |
| 46 | 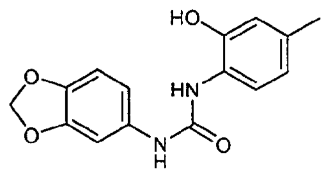 | 1-Benzo[1,3]dioxol-5-yl-3-(2-hydroxy-4-methyl-phenyl)-urea | C15H14N2O4 | 286.29 | 286.7 |
| 47 | 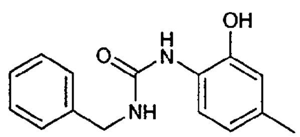 | 1-Benzyl-3-(2-hydroxy-4-methyl-phenyl)-urea | C15H16N2O2 | 256.30 | 256.7 |

| | | | | | |
|---|---|---|---|---|---|
| 48 | 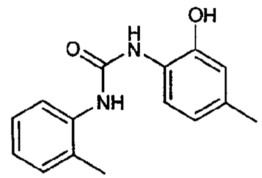 | 1-(2-Hydroxy-4-methyl-phenyl)-3-o-tolyl-urea | $C_{15}H_{16}N_2O_2$ | 256.30 | 256.3 |
| 49 | 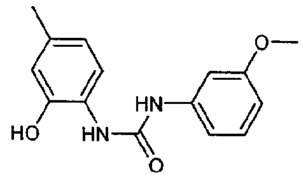 | 1-(2-Hydroxy-4-methyl-phenyl)-3-(3-methoxy-phenyl)-urea | $C_{15}H_{16}N_2O_3$ | 272.30 | 272.3 |
| 50 | 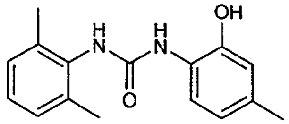 | 1-(2,6-Dimethyl-phenyl)-3-(2-hydroxy-4-methyl-phenyl)-urea | $C_{16}H_{18}N_2O_2$ | 270.33 | 270.7 |
| 51 | 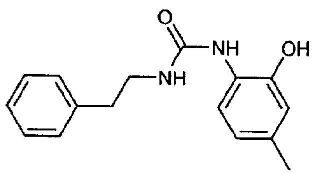 | 1-(2-Hydroxy-4-methyl-phenyl)-3-phenethyl-urea | $C_{16}H_{18}N_2O_2$ | 270.33 | 270.7 |
| 52 | 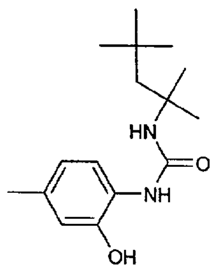 | 1-(2-Hydroxy-4-methyl-phenyl)-3-(1,1,3,3-tetramethyl-butyl)-urea | $C_{16}H_{26}N_2O_2$ | 278.39 | 278.5 |

| | | | | | |
|---|---|---|---|---|---|
| 53 | 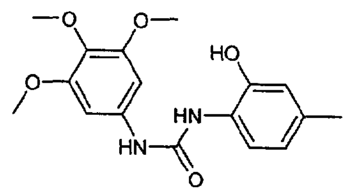 | 1-(2-Hydroxy-4-methyl-phenyl)-3-(3,4,5-trimethoxy-phenyl)-urea | C17H20N2O5 | 332.36 | 332.5 |
| 54 | 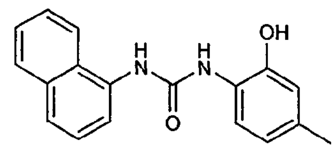 | 1-(2-Hydroxy-4-methyl-phenyl)-3-naphthalen-1-yl-urea | C18H16N2O2 | 292.34 | 292.5 |
| 55 | 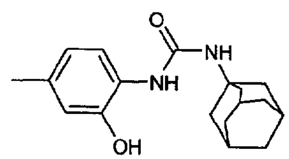 | 1-Adamantan-1-yl-3-(2-hydroxy-4-methyl-phenyl)-urea | C18H24N2O2 | 300.40 | 300.6 |
| 56 | 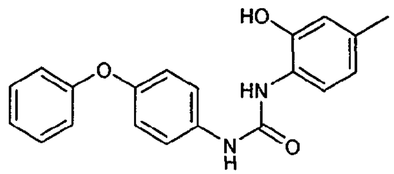 | 1-(2-Hydroxy-4-methyl-phenyl)-3-(4-phenoxy-phenyl)-urea | C20H18N2O3 | 334.37 | 334.6 |
| 57 | 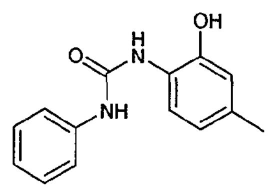 | 1-(2-Hydroxy-4-methyl-phenyl)-3-phenyl-urea | C14H14N2O2 | 242.28 | 242.3 |

| | | | | | |
|---|---|---|---|---|---|
| 58 | 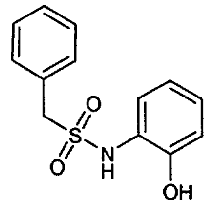 | N-(2-Hydroxy-phenyl)-C-phenyl-methanesulfonamide | C13H13NO3S | 263.31 | 264.0 |
| 59 | 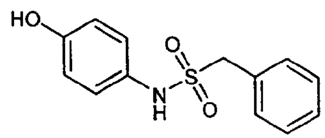 | N-(4-Hydroxy-phenyl)-C-phenyl-methanesulfonamide | C13H13NO3S | 263.31 | 264.0 |
| 60 | 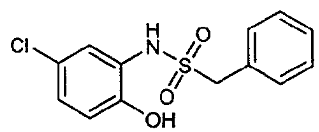 | N-(5-Chloro-2-hydroxy-phenyl)-C-phenyl-methanesulfonamide | C13H12ClNO3S | 297.76 | 298.1 |
| 61 | 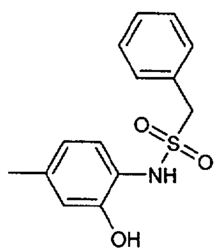 | N-(2-Hydroxy-4-methyl-phenyl)-C-phenyl-methanesulfonamide | C14H15NO3S | 277.34 | 278.1 |
| 62 | 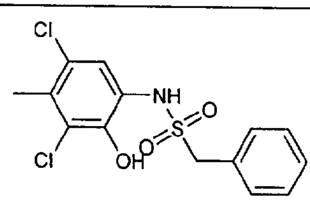 | N-(3,5-Dichloro-2-hydroxy-4-methyl-phenyl)-C-phenyl-methanesulfonamide | C14H13Cl2NO3S | 346.23 | 347.0 |

| | | | | | |
|---|---|---|---|---|---|
| 63 | 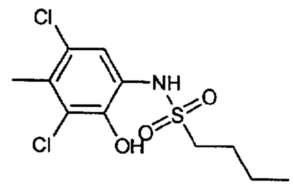 | Butane-1-sulfonic acid (3,5-dichloro-2-hydroxy-4-methyl-phenyl)-amide | C11H15Cl2NO3S | 312.21 | 312.1 |
| 64 | 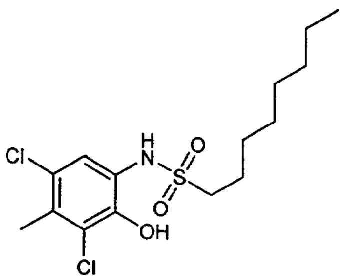 | Octane-1-sulfonic acid (3,5-dichloro-2-hydroxy-4-methyl-phenyl)-amide | C15H23Cl2NO3S | 368.32 | 368.3 |
| 65 | 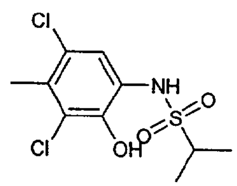 | Propane-2-sulfonic acid (3,5-dichloro-2-hydroxy-4-methyl-phenyl)-amide | C10H13Cl2NO3S | 298.18 | 298.1 |
| 66 | 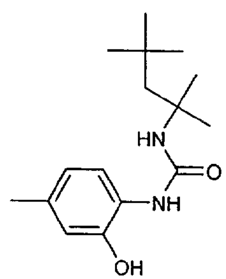 | 1-(2-Hydroxy-4-methyl-phenyl)-3-(1,1,3,3-tetramethyl-butyl)-urea | C16H26N2O2 | 278.39 | 278.5 |

| | | | | | |
|---|---|---|---|---|---|
| 67 | 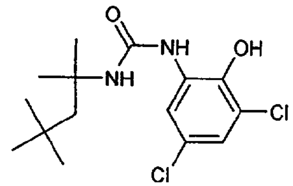 | 1-(3,5-Dichloro-2-hydroxy-phenyl)-3-(1,1,3,3-tetramethyl-butyl)-urea | C15H22Cl2N2O2 | 333.26 | 333.3 |
| 68 | 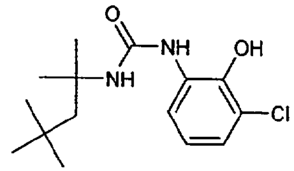 | 1-(3-Chloro-2-hydroxy-phenyl)-3-(1,1,3,3-tetramethyl-butyl)-urea | C15H23ClN2O2 | 298.81 | 298.9 |
| 69 | 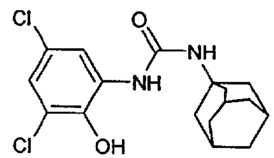 | 1-Adamantan-1-yl-3-(3,5-dichloro-2-hydroxy-phenyl)-urea | C17H20Cl2N2O2 | 355.26 | 355.4 |
| 70 | 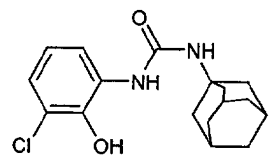 | 1-Adamantan-1-yl-3-(3-chloro-2-hydroxy-phenyl)-urea | C17H21ClN2O2 | 320.82 | 321.0 |
| 71 | 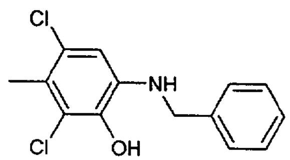 | 6-Benzylamino-2,4-dichloro-3-methyl-phenol | C14H13Cl2NO | 282.17 | 282.2 |
| 72 | 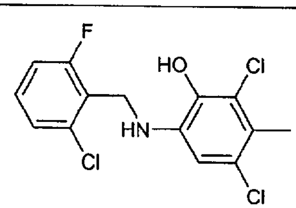 | 2,4-Dichloro-6-(2-chloro-6-fluoro-benzylamino)-3-methyl-phenol | C14H11Cl3FNO | 334.60 | 334.8 |

| | | | | | |
|---|---|---|---|---|---|
| 73 | 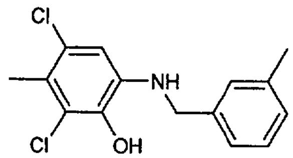 | 2,4-Dichloro-3-methyl-6-(3-methyl-benzylamino)-phenol | C15H15Cl2NO | 296.20 | 296.3 |
| 74 | 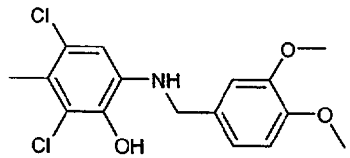 | 2,4-Dichloro-6-(3,4-dimethoxy-benzylamino)-3-methyl-phenol | C16H17Cl2NO3 | 342.22 | 342.2 |
| 75 | 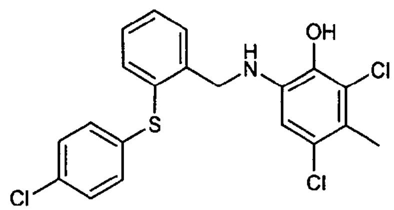 | 2,4-Dichloro-6-[2-(4-chloro-phenylsulfanyl)-benzylamino]-3-methyl-phenol | C20H16Cl3NOS | 424.77 | 424.8 |
| 76 | 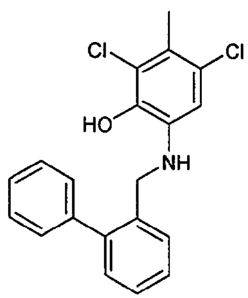 | 6-[(Biphenyl-2-ylmethyl)-amino]-2,4-dichloro-3-methyl-phenol | C20H17Cl2NO | 358.27 | 358.3 |
| 77 | 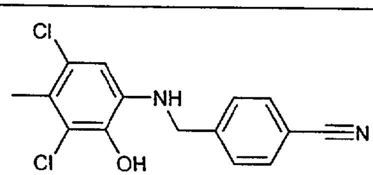 | 4-[(3,5-Dichloro-2-hydroxy-4-methyl-phenylamino)-methyl]-benzonitrile | C15H12Cl2N2O | 307.18 | 307.2 |

| | | | | | |
|---|---|---|---|---|---|
| 78 | 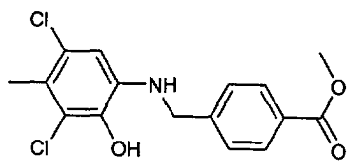 | 4-[(3,5-Dichloro-2-hydroxy-4-methyl-phenylamino)-methyl]-benzoic acid methyl ester | C16H15Cl2NO3 | 340.21 | 340.4 |
| 79 | 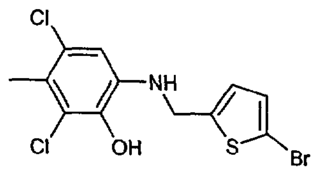 | 6-[(5-Bromo-thiophen-2-ylmethyl)-amino]-2,4-dichloro-3-methyl-phenol | C12H10BrCl2NOS | 367.09 | 367.2 |
| 80 | 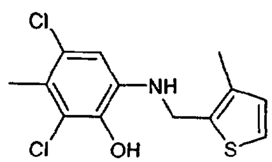 | 2,4-Dichloro-3-methyl-6-[(3-methyl-thiophen-2-ylmethyl)-amino]-phenol | C13H13Cl2NOS | 302.22 | 302.2 |
| 81 | 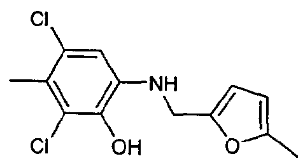 | 2,4-Dichloro-3-methyl-6-[(5-methyl-furan-2-ylmethyl)-amino]-phenol | C13H13Cl2NO2 | 286.16 | 286.2 |
| 82 | 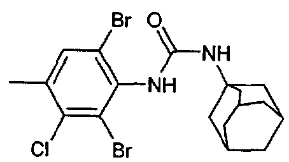 | 1-Adamantan-1-yl-3-(2,6-dibromo-3-chloro-4-methyl-phenyl)-urea | C18H21Br2ClN2O | 476.64 | 476.8 |
| 83 | 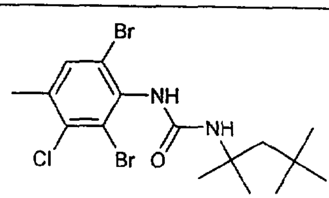 | 1-(2,6-Dibromo-3-chloro-4-methyl-phenyl)-3-(1,1,3,3-tetramethyl-butyl)-urea | C16H23Br2ClN2O | 454.63 | 454.8 |

| | | | | | |
|---|---|---|---|---|---|
| 84 | 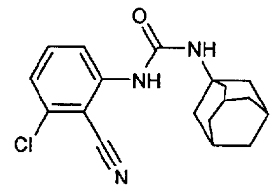 | 1-Adamantan-1-yl-3-(3-chloro-2-cyano-phenyl)-urea | C18H20ClN3O | 329.83 | 329.9 |
| 85 | 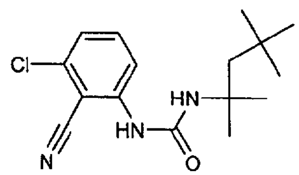 | 1-(3-Chloro-2-cyano-phenyl)-3-(1,1,3,3-tetramethyl-butyl)-urea | C16H22ClN3O | 307.82 | 308.1 |
| 86 | 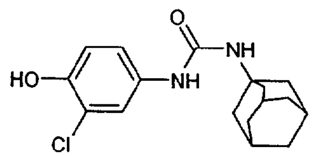 | 1-Adamantan-1-yl-3-(3-chloro-4-hydroxy-phenyl)-urea | C17H21ClN2O2 | 320.82 | 321.0 |
| 87 | 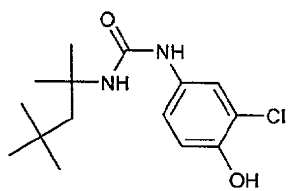 | 1-(3-Chloro-4-hydroxy-phenyl)-3-(1,1,3,3-tetramethyl-butyl)-urea | C15H23ClN2O2 | 298.81 | 299.0 |
| 88 | 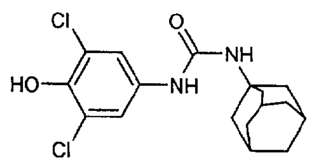 | 1-Adamantan-1-yl-3-(3,5-dichloro-4-hydroxy-phenyl)-urea | C17H20Cl2N2O2 | 355.26 | 355.4 |

| | | | | | |
|---|---|---|---|---|---|
| 89 | 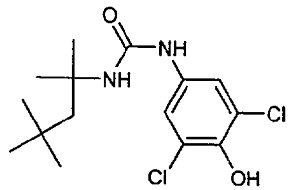 | 1-(3,5-Dichloro-4-hydroxy-phenyl)-3-(1,1,3,3-tetramethyl-butyl)-urea | C15H22Cl2N2O2 | 333.26 | 333.4 |
| 90 | 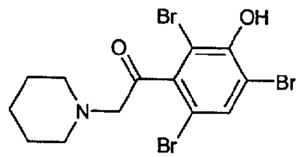 | 1-Piperidin-1-yl-2-(2,4,6-tribromo-3-hydroxy-phenyl)-ethanone | C13H14Br3NO2 | 455.97 | 456.2 |
| 91 | 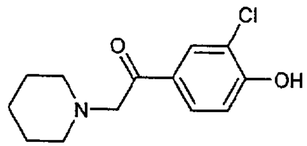 | 2-(3-Chloro-4-hydroxy-phenyl)-1-piperidin-1-yl-ethanone | C13H16ClNO2 | 253.73 | 254.0 |
| 92 | 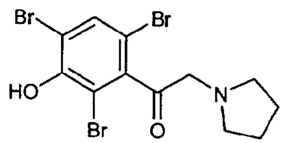 | 1-Pyrrolidin-1-yl-2-(2,4,6-tribromo-3-hydroxy-phenyl)-ethanone | C12H12Br3NO2 | 441.94 | 442.4 |
| 93 | 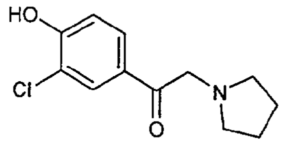 | 2-(3-Chloro-4-hydroxy-phenyl)-1-pyrrolidin-1-yl-ethanone | C12H14ClNO2 | 239.70 | 239.7 |
| 94 | 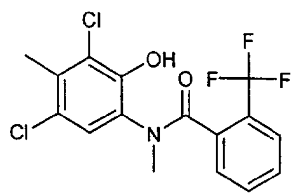 | N-(3,5-Dichloro-2-hydroxy-4-methyl-phenyl)-N-methyl-2-trifluoromethyl-benzamide | C16H12Cl2F3NO2 | 378.18 | 378.2 |

| | | | | | |
|---|---|---|---|---|---|
| 95 | 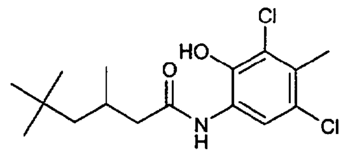 | 3,5,5-Trimethyl-hexanoic acid (3,5-dichloro-2-hydroxy-4-methyl-phenyl)-amide | C16H23Cl2NO2 | 332.27 | 332.3 |
| 96 | 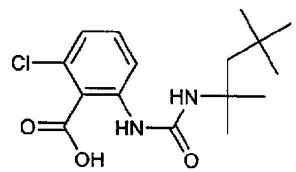 | 2-Chloro-6-[3-(1,1,3,3-tetramethyl-butyl)-ureido]-benzoic acid | C16H23ClN2O3 | 326.82 | 327.0 |
| 97 | 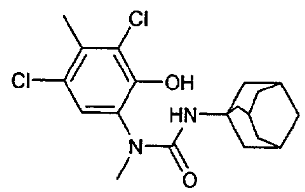 | 3-Adamantan-1-yl-1-(3,5-dichloro-2-hydroxy-4-methyl-phenyl)-1-methyl-urea | C19H24Cl2N2O2 | 383.32 | 383.4 |
| 98 | 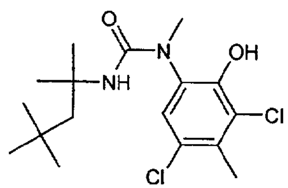 | 1-(3,5-Dichloro-2-hydroxy-4-methyl-phenyl)-1-methyl-3-(1,1,3,3-tetramethyl-butyl)-urea | C17H26Cl2N2O2 | 361.31 | 361.4 |
| 99 | 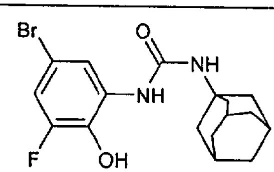 | 1-Adamantan-1-yl-3-(5-bromo-3-fluoro-2-hydroxy-phenyl)-urea | C17H20BrFN2O2 | 383.26 | 383.3 |

| | | | | | |
|---|---|---|---|---|---|
| 100 | 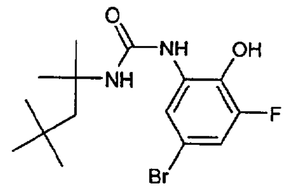 | 1-(5-Bromo-3-fluoro-2-hydroxy-phenyl)-3-(1,1,3,3-tetramethyl-butyl)-urea | C15H22BrFN2O2 | 361.25 | 361.2 |
| 101 | 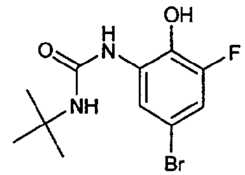 | 1-(5-Bromo-3-fluoro-2-hydroxy-phenyl)-3-tert-butyl-urea | C11H14BrFN2O2 | 305.15 | 305.2 |
| 102 | 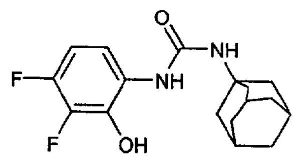 | 1-Adamantan-1-yl-3-(3,4-difluoro-2-hydroxy-phenyl)-urea | C17H20F2N2O2 | 322.35 | 322.5 |
| 103 | 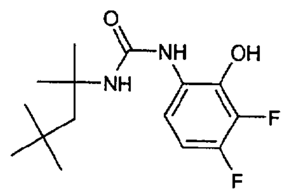 | 1-(3,4-Difluoro-2-hydroxy-phenyl)-3-(1,1,3,3-tetramethyl-butyl)-urea | C15H22F2N2O2 | 300.35 | 300.5 |
| 104 | 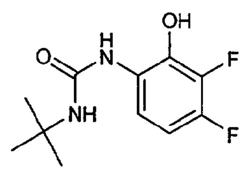 | 1-tert-Butyl-3-(3,4-difluoro-2-hydroxy-phenyl)-urea | C11H14F2N2O2 | 244.24 | 244.3 |
| 105 | 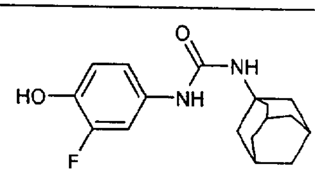 | 1-Adamantan-1-yl-3-(3-fluoro-4-hydroxy-phenyl)-urea | C17H21FN2O2 | 304.36 | 304.4 |

| | | | | | |
|---|---|---|---|---|---|
| 106 | 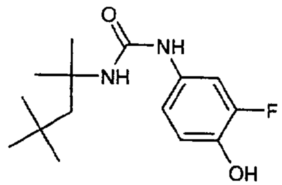 | 1-(3-Fluoro-4-hydroxy-phenyl)-3-(1,1,3,3-tetramethyl-butyl)-urea | C15H23FN2O2 | 282.36 | 282.4 |
| 107 | 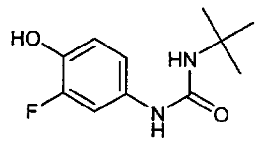 | 1-tert-Butyl-3-(3-fluoro-4-hydroxy-phenyl)-urea | C11H15FN2O2 | 226.25 | 226.4 |
| 108 | 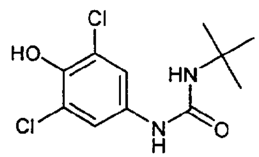 | 1-tert-Butyl-3-(3,5-dichloro-4-hydroxy-phenyl)-urea | C11H14Cl2N2O2 | 277.15 | 277.2 |
| 109 | 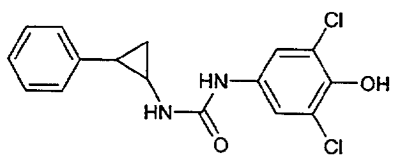 | 1-(3,5-Dichloro-4-hydroxy-phenyl)-3-(2-phenyl-cyclopropyl)-urea | C16H14Cl2N2O2 | 337.20 | 337.3 |
| 110 | 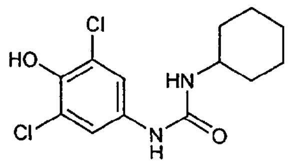 | 1-Cyclohexyl-3-(3,5-dichloro-4-hydroxy-phenyl)-urea | C13H16Cl2N2O2 | 303.19 | 303.4 |
| 111 | 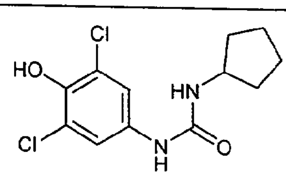 | 1-Cyclopentyl-3-(3,5-dichloro-4-hydroxy-phenyl)-urea | C12H14Cl2N2O2 | 289.16 | 289.4 |

| | | | | | |
|---|---|---|---|---|---|
| 112 | 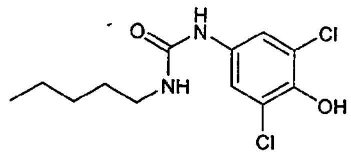 | 1-(3,5-Dichloro-4-hydroxy-phenyl)-3-pentyl-urea | C12H16Cl2N2O2 | 291.18 | 291.2 |
| 113 | 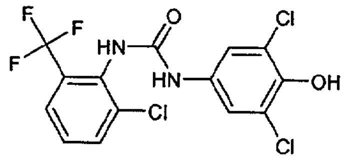 | 1-(2-Chloro-6-trifluoromethyl-phenyl)-3-(3,5-dichloro-4-hydroxy-phenyl)-urea | C14H8Cl3F3N2O2 | 399.58 | 399.8 |
| 114 | 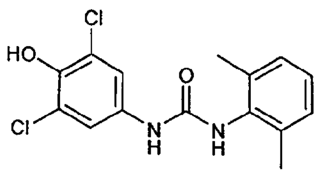 | 1-(3,5-Dichloro-4-hydroxy-phenyl)-3-(2,6-dimethyl-phenyl)-urea | C15H14Cl2N2O2 | 325.19 | 325.3 |
| 115 | 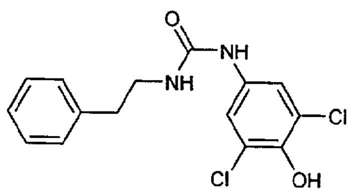 | 1-(3,5-Dichloro-4-hydroxy-phenyl)-3-phenethyl-urea | C15H14Cl2N2O2 | 325.19 | 325.3 |
| 116 | 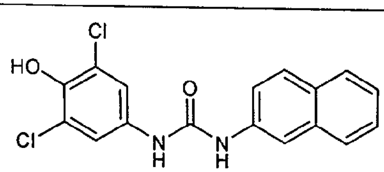 | 1-(3,5-Dichloro-4-hydroxy-phenyl)-3-naphthalen-2-yl-urea | C17H12Cl2N2O2 | 347.20 | 347.2 |

| | | | | | |
|---|---|---|---|---|---|
| 117 | 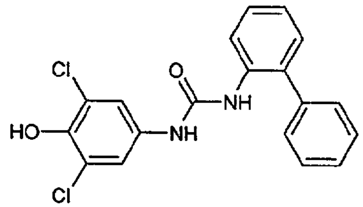 | 1-Biphenyl-2-yl-3-(3,5-dichloro-4-hydroxy-phenyl)-urea | C19H14Cl2N2O2 | 373.24 | 373.3 |
| 118 | 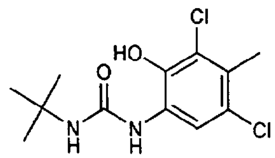 | 1-tert-Butyl-3-(3,5-dichloro-2-hydroxy-4-methyl-phenyl)-urea | C12H16Cl2N2O2 | 291.18 | 291.2 |
| 119 | 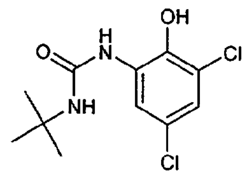 | 1-tert-Butyl-3-(3,5-dichloro-2-hydroxy-phenyl)-urea | C11H14Cl2N2O2 | 277.15 | 277.2 |
| 120 | 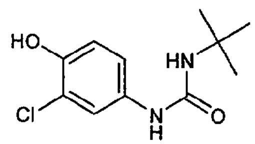 | 1-tert-Butyl-3-(3-chloro-4-hydroxy-phenyl)-urea | C11H15ClN2O2 | 242.70 | 242.9 |
| 121 | 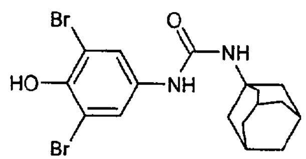 | 1-Adamantan-1-yl-3-(3,5-dibromo-4-hydroxy-phenyl)-urea | C17H20Br2N2O2 | 444.17 | 444.1 |
| 122 | 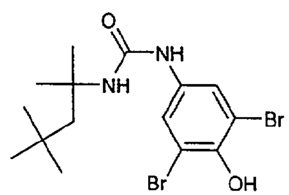 | 1-(3,5-Dibromo-4-hydroxy-phenyl)-3-(1,1,3,3-tetramethyl-butyl)-urea | C15H22Br2N2O2 | 422.16 | 422.3 |

| | | | | | |
|---|---|---|---|---|---|
| 123 | 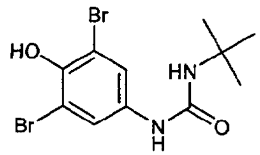 | 1-tert-Butyl-3-(3,5-dibromo-4-hydroxy-phenyl)-urea | C11H14Br2N2O2 | 366.05 | 366.1 |
| 124 | 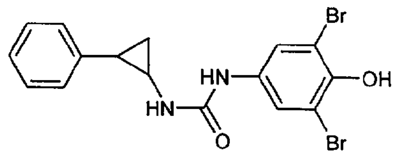 | 1-(3,5-Dibromo-4-hydroxy-phenyl)-3-(2-phenyl-cyclopropyl)-urea | C16H14Br2N2O2 | 426.11 | 426.1 |
| 125 | 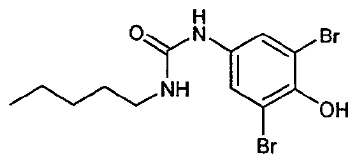 | 1-(3,5-Dibromo-4-hydroxy-phenyl)-3-pentyl-urea | C12H16Br2N2O2 | 380.08 | 380.1 |
| 126 | 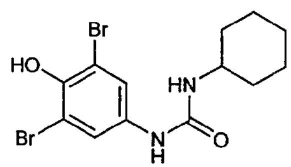 | 1-Cyclohexyl-3-(3,5-dibromo-4-hydroxy-phenyl)-urea | C13H16Br2N2O2 | 392.09 | 392.1 |
| 127 | 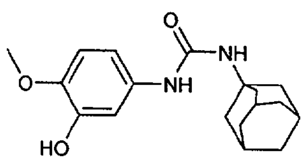 | 1-Adamantan-1-yl-3-(3-hydroxy-4-methoxy-phenyl)-urea | C18H24N2O3 | 316.40 | 317.2 |

| | | | | | |
|---|---|---|---|---|---|
| 128 | 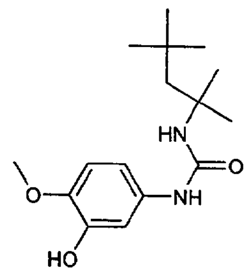 | 1-(3-Hydroxy-4-methoxy-phenyl)-3-(1,1,3,3-tetramethyl-butyl)-urea | C16H26N2O3 | 294.39 | 295.4 |
| 129 | 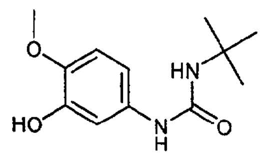 | 1-tert-Butyl-3-(3-hydroxy-4-methoxy-phenyl)-urea | C12H18N2O3 | 238.29 | 239.2 |
| 130 | 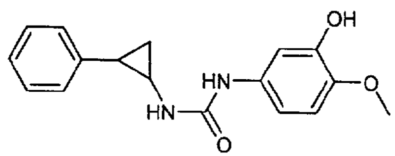 | 1-(3-Hydroxy-4-methoxy-phenyl)-3-(2-phenyl-cyclopropyl)-urea | C17H18N2O3 | 298.34 | 299.3 |
| 131 | 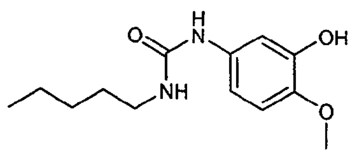 | 1-(3-Hydroxy-4-methoxy-phenyl)-3-pentyl-urea | C13H20N2O3 | 252.31 | 253.4 |
| 132 | 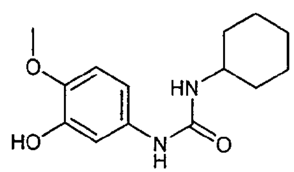 | 1-Cyclohexyl-3-(3-hydroxy-4-methoxy-phenyl)-urea | C14H20N2O3 | 264.32 | 265.2 |

| | | | | | |
|---|---|---|---|---|---|
| 133 | 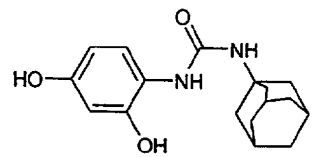 | 1-Adamantan-1-yl-3-(2,4-dihydroxy-phenyl)-urea | C17H22N2O3 | 302.37 | 303.4 |
| 134 | 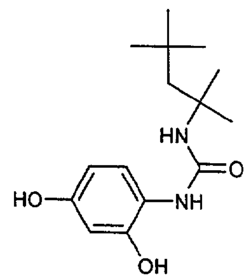 | 1-(2,4-Dihydroxy-phenyl)-3-(1,1,3,3-tetramethyl-butyl)-urea | C15H24N2O3 | 280.37 | 281.3 |
| 135 | 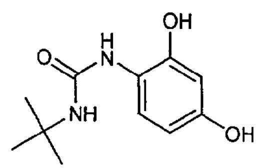 | 1-tert-Butyl-3-(2,4-dihydroxy-phenyl)-urea | C11H16N2O3 | 224.26 | 225.2 |
| 136 | 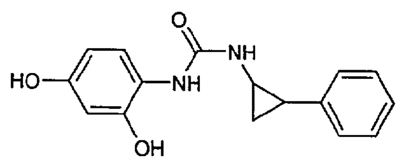 | 1-(2,4-Dihydroxy-phenyl)-3-(2-phenyl-cyclopropyl)-urea | C16H16N2O3 | 284.31 | 285.4 |
| 137 | 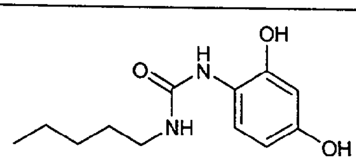 | 1-(2,4-Dihydroxy-phenyl)-3-pentyl-urea | C12H18N2O3 | 238.29 | 239.3 |

| | | | | | |
|---|---|---|---|---|---|
| 138 | 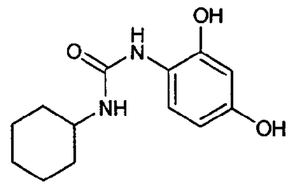 | 1-Cyclohexyl-3-(2,4-dihydroxy-phenyl)-urea | C13H18N2O3 | 250.30 | 251.3 |
| 139 | 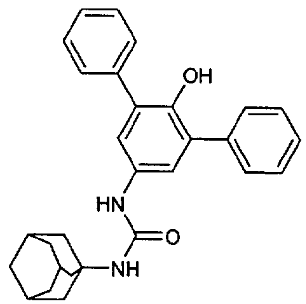 | 1-Adamantan-1-yl-3-(2'-hydroxy-[1,1';3',1"]terphenyl-5'-yl)-urea | C29H30N2O2 | 438.57 | 439.5 |
| 140 | 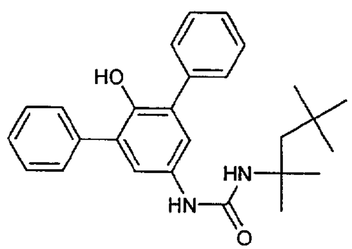 | 1-(2'-Hydroxy-[1,1';3',1"]terphenyl-5'-yl)-3-(1,1,3,3-tetramethyl-butyl)-urea | C27H32N2O2 | 416.56 | 417.6 |
| 141 | 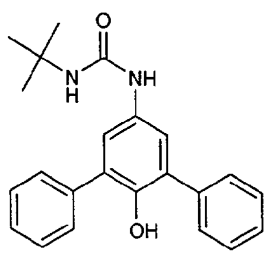 | 1-tert-Butyl-3-(2'-hydroxy-[1,1';3',1"]terphenyl-5'-yl)-urea | C23H24N2O2 | 360.45 | 361.3 |

| | | | | | |
|---|---|---|---|---|---|
| 142 | 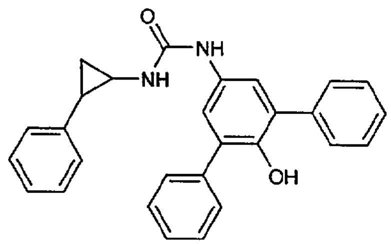 | 1-(2'-Hydroxy-[1,1';3',1"]terphenyl-5'-yl)-3-(2-phenyl-cyclopropyl)-urea | C28H24N2O2 | 420.51 | 421.4 |
| 143 | 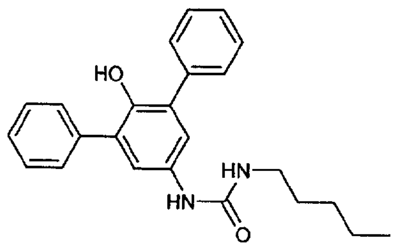 | 1-(2'-Hydroxy-[1,1';3',1"]terphenyl-5'-yl)-3-pentyl-urea | C24H26N2O2 | 374.48 | 375.4 |
| 144 | 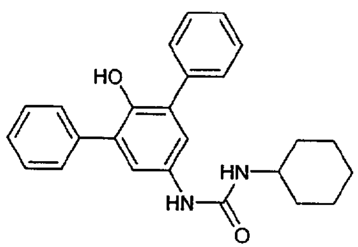 | 1-Cyclohexyl-3-(2'-hydroxy-[1,1';3',1"]terphenyl-5'-yl)-urea | C25H26N2O2 | 386.49 | 387.5 |
| 145 | 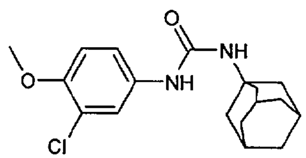 | 1-Adamantan-1-yl-3-(3-chloro-4-methoxy-phenyl)-urea | C18H23ClN2O2 | 334.84 | 335.7 |

| | | | | | |
|---|---|---|---|---|---|
| 146 | 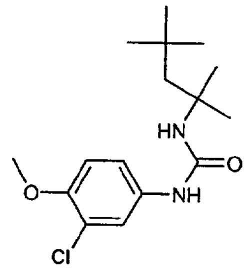 | 1-(3-Chloro-4-methoxy-phenyl)-3-(1,1,3,3-tetramethyl-butyl)-urea | C16H25ClN2O2 | 312.84 | 313.8 |
| 147 | 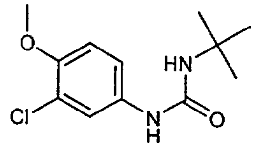 | 1-tert-Butyl-3-(3-chloro-4-methoxy-phenyl)-urea | C12H17ClN2O2 | 256.73 | 257.8 |
| 148 | 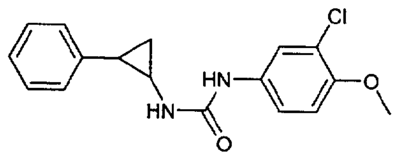 | 1-(3-Chloro-4-methoxy-phenyl)-3-(2-phenyl-cyclopropyl)-urea | C17H17ClN2O2 | 316.79 | 317.8 |
| 149 | 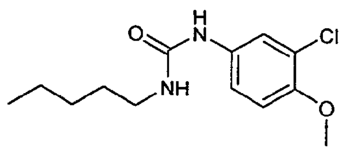 | 1-(3-Chloro-4-methoxy-phenyl)-3-pentyl-urea | C13H19ClN2O2 | 270.76 | 271.8 |
| 150 | 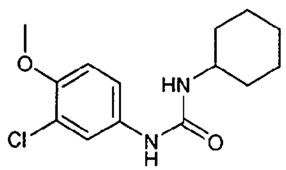 | 1-(3-Chloro-4-methoxy-phenyl)-3-cyclohexyl-urea | C14H19ClN2O2 | 282.77 | 283.8 |
| 151 | 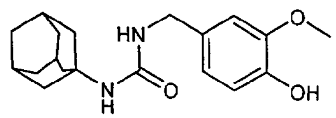 | 1-Adamantan-1-yl-3-(4-hydroxy-3-methoxy-benzyl)-urea | C19H26N2O3 | 330.43 | 331.3 |

| | | | | | |
|---|---|---|---|---|---|
| 152 | 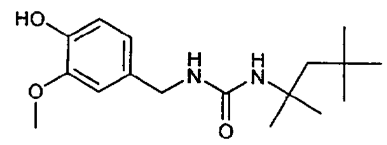 | 1-(4-Hydroxy-3-methoxy-benzyl)-3-(1,1,3,3-tetramethyl-butyl)-urea | C17H28N2O3 | 308.42 | 309.3 |
| 153 | 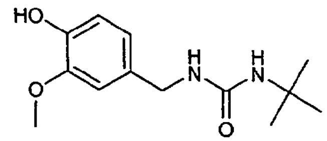 | 1-tert-Butyl-3-(4-hydroxy-3-methoxy-benzyl)-urea | C13H20N2O3 | 252.31 | 253.3 |
| 154 | 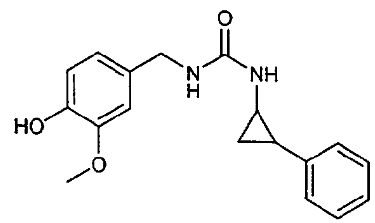 | 1-(4-Hydroxy-3-methoxy-benzyl)-3-(2-phenyl-cyclopropyl)-urea | C18H20N2O3 | 312.37 | 313.3 |
| 155 | 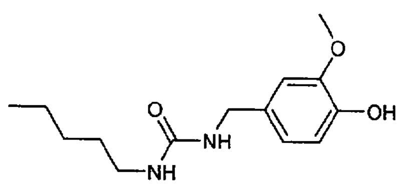 | 1-(4-Hydroxy-3-methoxy-benzyl)-3-pentyl-urea | C14H22N2O3 | 266.34 | 267.3 |
| 156 | 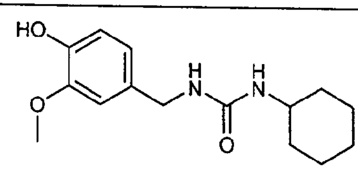 | 1-Cyclohexyl-3-(4-hydroxy-3-methoxy-benzyl)-urea | C15H22N2O3 | 278.35 | 279.4 |
| 157 | 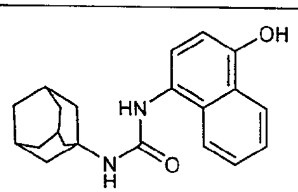 | 1-Adamantan-1-yl-3-(4-hydroxy-naphthalen-1-yl)-urea | C21H24N2O2 | 336.43 | 337.4 |

| | | | | | |
|---|---|---|---|---|---|
| 158 | 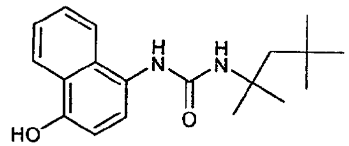 | 1-(4-Hydroxy-naphthalen-1-yl)-3-(1,1,3,3-tetramethyl-butyl)-urea | C19H26N2O2 | 314.43 | 315.3 |
| 159 | 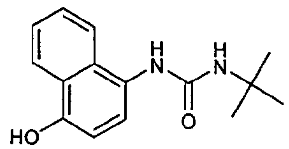 | 1-tert-Butyl-3-(4-hydroxy-naphthalen-1-yl)-urea | C15H18N2O2 | 258.32 | 259.2 |
| 160 | 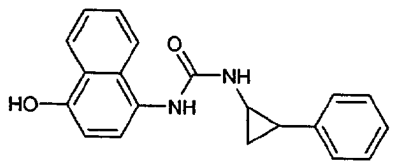 | 1-(4-Hydroxy-naphthalen-1-yl)-3-(2-phenyl-cyclopropyl)-urea | C20H18N2O2 | 318.37 | 319.3 |
| 161 | 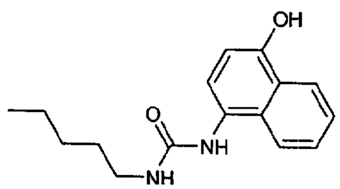 | 1-(4-Hydroxy-naphthalen-1-yl)-3-pentyl-urea | C16H20N2O2 | 272.35 | 273.3 |
| 162 | 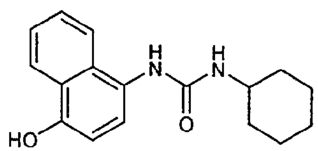 | 1-Cyclohexyl-3-(4-hydroxy-naphthalen-1-yl)-urea | C17H20N2O2 | 284.36 | 285.3 |
| 163 | 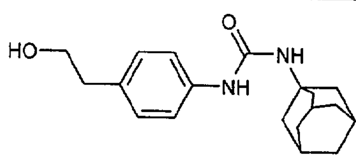 | 1-Adamantan-1-yl-3-[4-(2-hydroxy-ethyl)-phenyl]-urea | C19H26N2O2 | 314.43 | 315.4 |

| | | | | | |
|---|---|---|---|---|---|
| 164 | 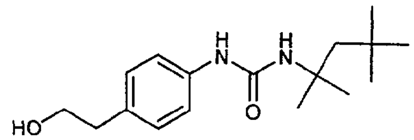 | 1-[4-(2-Hydroxy-ethyl)-phenyl]-3-(1,1,3,3-tetramethyl-butyl)-urea | C17H28N2O2 | 292.42 | 293.3 |
| 165 | 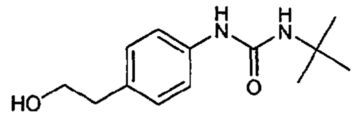 | 1-tert-Butyl-3-[4-(2-hydroxy-ethyl)-phenyl]-urea | C13H20N2O2 | 236.31 | 237.3 |
| 166 | 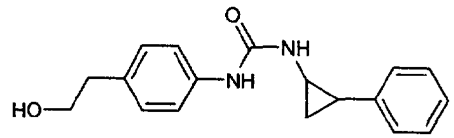 | 1-[4-(2-Hydroxy-ethyl)-phenyl]-3-(2-phenyl-cyclopropyl)-urea | C18H20N2O2 | 296.37 | 297.3 |
| 167 | 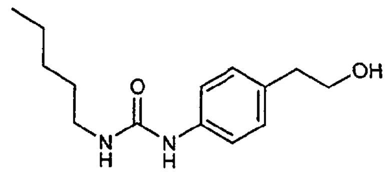 | 1-[4-(2-Hydroxy-ethyl)-phenyl]-3-pentyl-urea | C14H22N2O2 | 250.34 | 251.3 |
| 168 | 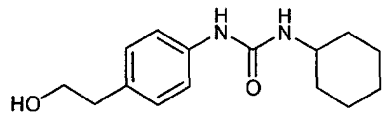 | 1-Cyclohexyl-3-[4-(2-hydroxy-ethyl)-phenyl]-urea | C15H22N2O2 | 262.35 | 263.3 |
| 169 | 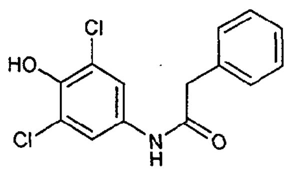 | N-(3,5-Dichloro-4-hydroxy-phenyl)-2-phenyl-acetamide | C14H11Cl2NO2 | 296.15 | 296.2 |

| | | | | | |
|---|---|---|---|---|---|
| 170 | 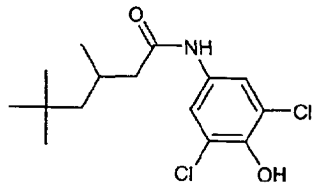 | 3,5,5-Trimethyl-hexanoic acid (3,5-dichloro-4-hydroxy-phenyl)-amide | C15H21Cl2NO2 | 318.24 | 318.4 |
| 171 | 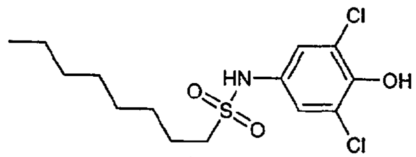 | Octane-1-sulfonic acid (3,5-dichloro-4-hydroxy-phenyl)-amide | C14H21Cl2NO3S | 354.29 | 354.5 |
| 172 | 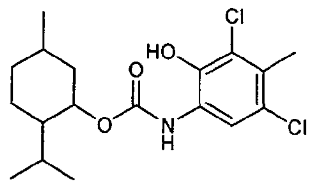 | (3,5-Dichloro-2-hydroxy-4-methyl-phenyl)-carbamic acid 2-isopropyl-5-methyl-cyclohexyl ester | C18H25Cl2NO3 | 374.31 | 374.4 |
| 173 | 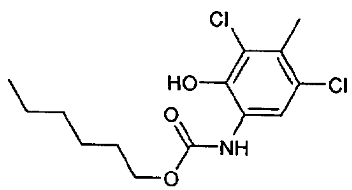 | (3,5-Dichloro-2-hydroxy-4-methyl-phenyl)-carbamic acid hexyl ester | C14H19Cl2NO3 | 320.22 | 320.2 |
| 174 | 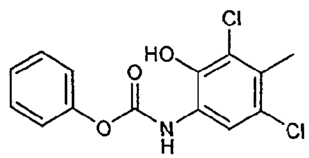 | (3,5-Dichloro-2-hydroxy-4-methyl-phenyl)-carbamic acid phenyl ester | C14H11Cl2NO3 | 312.15 | 320.2 |
| 175 | 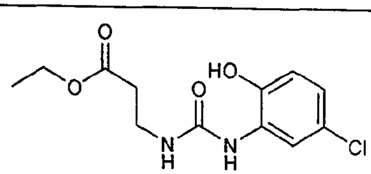 | 3-[3-(5-Chloro-2-hydroxy-phenyl)-ureido]-propionic acid ethyl ester | C13H9Cl2NO3 | 298.13 | 298.2 |

| | | | | | |
|---|---|---|---|---|---|
| 176 | 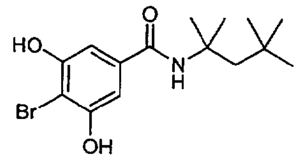 | 4-Bromo-3,5-dihydroxy-N-(1,1,3,3-tetramethyl-butyl)-benzamide | C15H22BrNO3 | 344.2 | 344.3 |
| 177 | 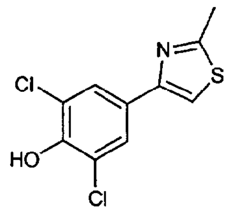 | 2,6-Dichloro-4-(2-methyl-thiazol-4-yl)-phenol | C10H7Cl2NOS | 260.1 | 260.4 |
| 178 | 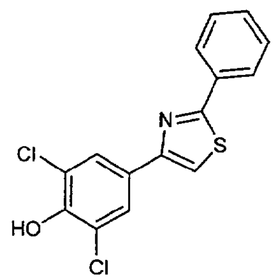 | 2,6-Dichloro-4-(2-phenyl-thiazol-4-yl)-phenol | C15H9Cl2NOS | 322.2 | 322.2 |
| 179 | 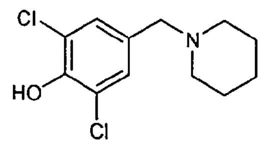 | 2,6-Dichloro-4-piperidin-1-ylmethyl-phenol | C12H15Cl2NO | 260.2 | 260.3 |
| 180 | 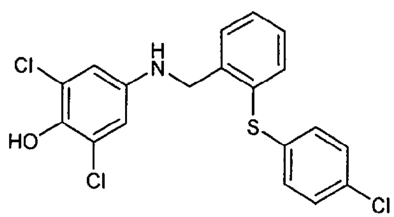 | 2,6-Dichloro-4-[2-(4-chloro-phenylsulfanyl)-benzylamino]-phenol | C19H14Cl3NOS | 410.7 | 409.9 |

| | | | | | |
|---|---|---|---|---|---|
| 181 | 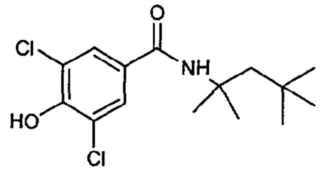 | 3,5-Dichloro-4-hydroxy-N-(1,1,3,3-tetramethyl-butyl)-benzamide | C15H21Cl2NO2 | 318.2 | 318.1 |
| 182 | 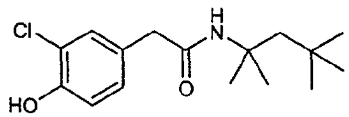 | 2-(3-Chloro-4-hydroxy-phenyl)-N-(1,1,3,3-tetramethyl-butyl)-acetamide | C16H24ClNO2 | 297.8 | 298.1 |
| 183 | 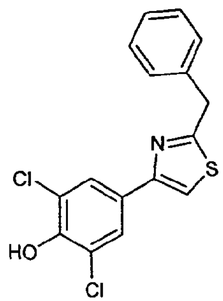 | 4-(2-Benzyl-thiazol-4-yl)-2,6-dichloro-phenol | C16H11Cl2NOS | 336.2 | 336.1 |
| 184 | 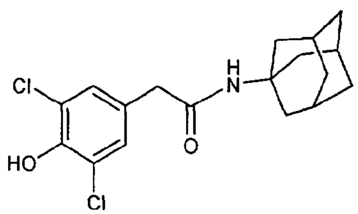 | N-Adamantan-1-yl-2-(3,5-dichloro-4-hydroxy-phenyl)-acetamide | C18H21Cl2NO2 | 354.3 | 354.1 |
| 185 | 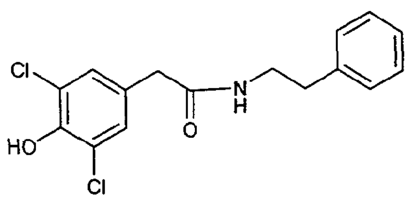 | 2-(3,5-Dichloro-4-hydroxy-phenyl)-N-phenethyl-acetamide | C16H15Cl2NO2 | 324.2 | 324.1 |

| | | | | | |
|---|---|---|---|---|---|
| 186 | 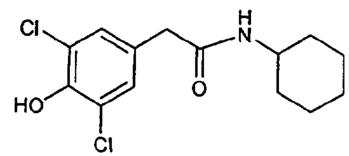 | N-Cyclohexyl-2-(3,5-dichloro-4-hydroxy-phenyl)-acetamide | C14H17Cl2NO2 | 302.2 | 302.1 |
| 187 | 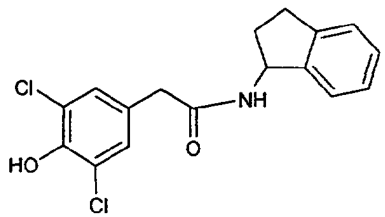 | 2-(3,5-Dichloro-4-hydroxy-phenyl)-N-indan-1-yl-acetamide | C17H15Cl2NO2 | 336.2 | 335.9 |
| 188 | 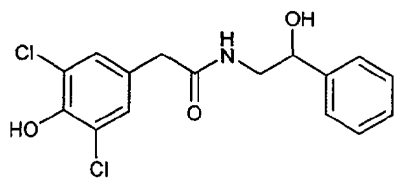 | 2-(3,5-Dichloro-4-hydroxy-phenyl)-N-(2-hydroxy-2-phenyl-ethyl)-acetamide | C16H15Cl2NO3 | 340.2 | 339.9 |
| 189 | 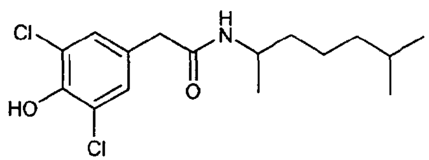 | 2-(3,5-Dichloro-4-hydroxy-phenyl)-N-(1,5-dimethyl-hexyl)-acetamide | C16H23Cl2NO2 | 332.3 | 332.1 |
| 190 | 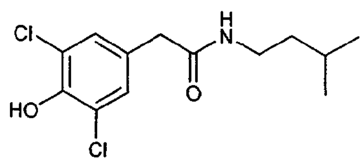 | 2-(3,5-Dichloro-4-hydroxy-phenyl)-N-(3-methyl-butyl)-acetamide | C13H17Cl2NO2 | 290.2 | 290.2 |

| | | | | | |
|---|---|---|---|---|---|
| 191 | 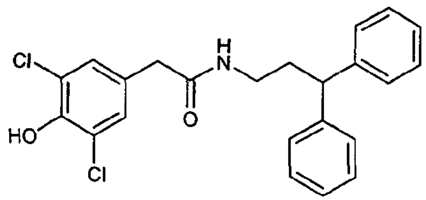 | 2-(3,5-Dichloro-4-hydroxy-phenyl)-N-(3,3-diphenyl-propyl)-acetamide | C23H21Cl2NO2 | 414.3 | 414.1 |
| 192 | 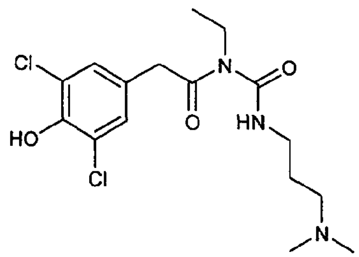 | 1-[2-(3,5-Dichloro-4-hydroxy-phenyl)-acetyl]-3-(3-dimethylamino-propyl)-1-ethyl-urea | C16H23Cl2N3O3 | 376.3 | 376.0 |
| 193 | 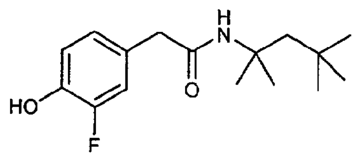 | 2-(3-Fluoro-4-hydroxy-phenyl)-N-(1,1,3,3-tetramethyl-butyl)-acetamide | C16H24FNO2 | 281.4 | 282.0 |
| 194 | 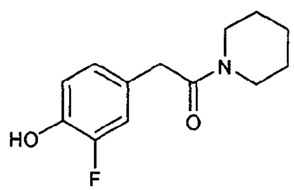 | 2-(3-Fluoro-4-hydroxy-phenyl)-1-piperidin-1-yl-ethanone | C13H16FNO2 | 237.3 | 238.2 |

| | | | | | |
|---|---|---|---|---|---|
| 195 | 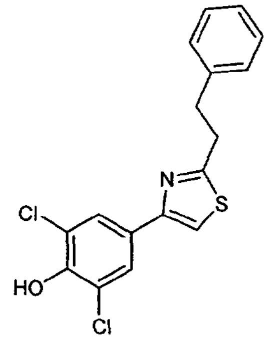 | 2,6-Dichloro-4-(2-phenethyl-thiazol-4-yl)-phenol | C17H13Cl2NOS | 350.3 | 350.2 |
| 196 | 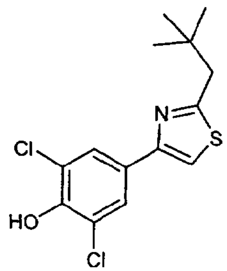 | 2,6-Dichloro-4-[2-(2,2-dimethyl-propyl)-thiazol-4-yl]-phenol | C14H15Cl2NOS | 316.2 | 316.1 |
| 197 | 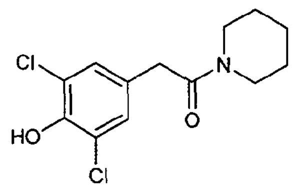 | 2-(3,5-Dichloro-4-hydroxy-phenyl)-1-piperidin-1-yl-ethanone | C13H15Cl2NO2 | 288.2 | 288.3 |
| 198 | 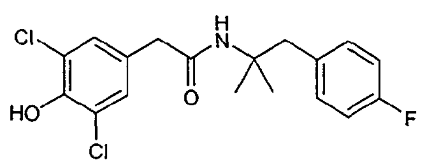 | 2-(3,5-Dichloro-4-hydroxy-phenyl)-N-[2-(4-fluoro-phenyl)-1,1-dimethyl-ethyl]-acetamide | C18H18Cl2FNO2 | 370.2 | 370.0 |

| | | | | | |
|---|---|---|---|---|---|
| 199 | 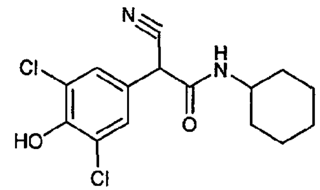 | 2-Cyano-N-cyclohexyl-2-(3,5-dichloro-4-hydroxy-phenyl)-acetamide | C15H16Cl2N2O2 | 327.2 | 327.1 |
| 200 | 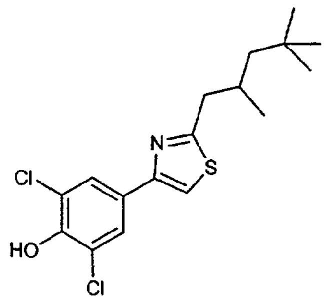 | 2,6-Dichloro-4-[2-(2,4,4-trimethyl-pentyl)-thiazol-4-yl]-phenol | C17H21Cl2NOS | 358.3 | 358.2 |
| 201 | 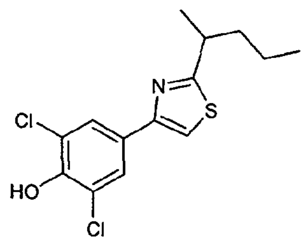 | 2,6-Dichloro-4-[2-(1-methyl-butyl)-thiazol-4-yl]-phenol | C14H15Cl2NOS | 316.2 | 316.2 |
| 202 | 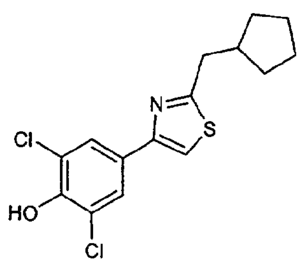 | 2,6-Dichloro-4-(2-cyclopentylmethyl-thiazol-4-yl)-phenol | C15H15Cl2NOS | 328.3 | 328.2 |

| | | | | | |
|---|---|---|---|---|---|
| 203 | 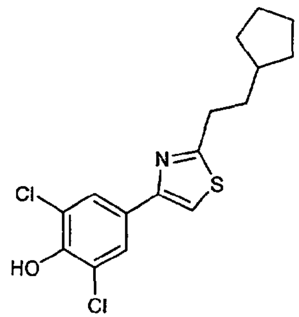 | 2,6-Dichloro-4-[2-(2-cyclopentyl-ethyl)-thiazol-4-yl]-phenol | C16H17Cl2NOS | 342.3 | 342.3 |
| 204 | 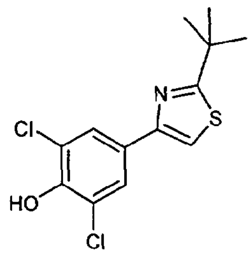 | 4-(2-tert-Butyl-thiazol-4-yl)-2,6-dichloro-phenol | C13H13Cl2NOS | 302.2 | 302.2 |
| 205 | 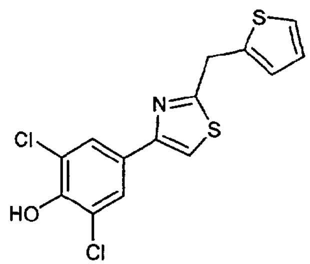 | 2,6-Dichloro-4-(2-thiophen-2-ylmethyl-thiazol-4-yl)-phenol | C14H9Cl2NOS2 | 342.3 | 342.1 |

| | | | | | |
|---|---|---|---|---|---|
| 206 | 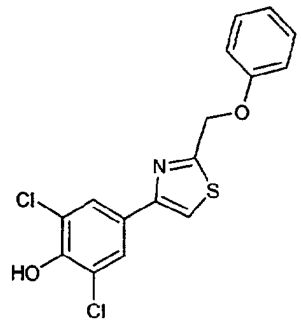 | 2,6-Dichloro-4-(2-phenoxymethyl-thiazol-4-yl)-phenol | C16H11Cl2NO2S | 352.2 | 352.2 |
| 207 | 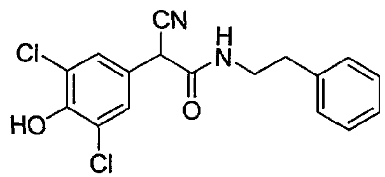 | 2-Cyano-2-(3,5-dichloro-4-hydroxy-phenyl)-N-phenethyl-acetamide | C17H14Cl2N2O2 | 349.2 | 349.0 |
| 208 | 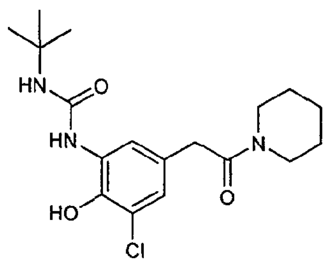 | 1-tert-Butyl-3-[3-chloro-2-hydroxy-5-(2-oxo-2-piperidin-1-yl-ethyl)-phenyl]-urea | C18H26ClN3O3 | 367.9 | 368.1 |
| 209 | 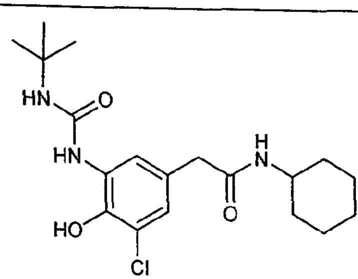 | 2-[3-(3-tert-Butyl-ureido)-5-chloro-4-hydroxy-phenyl]-N-cyclohexyl-acetamide | C19H28ClN3O3 | 381.9 | 382.1 |

| | | | | | |
|---|---|---|---|---|---|
| 210 | 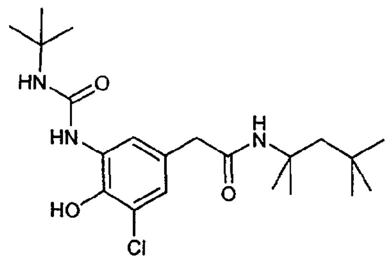 | 2-[3-(3-tert-Butyl-ureido)-5-chloro-4-hydroxy-phenyl]-N-(1,1,3,3-tetramethyl-butyl)-acetamide | C21H34ClN3O3 | 412.0 | 412.1 |
| 211 | 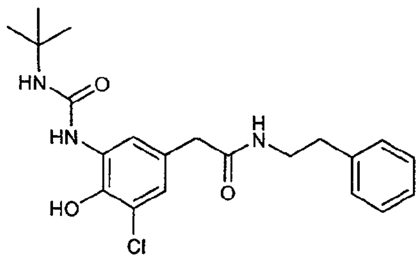 | 2-[3-(3-tert-Butyl-ureido)-5-chloro-4-hydroxy-phenyl]-N-phenethyl-acetamide | C21H26ClN3O3 | 403.9 | 404.1 |
| 212 | 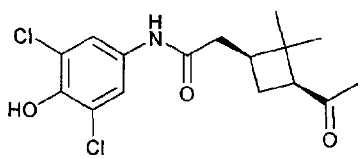 | 2-(3-Acetyl-2,2-dimethyl-cyclobutyl)-N-(3,5-dichloro-4-hydroxy-phenyl)-acetamide | C16H19Cl2NO3 | 344.23 | 344.4 |
| 213 | 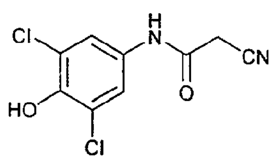 | 2-Cyano-N-(3,5-dichloro-4-hydroxy-phenyl)-acetamide | C9H6Cl2N2O2 | 245.06 | 245.2 |
| 214 | 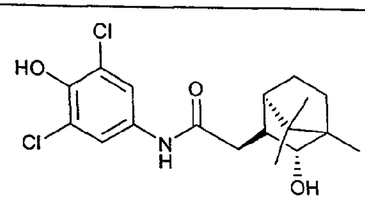 | N-(3,5-Dichloro-4-hydroxy-phenyl)-2-(3-hydroxy-4,7,7-trimethyl-bicyclo[2.2.1]hept-2- | C18H23Cl2NO3 | 372.29 | 372.2 |

| | | | | | |
|---|---|---|---|---|---|
| | | yl)-acetamide | | | |
| 215 | 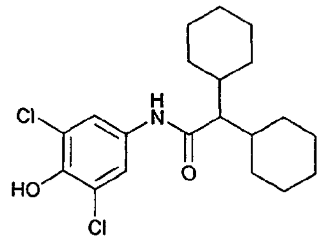 | 2,2-Dicyclohexyl-N-(3,5-dichloro-4-hydroxy-phenyl)-acetamide | C20H27Cl2NO2 | 384.34 | 384.0 |
| 216 | 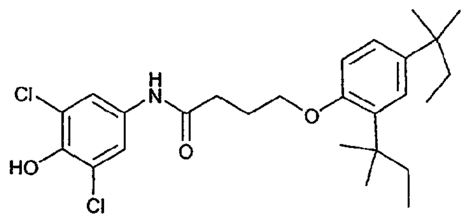 | 4-[2,4-Bis-(1,1-dimethyl-propyl)-phenoxy]-N-(3,5-dichloro-4-hydroxy-phenyl)-butyramide | C26H35Cl2NO3 | 480.47 | 480.5 |
| 217 | 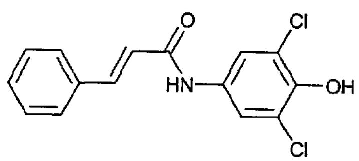 | N-(3,5-Dichloro-4-hydroxy-phenyl)-3-phenyl-acrylamide | C15H11Cl2NO2 | 308.16 | 308.1 |
| 218 | 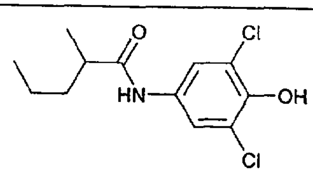 | 2-Methyl-pentanoic acid (3,5-dichloro-4-hydroxy-phenyl)-amide | C12H15Cl2NO2 | 276.16 | 276.3 |

| | | | | | |
|---|---|---|---|---|---|
| 219 | 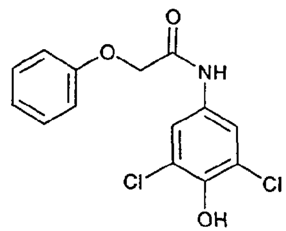 | N-(3,5-Dichloro-4-hydroxy-phenyl)-2-phenoxy-acetamide | C14H11Cl2NO3 | 312.15 | 312.0 |
| 220 | 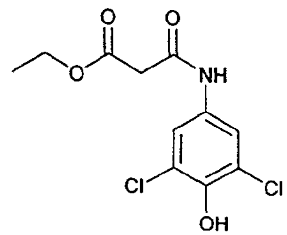 | N-(3,5-Dichloro-4-hydroxy-phenyl)-malonamic acid ethyl ester | C11H11Cl2NO4 | 292.12 | 292.1 |
| 221 | 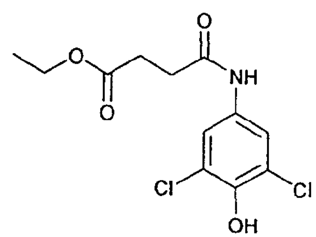 | N-(3,5-Dichloro-4-hydroxy-phenyl)-succinamic acid ethyl ester | C12H13Cl2NO4 | 306.14 | 306.0 |
| 222 | 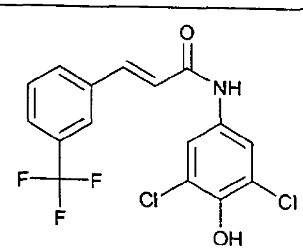 | N-(3,5-Dichloro-4-hydroxy-phenyl)-3-(3-trifluoromethyl-phenyl)-acrylamide | C16H10Cl2F3NO2 | 376.16 | 376.2 |

| | | | | | |
|---|---|---|---|---|---|
| 223 | 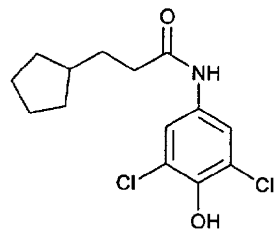 | 3-Cyclopentyl-N-(3,5-dichloro-4-hydroxy-phenyl)-propionamide | C14H17Cl2NO2 | 302.20 | 302.2 |
| 224 | 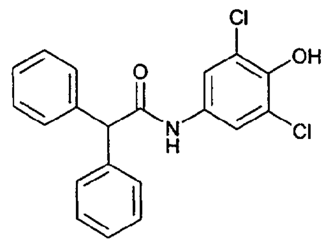 | N-(3,5-Dichloro-4-hydroxy-phenyl)-2,2-diphenyl-acetamide | C20H15Cl2NO2 | 372.24 | 372.1 |
| 225 | 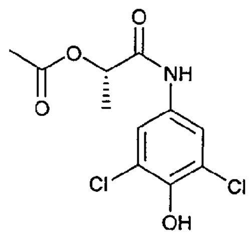 | Acetic acid 1-(3,5-dichloro-4-hydroxy-phenylcarbamoyl)-ethyl ester | C11H11Cl2NO4 | 292.12 | 292.1 |
| 226 | 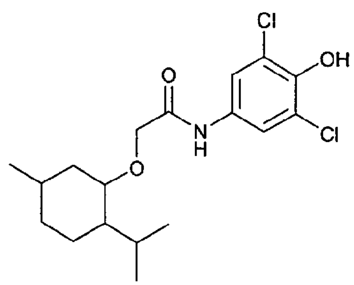 | N-(3,5-Dichloro-4-hydroxy-phenyl)-2-(2-isopropyl-5-methyl-cyclohexyloxy)-acetamide | C18H25Cl2NO3 | 374.30 | 374.8 |

| | | | | | |
|---|---|---|---|---|---|
| 227 | 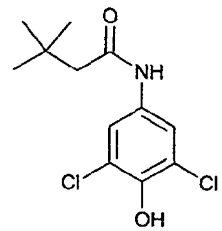 | N-(3,5-Dichloro-4-hydroxy-phenyl)-3,3-dimethyl-butyramide | C12H15Cl2NO2 | 276.16 | 276.4 |
| 228 | 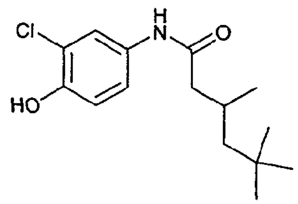 | 3,5,5-Trimethyl-hexanoic acid (3-chloro-4-hydroxy-phenyl)-amide | C15H22ClNO2 | 283.79 | 284.4 |
| 229 | 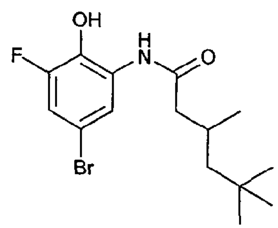 | 3,5,5-Trimethyl-hexanoic acid (5-bromo-3-fluoro-2-hydroxy-phenyl)-amide | C15H21BrFNO2 | 346.24 | 346.5 |
| 230 | 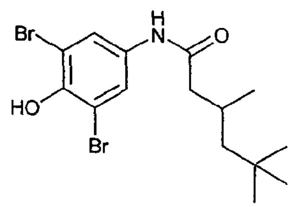 | 3,5,5-Trimethyl-hexanoic acid (3,5-dibromo-4-hydroxy-phenyl)-amide | C15H21Br2NO2 | 407.14 | 408.3 |

| | | | | | |
|---|---|---|---|---|---|
| 231 | 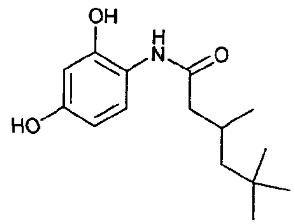 | 3,5,5-Trimethyl-hexanoic acid (2,4-dihydroxy-phenyl)-amide | C15H23NO3 | 265.35 | 266.3 |
| 232 | 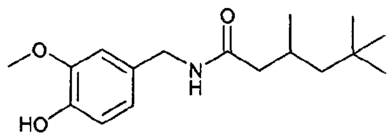 | 3,5,5-Trimethyl-hexanoic acid 4-hydroxy-3-methoxy-benzylamide | C17H27NO3 | 293.40 | 294.3 |
| 233 | 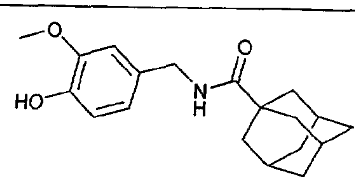 | Adamantane-1-carboxylic acid 4-hydroxy-3-methoxy-benzylamide | C19H25NO3 | 315.41 | 316.3 |
| 234 | 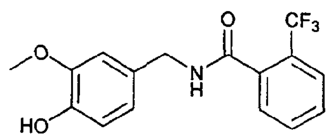 | N-(4-Hydroxy-3-methoxy-benzyl)-2-trifluoromethyl-benzamide | C16H14F3NO3 | 325.28 | 326.2 |
| 235 | 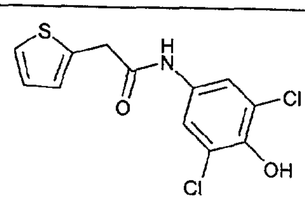 | N-(3,5-Dichloro-4-hydroxy-phenyl)-2-thiophen-2-yl-acetamide | C12H9Cl2NO2S | 302.18 | 302.0 |

| | | | | | |
|---|---|---|---|---|---|
| 236 | 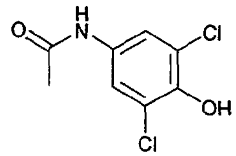 | N-(3,5-Dichloro-4-hydroxy-phenyl)-acetamide | C8H7Cl2NO2 | 220.05 | 220.2 |
| 237 | 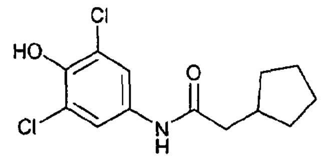 | 2-Cyclopentyl-N-(3,5-dichloro-4-hydroxy-phenyl)-acetamide | C13H15Cl2NO2 | 288.17 | 288.5 |
| 238 | 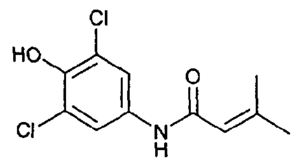 | 3-Methyl-but-2-enoic acid (3,5-dichloro-4-hydroxy-phenyl)-amide | C11H11Cl2NO2 | 260.12 | 260.1 |
| 239 | 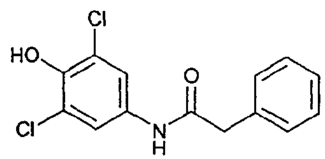 | N-(3,5-Dichloro-4-hydroxy-phenyl)-2-phenyl-acetamide | C14H11Cl2NO2 | 296.15 | 296.1 |
| 240 | 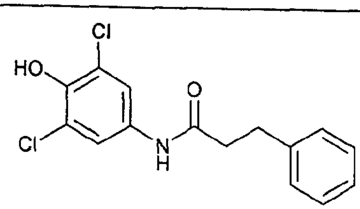 | N-(3,5-Dichloro-4-hydroxy-phenyl)-3-phenyl-propionamide | C15H13Cl2NO2 | 310.18 | 310.1 |
| 241 | 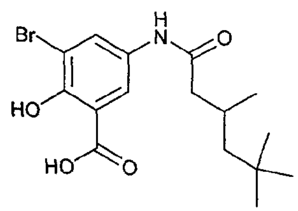 | 3-Bromo-2-hydroxy-5-(3,5,5-trimethyl-hexanoylamino)-benzoic acid | C16H22BrNO4 | 372.25 | 372.5 |

| | | | | | |
|---|---|---|---|---|---|
| 242 | 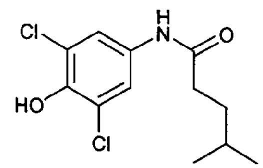 | 4-Methyl-pentanoic acid (3,5-dichloro-4-hydroxy-phenyl)-amide | C12H15Cl2NO2 | 276.16 | 276.4 |
| 243 | 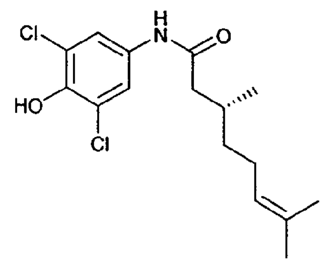 | 3,7-Dimethyl-oct-6-enoic acid (3,5-dichloro-4-hydroxy-phenyl)-amide | C16H21Cl2NO2 | 330.25 | 330.7 |
| 244 | 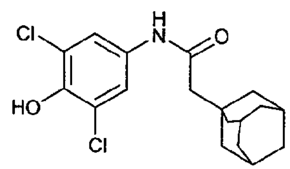 | 2-Adamantan-1-yl-N-(3,5-dichloro-4-hydroxy-phenyl)-acetamide | C18H21Cl2NO2 | 354.27 | 354.4 |
| 245 | 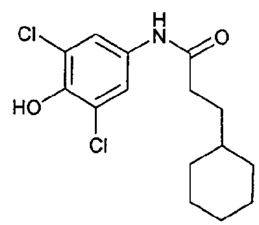 | 3-Cyclohexyl-N-(3,5-dichloro-4-hydroxy-phenyl)-propionamide | C15H19Cl2NO2 | 316.22 | 316.5 |

| | | | | | |
|---|---|---|---|---|---|
| 246 | 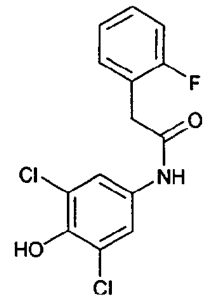 | N-(3,5-Dichloro-4-hydroxy-phenyl)-2-(2-fluoro-phenyl)-acetamide | C14H10Cl2FNO2 | 314.14 | 314.5 |
| 247 | 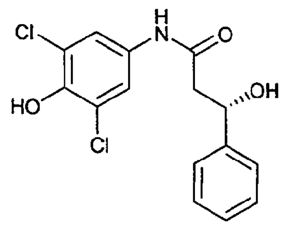 | N-(3,5-Dichloro-4-hydroxy-phenyl)-3-hydroxy-3-phenyl-propionamide | C15H13Cl2NO3 | 326.17 | 326.2 |
| 248 | 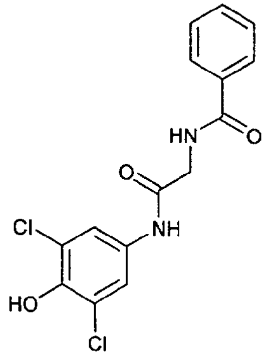 | N-[(3,5-Dichloro-4-hydroxy-phenylcarbamoyl)-methyl]-benzamide | C15H12Cl2N2O3 | 339.17 | 339.2 |
| 249 | 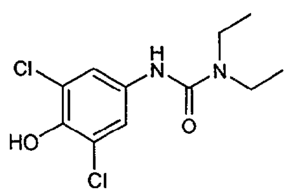 | 3-(3,5-Dichloro-4-hydroxy-phenyl)-1,1-diethyl-urea | C11H14Cl2N2O2 | 277.31 | 277.2 |

| | | | | | |
|---|---|---|---|---|---|
| 250 | 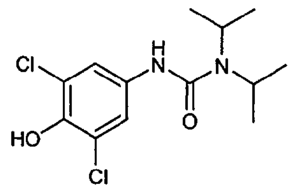 | 3-(3,5-Dichloro-4-hydroxy-phenyl)-1,1-diisopropyl-urea | C13H18Cl2N2O2 | 305.22 | 305.3 |
| 251 | 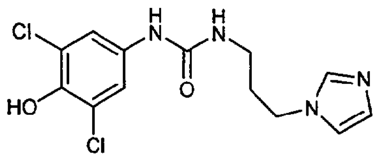 | 1-(3,5-Dichloro-4-hydroxy-phenyl)-3-(3-imidazol-1-yl-propyl)-urea | C13H14Cl2N4O2 | 329.31 | 329.1 |
| 252 | 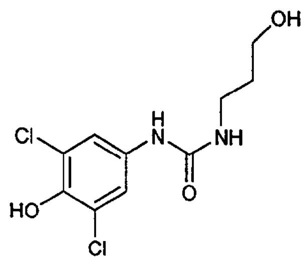 | 1-(3,5-Dichloro-4-hydroxy-phenyl)-3-(3-hydroxy-propyl)-urea | C10H12Cl2N2O3 | 278.02 | 279.1 |
| 253 | 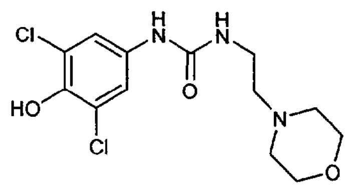 | 1-(3,5-Dichloro-4-hydroxy-phenyl)-3-(2-morpholin-4-yl-ethyl)-urea | C13H17Cl2N3O3 | 334.20 | 334.1 |
| 254 | 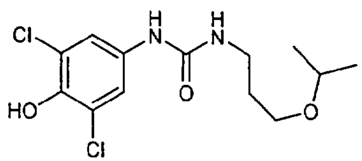 | 1-(3,5-Dichloro-4-hydroxy-phenyl)-3-(3-isopropoxy-propyl)-urea | C13H18Cl2N2O3 | 321.20 | 321.1 |

| | | | | | |
|---|---|---|---|---|---|
| 255 | 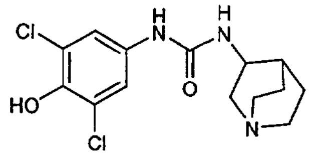 | 1-(1-Aza-bicyclo[2.2.2]oct-3-yl)-3-(3,5-dichloro-4-hydroxy-phenyl)-urea | C14H17Cl2N3O2 | 330.21 | 330.1 |
| 256 | 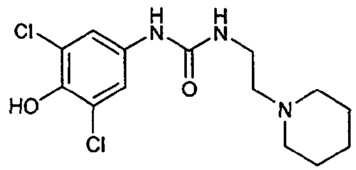 | 1-(3,5-Dichloro-4-hydroxy-phenyl)-3-(2-piperidin-1-yl-ethyl)-urea | C14H19Cl2N3O2 | 332.23 | 332.1 |
| 257 | 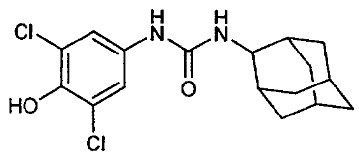 | 1-Adamantan-2-yl-3-(3,5-dichloro-4-hydroxy-phenyl)-urea | C17H20Cl2N2O2 | 355.26 | 355.1 |
| 258 | 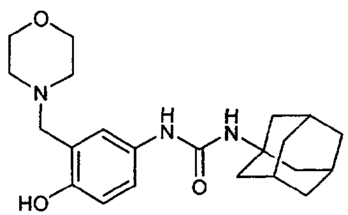 | 1-Adamantan-1-yl-3-(4-hydroxy-3-morpholin-4-ylmethyl-phenyl)-urea | C22H31N3O3 | 385.51 | 386.3 |
| 259 | 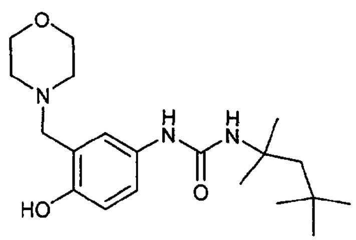 | 1-(4-Hydroxy-3-morpholin-4-ylmethyl-phenyl)-3-(1,1,3,3-tetramethyl-butyl)-urea | C20H33N3O3 | 363.49 | 364.3 |

| | | | | | |
|---|---|---|---|---|---|
| 260 | 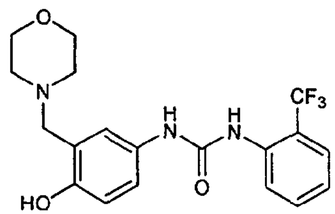 | 1-(4-Hydroxy-3-morpholin-4-ylmethyl-phenyl)-3-(2-trifluoromethyl-phenyl)-urea | C19H20F3N3O3 | 395.38 | 396.3 |
| 261 | 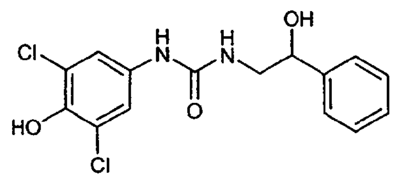 | 1-(3,5-Dichloro-4-hydroxy-phenyl)-3-(2-hydroxy-2-phenyl-ethyl)-urea | C15H14Cl2N2O3 | 341.19 | 341.1 |
| 262 | 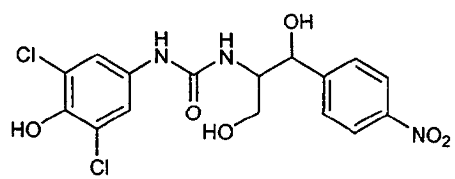 | 1-(3,5-Dichloro-4-hydroxy-phenyl)-3-[2-hydroxy-1-hydroxymethyl-2-(4-nitro-phenyl)-ethyl]-urea | C16H15Cl2N3O6 | 416.21 | 416.1 |
| 263 | 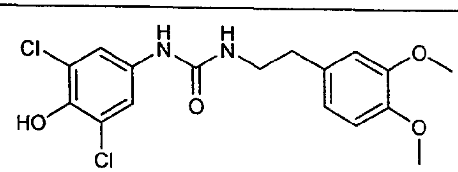 | 1-(3,5-Dichloro-4-hydroxy-phenyl)-3-[2-(3,4-dimethoxy-phenyl)-ethyl]-urea | C17H18Cl2N2O4 | 385.24 | 385.1 |
| 264 | 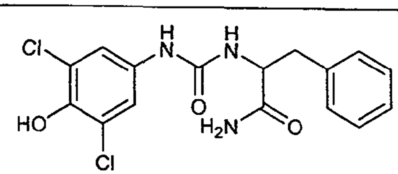 | 2-[3-(3,5-Dichloro-4-hydroxy-phenyl)-ureido]-3-phenyl-propionamide | C16H15Cl2N3O3 | 368.21 | 368.1 |

| | | | | | |
|---|---|---|---|---|---|
| 265 | 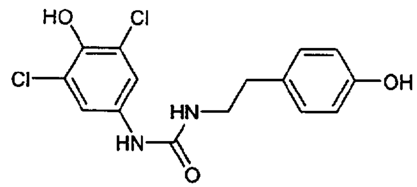 | 1-(3,5-Dichloro-4-hydroxy-phenyl)-3-[2-(4-hydroxy-phenyl)-ethyl]-urea | C15H14Cl2N2O3 | 341.28 | 341.1 |
| 266 | 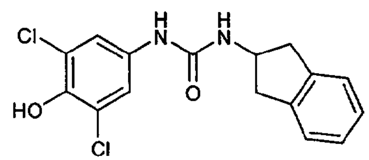 | 1-(3,5-Dichloro-4-hydroxy-phenyl)-3-indan-2-yl-urea | C16H14Cl2N2O2 | 337.20 | 337.1 |
| 267 | 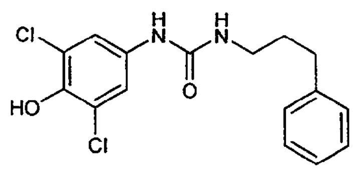 | 1-(3,5-Dichloro-4-hydroxy-phenyl)-3-(3-phenyl-propyl)-urea | C16H16Cl2N2O2 | 339.22 | 339.1 |
| 268 | 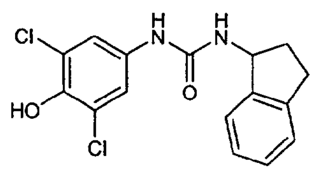 | 1-(3,5-Dichloro-4-hydroxy-phenyl)-3-indan-1-yl-urea | C16H14Cl2N2O2 | 337.20 | 337.1 |
| 269 | 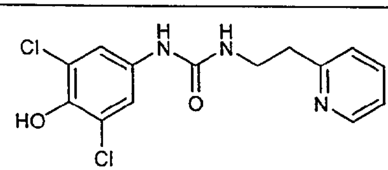 | 1-(3,5-Dichloro-4-hydroxy-phenyl)-3-(2-pyridin-2-yl-ethyl)-urea | C14H13Cl2N3O2 | 326.18 | 326.1 |
| 270 | 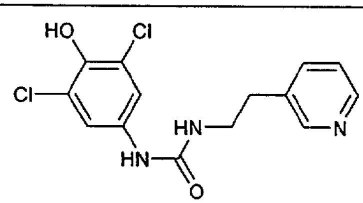 | 1-(3,5-Dichloro-4-hydroxy-phenyl)-3-(2-pyridin-3-yl-ethyl)-urea | C14H13Cl2N3O2 | 326.18 | 326.1 |

| | | | | | |
|---|---|---|---|---|---|
| 271 | 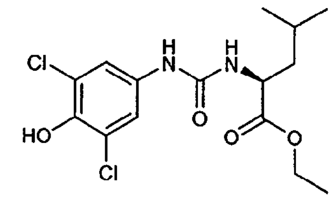 | 2-[3-(3,5-Dichloro-4-hydroxy-phenyl)-ureido]-4-methyl-pentanoic acid ethyl ester | C15H20Cl2N2O4 | 363.24 | 363.1 |
| 272 | 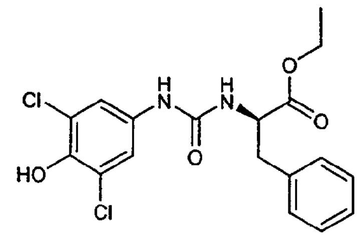 | 2-[3-(3,5-Dichloro-4-hydroxy-phenyl)-ureido]-3-phenyl-propionic acid ethyl ester | C18H18Cl2N2O4 | 397.24 | 397.1 |
| 273 | 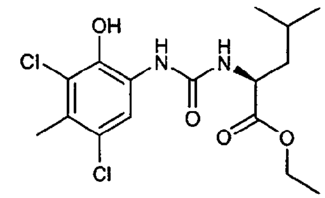 | 2-[3-(3,5-Dichloro-2-hydroxy-4-methyl-phenyl)-ureido]-4-methyl-pentanoic acid ethyl ester | C16H22Cl2N2O4 | 377.26 | 377.1 |
| 274 | 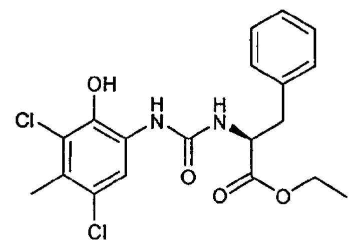 | 2-[3-(3,5-Dichloro-2-hydroxy-4-methyl-phenyl)-ureido]-3-phenyl-propionic acid ethyl ester | C19H20Cl2N2O4 | 411.28 | 411.1 |
| 275 | 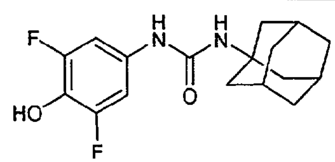 | 1-Adamantan-1-yl-3-(3,5-difluoro-4-hydroxy-phenyl)-urea | C17H20F2N2O2 | 322.25 | 323.2 |

| | | | | | |
|---|---|---|---|---|---|
| 276 | 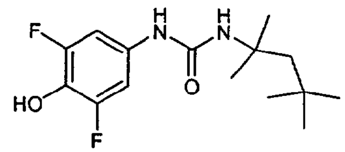 | 1-(3,5-Difluoro-4-hydroxy-phenyl)-3-(1,1,3,3-tetramethyl-butyl)-urea | C15H22F2N2O2 | 300.34 | 301.3 |
| 277 | 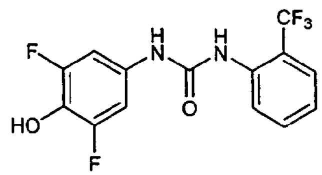 | 1-(3,5-Difluoro-4-hydroxy-phenyl)-3-(2-trifluoromethyl-phenyl)-urea | C14H9F5N2O2 | 332.23 | 333.2 |
| 278 | 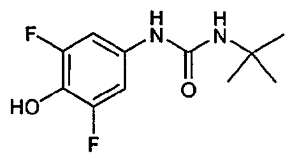 | 1-tert-Butyl-3-(3,5-difluoro-4-hydroxy-phenyl)-urea | C11H14F2N2O2 | 244.24 | 245.2 |
| 279 | 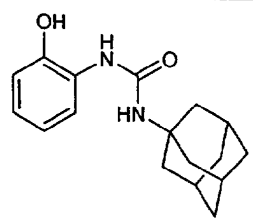 | 1-Adamantan-1-yl-3-(2-hydroxy-phenyl)-urea | C17H22N2O2 | 286.37 | 287.3 |
| 280 | 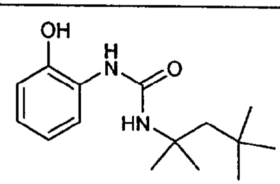 | 1-(2-Hydroxy-phenyl)-3-(1,1,3,3-tetramethyl-butyl)-urea | C15H24N2O2 | 264.36 | 265.3 |

| | | | | | |
|---|---|---|---|---|---|
| 281 | 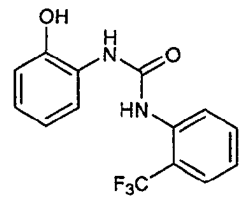 | 1-(2-Hydroxy-phenyl)-3-(2-trifluoromethyl-phenyl)-urea | C14H11F3N2O2 | 296.24 | 297.3 |
| 282 | 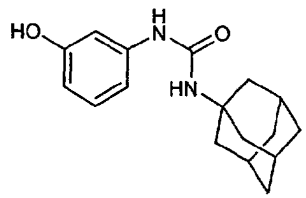 | 1-Adamantan-1-yl-3-(3-hydroxy-phenyl)-urea | C17H22N2O2 | 286.37 | 287.3 |
| 283 | 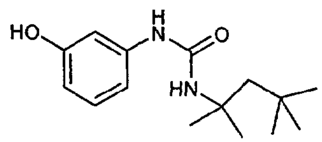 | 1-(3-Hydroxy-phenyl)-3-(1,1,3,3-tetramethyl-butyl)-urea | C15H24N2O2 | 264.36 | 265.3 |
| 284 | 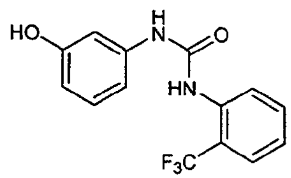 | 1-(3-Hydroxy-phenyl)-3-(2-trifluoromethyl-phenyl)-urea | C14H11F3N2O2 | 296.24 | 297.3 |
| 285 | 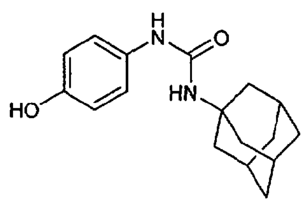 | 1-Adamantan-1-yl-3-(3-hydroxy-phenyl)-urea | C17H22N2O2 | 286.37 | 287.3 |

| | | | | | |
|---|---|---|---|---|---|
| 286 | 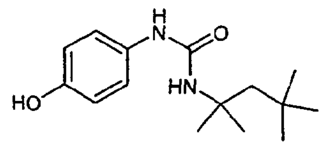 | 1-(3-Hydroxy-phenyl)-3-(1,1,3,3-tetramethyl-butyl)-urea | C15H24N2O2 | 264.36 | 265.3 |
| 287 | 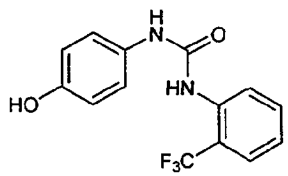 | 1-(3-Hydroxy-phenyl)-3-(2-trifluoromethyl-phenyl)-urea | C14H11F3N2O2 | 296.24 | 297.3 |
| 288 | 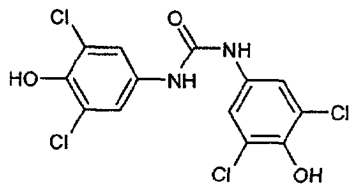 | 1,3-Bis-(3,5-dichloro-4-hydroxy-phenyl)-urea | C13H8Cl4N2O3 | 382.03 | 382.9 |
| 289 | 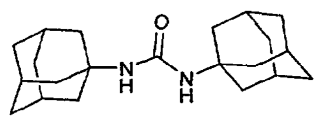 | 1,3-Di-adamantan-1-yl-urea | C21H32N2O | 328.49 | 329.3 |
| 290 | 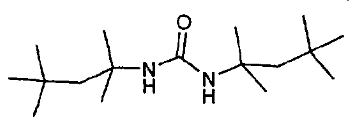 | 1,3-Bis-(1,1,3,3-tetramethyl-butyl)-urea | C17H36N2O | 284.48 | 285.3 |

| | | | | | |
|---|---|---|---|---|---|
| 291 | 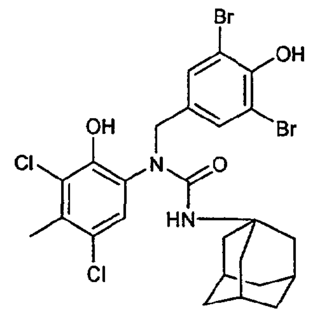 | 3-Adamantan-1-yl-1-(3,5-dibromo-4-hydroxy-benzyl)-1-(3,5-dichloro-2-hydroxy-4-methyl-phenyl)-urea | C25H26Br2Cl2N2O3 | 633.20 | 633.0 |
| 292 | 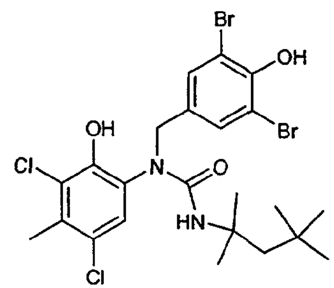 | 1-(3,5-Dibromo-4-hydroxy-benzyl)-1-(3,5-dichloro-2-hydroxy-4-methyl-phenyl)-3-(1,1,3,3-tetramethyl-butyl)-urea | C23H28Br2Cl2N2O3 | 611.19 | 610.3 |
| 293 | 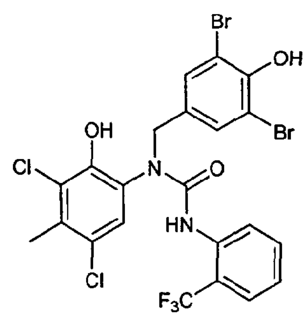 | 1-(3,5-Dibromo-4-hydroxy-benzyl)-1-(3,5-dichloro-2-hydroxy-4-methyl-phenyl)-3-(2-trifluoromethyl-phenyl)-urea | C22H15Br2Cl2F3N2O3 | 643.08 | 644.8 |

| | | | | | |
|---|---|---|---|---|---|
| 294 | 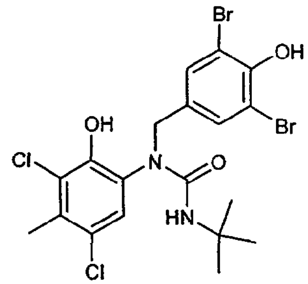 | 3-tert-Butyl-1-(3,5-dibromo-4-hydroxy-benzyl)-1-(3,5-dichloro-2-hydroxy-4-methyl-phenyl)-urea | C19H20Br2Cl2N2O3 | 555.05 | 554.9 |
| 295 | 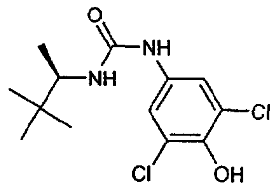 | 1-(3,5-Dichloro-4-hydroxy-phenyl)-3-(1,2,2-trimethyl-propyl)-urea | C13H18Cl2N2O2 | 305.20 | 305.1 |
| 296 | 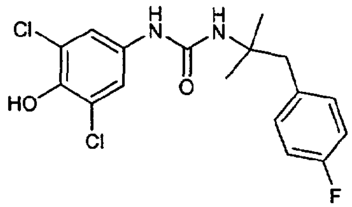 | 1-(3,5-Dichloro-4-hydroxy-phenyl)-3-[2-(4-fluoro-phenyl)-1,1-dimethyl-ethyl]-urea | C17H17Cl2FN2O2 | 371.23 | 371.1 |
| 297 | 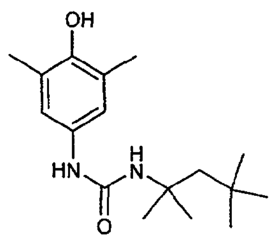 | 1-(4-Hydroxy-3,5-dimethyl-phenyl)-3-(1,1,3,3-tetramethyl-butyl)-urea | C17H28N2O2 | 292.42 | 293.3 |

| | | | | | |
|---|---|---|---|---|---|
| 298 | 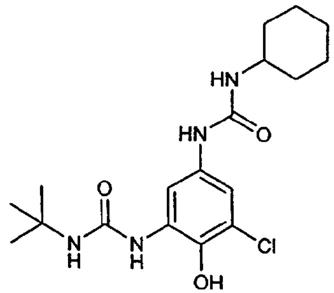 | 1-tert-Butyl-3-[3-chloro-5-(3-cyclohexyl-ureido)-2-hydroxy-phenyl]-urea | C18H27ClN4O3 | 382.88 | 383.4 |
| 299 | 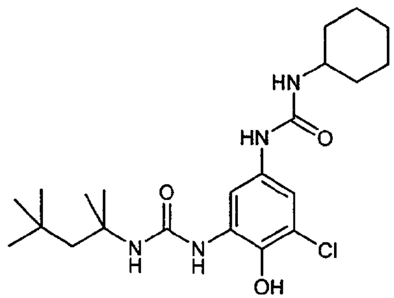 | 1-[3-Chloro-5-(3-cyclohexyl-ureido)-2-hydroxy-phenyl]-3-(1,1,3,3-tetramethyl-butyl)-urea | C22H35ClN4O3 | 438.99 | 439.8 |
| 300 | 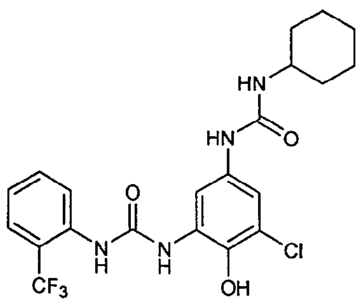 | 1-[3-Chloro-5-(3-cyclohexyl-ureido)-2-hydroxy-phenyl]-3-(2-trifluoromethyl-phenyl)-urea | C21H22ClF3N4O3 | 470.87 | 471.7 |

| | | | | | |
|---|---|---|---|---|---|
| 301 | 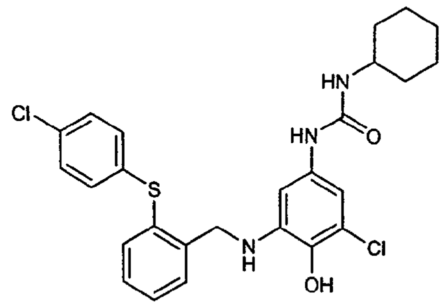 | 1-{3-Chloro-5-[2-(4-chloro-phenylsulfanyl)-benzylamino]-4-hydroxy-phenyl}-3-cyclohexyl-urea | $C_{26}H_{27}Cl_2N_3O_2S$ | 516.48 | 517.4 |
| 302 | 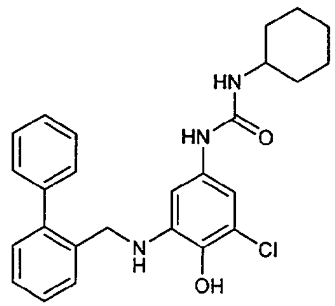 | 1-{3-[(Biphenyl-2-ylmethyl)-amino]-5-chloro-4-hydroxy-phenyl}-3-cyclohexyl-urea | $C_{26}H_{28}ClN_3O_2$ | 449.97 | 450.9 |
| 303 | 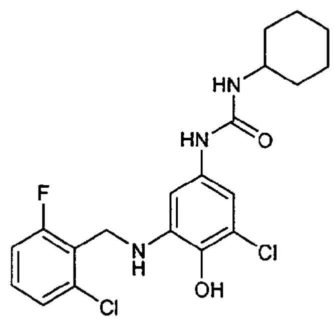 | 1-[3-Chloro-5-(2-chloro-6-fluoro-benzylamino)-4-hydroxy-phenyl]-3-cyclohexyl-urea | $C_{20}H_{22}Cl_2FN_3O_2$ | 426.31 | 426.4 |

| | | | | | |
|---|---|---|---|---|---|
| 304 | 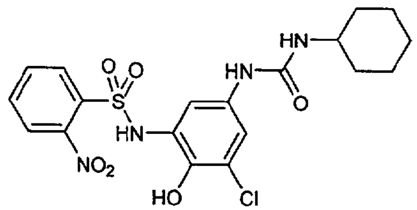 | N-[3-Chloro-5-(3-cyclohexyl-ureido)-2-hydroxy-phenyl]-2-nitro-benzenesulfonamide | C19H21ClN4O6S | 468.91 | 469.8 |
| 305 | 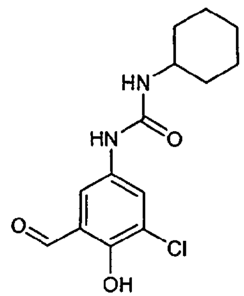 | 1-(3-Chloro-5-formyl-4-hydroxy-phenyl)-3-cyclohexyl-urea | C14H17ClN2O3 | 296.75 | 296.8 |
| 306 | 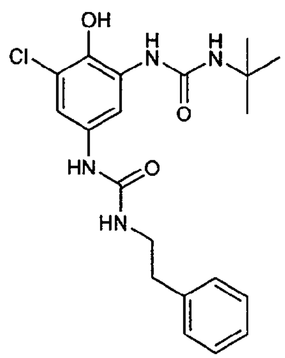 | 1-tert-Butyl-3-[3-chloro-2-hydroxy-5-(3-phenethyl-ureido)-phenyl]-urea | C20H25ClN4O3 | 404.89 | 404.9 |

| | | | | | |
|---|---|---|---|---|---|
| 307 | 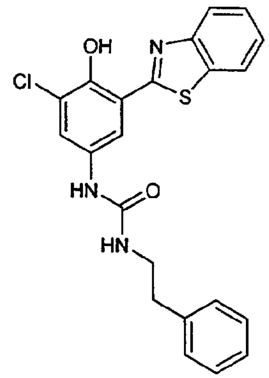 | 1-(3-Benzothiazol-2-yl-5-chloro-4-hydroxy-phenyl)-3-phenethyl-urea | C22H18ClN3O2S | 423.92 | 423.9 |
| 308 | 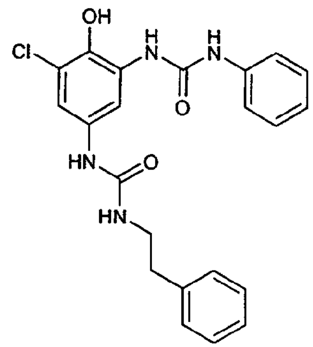 | 1-[3-Chloro-2-hydroxy-5-(3-phenethyl-ureido)-phenyl]-3-phenyl-urea | C22H21ClN4O3 | 424.88 | 424.9 |
| 309 | 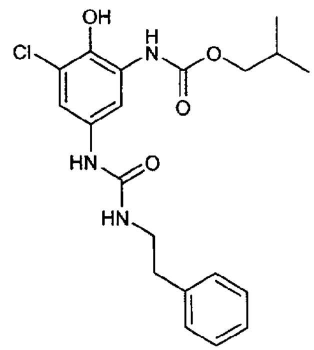 | [3-Chloro-2-hydroxy-5-(3-phenethyl-ureido)-phenyl]-carbamic acid isobutyl ester | C20H24ClN3O4 | 405.88 | 406.8 |

| | | | | | |
|---|---|---|---|---|---|
| 310 | 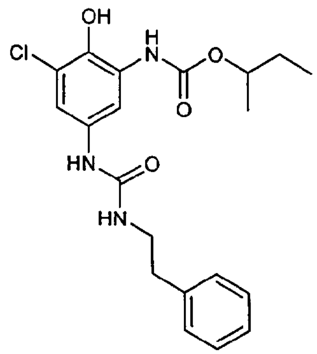 | [3-Chloro-2-hydroxy-5-(3-phenethyl-ureido)-phenyl]-carbamic acid sec-butyl ester | C20H24ClN3O4 | 405.88 | 406.8 |
| 311 | 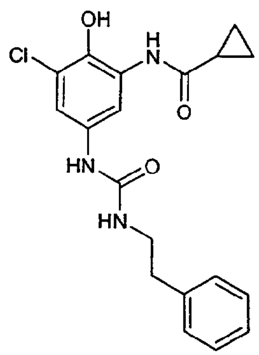 | Cyclopropanecarboxylic acid [3-chloro-2-hydroxy-5-(3-phenethyl-ureido)-phenyl]-amide | C19H20ClN3O3 | 373.83 | 373.8 |
| 312 | 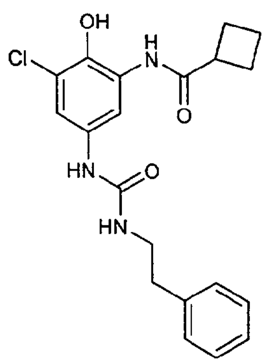 | Cyclobutanecarboxylic acid [3-chloro-2-hydroxy-5-(3-phenethyl-ureido)-phenyl]-amide | C20H22ClN3O3 | 387.86 | 388.8 |

| | | | | | |
|---|---|---|---|---|---|
| 313 | 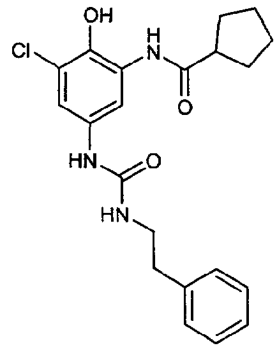 | Cyclopentanecarboxylic acid [3-chloro-2-hydroxy-5-(3-phenethyl-ureido)-phenyl]-amide | C21H24ClN3O3 | 401.89 | 401.9 |
| 314 | 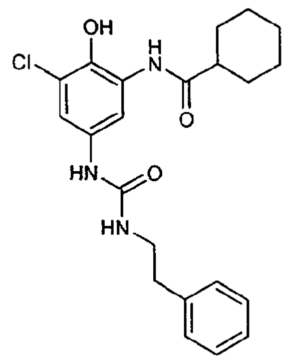 | Cyclohexanecarboxylic acid [3-chloro-2-hydroxy-5-(3-phenethyl-ureido)-phenyl]-amide | C22H26ClN3O3 | 415.92 | 415.9 |
| 315 | 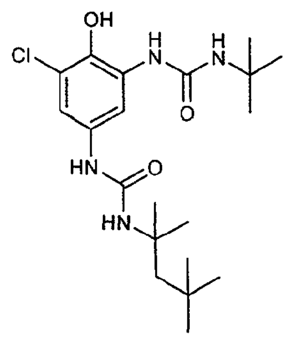 | 1-tert-Butyl-3-{3-chloro-2-hydroxy-5-[3-(1,1,3,3-tetramethyl-butyl)-ureido]-phenyl}-urea | C20H33ClN4O3 | 412.95 | 413.8 |

| | | | | | |
|---|---|---|---|---|---|
| 316 | 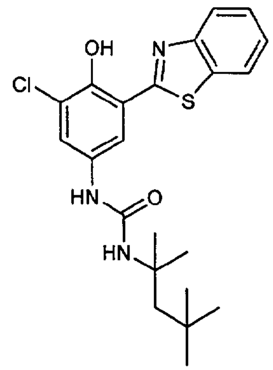 | 1-(3-Benzothiazol-2-yl-5-chloro-4-hydroxy-phenyl)-3-(1,1,3,3-tetramethyl-butyl)-urea | C22H26ClN3O2S | 431.98 | 432.8 |
| 317 | 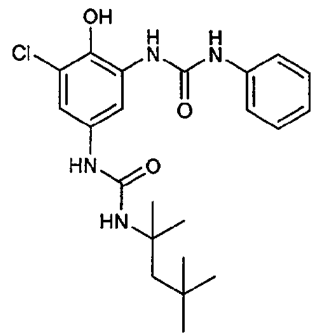 | 1-{3-Chloro-2-hydroxy-5-[3-(1,1,3,3-tetramethyl-butyl)-ureido]-phenyl}-3-phenyl-urea | C22H29ClN4O3 | 432.94 | 432.8 |
| 318 | 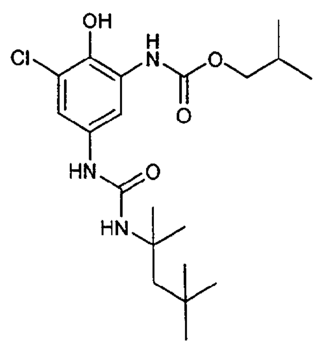 | {3-Chloro-2-hydroxy-5-[3-(1,1,3,3-tetramethyl-butyl)-ureido]-phenyl}-carbamic acid isobutyl ester | C20H32ClN3O4 | 413.94 | 413.8 |

| | | | | | |
|---|---|---|---|---|---|
| 319 | 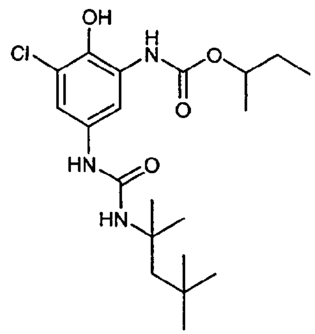 | {3-Chloro-2-hydroxy-5-[3-(1,1,3,3-tetramethyl-butyl)-ureido]-phenyl}-carbamic acid sec-butyl ester | C20H32ClN3O4 | 413.94 | 414.1 |
| 320 | 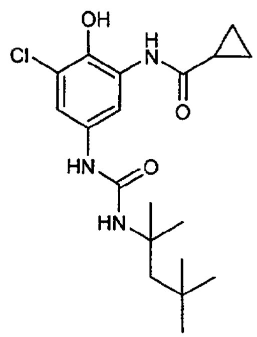 | Cyclopropanecarboxylic acid {3-chloro-2-hydroxy-5-[3-(1,1,3,3-tetramethyl-butyl)-ureido]-phenyl}-amide | C19H28ClN3O3 | 381.90 | 381.9 |
| 321 | 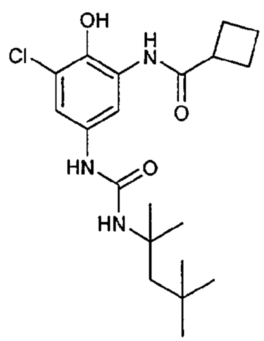 | Cyclobutanecarboxylic acid {3-chloro-2-hydroxy-5-[3-(1,1,3,3-tetramethyl-butyl)-ureido]-phenyl}-amide | C20H30ClN3O3 | 395.92 | 395.9 |

| | | | | | |
|---|---|---|---|---|---|
| 322 | 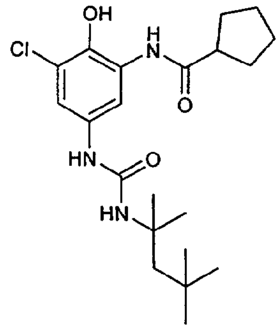 | Cyclopentanecarboxylic acid {3-chloro-2-hydroxy-5-[3-(1,1,3,3-tetramethyl-butyl)-ureido]-phenyl}-amide | C21H32ClN3O3 | 409.95 | 410.1 |
| 323 | 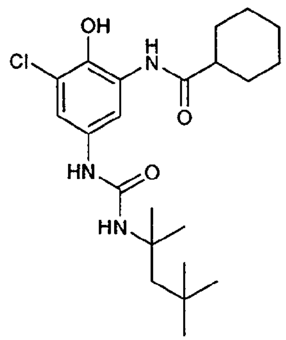 | Cyclohexanecarboxylic acid {3-chloro-2-hydroxy-5-[3-(1,1,3,3-tetramethyl-butyl)-ureido]-phenyl}-amide | C22H34ClN3O3 | 423.98 | 424.0 |
| 324 | 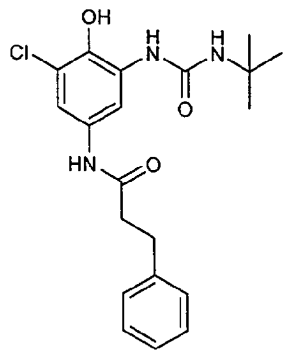 | N-[3-(3-tert-Butyl-ureido)-5-chloro-4-hydroxy-phenyl]-3-phenyl-propionamide | C20H24ClN3O3 | 389.88 | 389.9 |

| | | | | | |
|---|---|---|---|---|---|
| 325 | 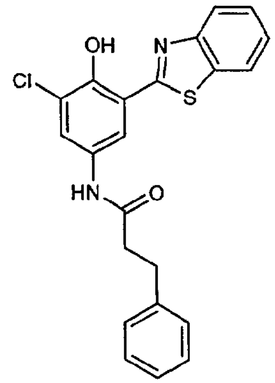 | N-(3-Benzothiazol-2-yl-5-chloro-4-hydroxy-phenyl)-3-phenyl-propionamide | C22H17ClN2O2S | 408.90 | 408.9 |
| 326 | 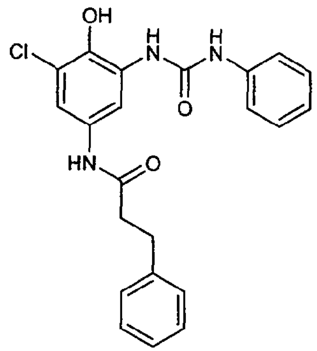 | N-[3-Chloro-4-hydroxy-5-(3-phenyl-ureido)-phenyl]-3-phenyl-propionamide | C22H20ClN3O3 | 409.87 | 410.1 |
| 327 | 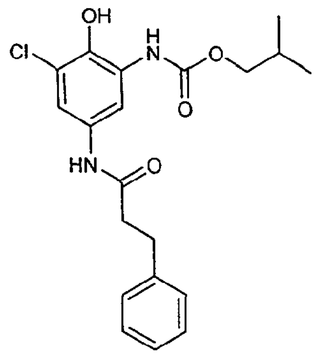 | [3-Chloro-2-hydroxy-5-(3-phenyl-propionylamino)-phenyl]-carbamic acid isobutyl ester | C20H23ClN2O4 | 390.86 | 390.8 |

| | | | | | |
|---|---|---|---|---|---|
| 328 | 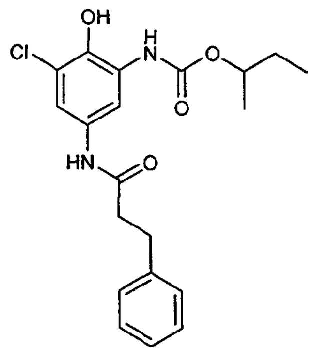 | [3-Chloro-2-hydroxy-5-(3-phenyl-propionylamino)-phenyl]-carbamic acid sec-butyl ester | C20H23ClN2O4 | 390.86 | 390.8 |
| 329 | 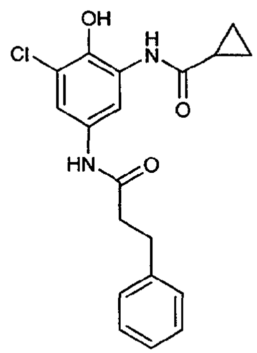 | Cyclopropanecarboxylic acid [3-chloro-2-hydroxy-5-(3-phenyl-propionylamino)-phenyl]-amide | C19H19ClN2O3 | 358.82 | 358.9 |
| 330 | 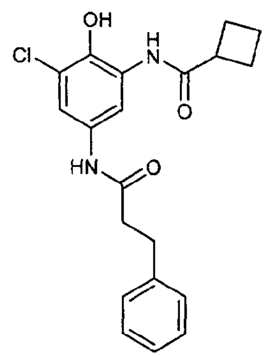 | Cyclobutanecarboxylic acid [3-chloro-2-hydroxy-5-(3-phenyl-propionylamino)-phenyl]-amide | C20H21ClN2O3 | 372.85 | 373.8 |

| | | | | | |
|---|---|---|---|---|---|
| 331 | 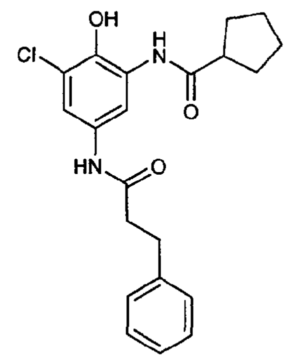 | Cyclopentanecarboxylic acid [3-chloro-2-hydroxy-5-(3-phenyl-propionylamino)-phenyl]-amide | C21H23ClN2O3 | 386.87 | 386.8 |
| 332 | 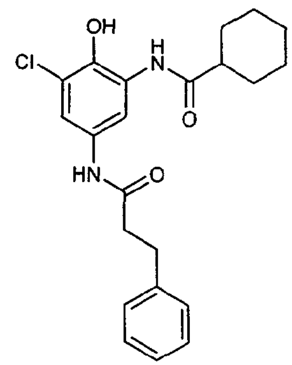 | Cyclohexanecarboxylic acid [3-chloro-2-hydroxy-5-(3-phenyl-propionylamino)-phenyl]-amide | C22H24ClN2O3 | 400.90 | 401.8 |
| 333 | 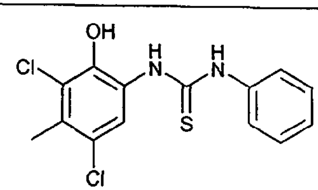 | 1-(3,5-Dichloro-2-hydroxy-4-methyl-phenyl)-3-phenyl-thiourea | C14H12Cl2N2OS | 326.0 | 327.1 |
| 334 | 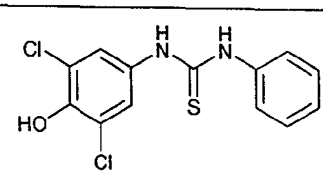 | 1-(3,5-Dichloro-4-hydroxy-phenyl)-3-phenyl-thiourea | C13H10Cl2N2OS | 312.0 | 313.0 |

| | | | | | |
|---|---|---|---|---|---|
| 335 | 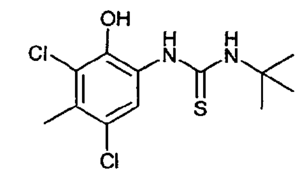 | 1-tert-Butyl-3-(3,5-dichloro-2-hydroxy-4-methyl-phenyl)-thiourea | C12H16Cl2N2OS | 306.0 | 307.1 |
| 336 | 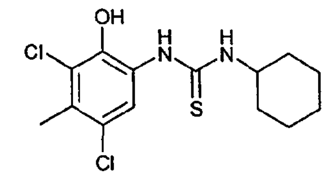 | 1-Cyclohexyl-3-(3,5-dichloro-2-hydroxy-4-methyl-phenyl)-thiourea | C14H18Cl2N2OS | 332.0 | 333.0 |
| 337 | 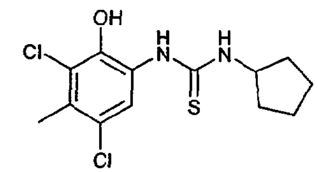 | 1-Cyclopentyl-3-(3,5-dichloro-2-hydroxy-4-methyl-phenyl)-thiourea | C13H16Cl2N2OS | 318.0 | 319.0 |
| 338 | 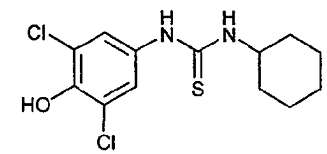 | 1-Cyclohexyl-3-(3,5-dichloro-4-hydroxy-phenyl)-thiourea | C13H16Cl2N2OS | 318.0 | 319.1 |
| 339 | 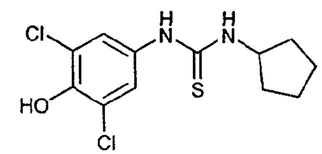 | 1-Cyclopentyl-3-(3,5-dichloro-4-hydroxy-phenyl)-thiourea | C12H14Cl2N2OS | 304.0 | 305.0 |
| 340 | 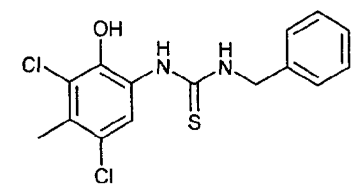 | 1-Benzyl-3-(3,5-dichloro-2-hydroxy-4-methyl-phenyl)-thiourea | C15H14Cl2N2OS | 340.0 | 341.1 |

| | | | | | |
|---|---|---|---|---|---|
| 341 | 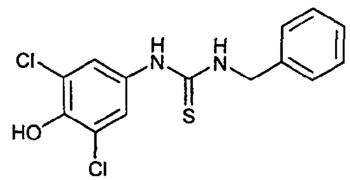 | 1-Benzyl-3-(3,5-dichloro-4-hydroxy-phenyl)-thiourea | C14H12Cl2N2OS | 326.0 | 327.0 |
| 342 | 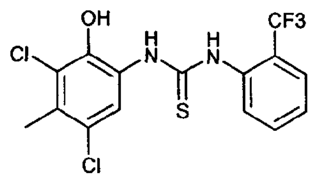 | 1-(3,5-Dichloro-2-hydroxy-4-methyl-phenyl)-3-(2-trifluoromethyl-phenyl)-thiourea | C15H11Cl2F3N2OS | 394.0 | 395.1 |
| 343 | 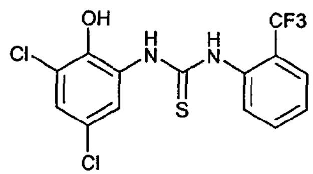 | 1-(3,5-Dichloro-2-hydroxy-phenyl)-3-(2-trifluoromethyl-phenyl)-thiourea | C14H9Cl2F3N2OS | 380.0 | 381.0 |
| 344 | 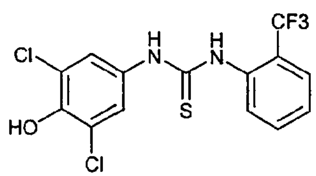 | 1-(3,5-Dichloro-4-hydroxy-phenyl)-3-(2-trifluoromethyl-phenyl)-thiourea | C14H9Cl2F3N2OS | 380.0 | 381.0 |
| 345 | 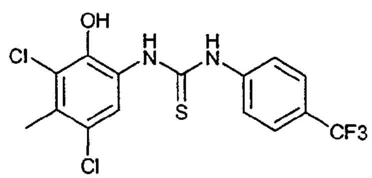 | 1-(3,5-Dichloro-2-hydroxy-4-methyl-phenyl)-3-(4-trifluoromethyl-phenyl)-thiourea | C15H11Cl2F3N2OS | 394.0 | 395.1 |

| | | | | | |
|---|---|---|---|---|---|
| 346 | 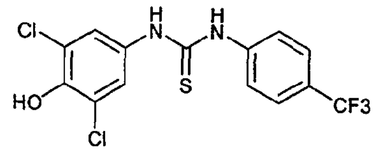 | 1-(3,5-Dichloro-4-hydroxy-phenyl)-3-(4-trifluoromethyl-phenyl)-thiourea | C14H9Cl2F3N2OS | 380.0 | 381.0 |
| 347 | 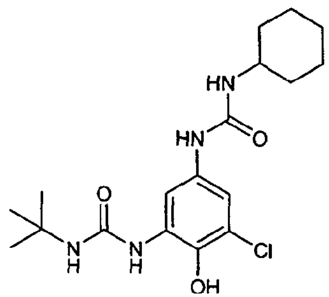 | 1-tert-Butyl-3-[3-chloro-5-(3-cyclohexyl-ureido)-2-hydroxy-phenyl]-urea | C18H27ClN4O3 | 382.9 | 383.0 |
| 348 | 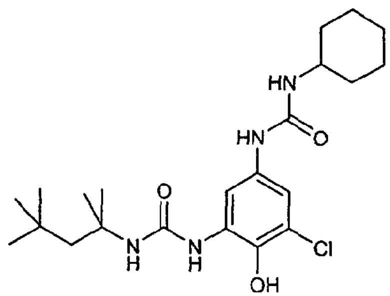 | 1-[3-Chloro-5-(3-cyclohexyl-ureido)-2-hydroxy-phenyl]-3-(1,1,3,3-tetramethyl-butyl)-urea | C22H35ClN4O3 | 439.0 | 439.1 |
| 349 | 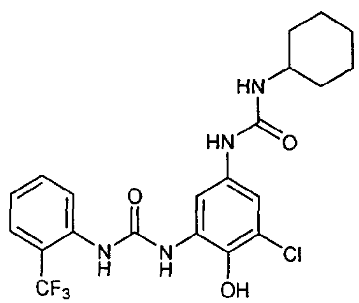 | 1-[3-Chloro-5-(3-cyclohexyl-ureido)-2-hydroxy-phenyl]-3-(2-trifluoromethyl-phenyl)-urea | C21H22ClF3N4O3 | 470.9 | 471.1 |

| | | | | | |
|---|---|---|---|---|---|
| 350 | 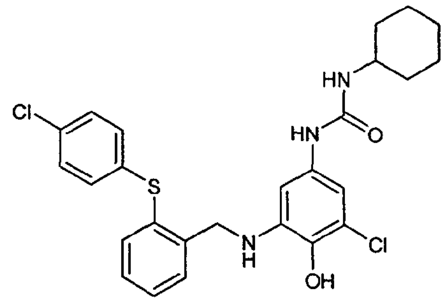 | 1-{3-Chloro-5-[2-(4-chloro-phenylsulfanyl)-benzylamino]-4-hydroxy-phenyl}-3-cyclohexyl-urea | C26H27Cl2N3O2S | 516.5 | 516.1 |
| 351 | 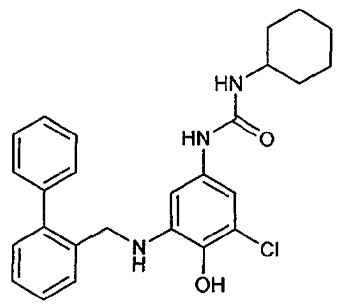 | 1-{3-[(Biphenyl-2-ylmethyl)-amino]-5-chloro-4-hydroxy-phenyl}-3-cyclohexyl-urea | C26H28ClN3O2 | 450.0 | 450.1 |
| 352 | 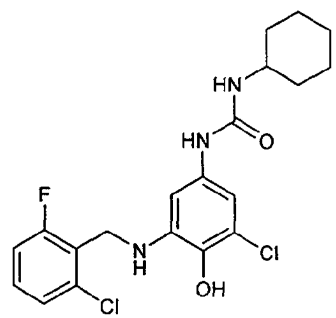 | 1-[3-Chloro-5-(2-chloro-6-fluoro-benzylamino)-4-hydroxy-phenyl]-3-cyclohexyl-urea | C20H22Cl2FN3O2 | 426.3 | 426.2 |

| | | | | | |
|---|---|---|---|---|---|
| 353 | 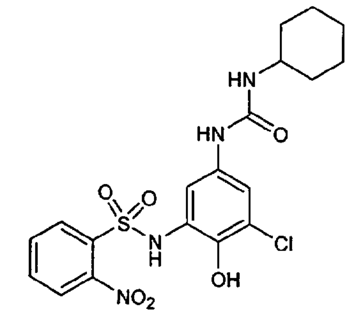 | N-[3-Chloro-5-(3-cyclohexyl-ureido)-2-hydroxy-phenyl]-2-nitro-benzenesulfonamide | C19H21ClN4O6S | 468.9 | 469.1 |
| 354 | 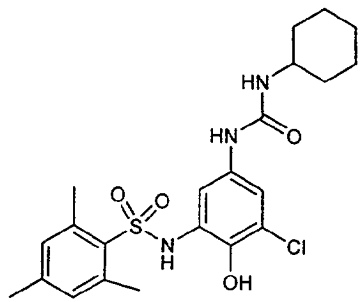 | N-[3-Chloro-5-(3-cyclohexyl-ureido)-2-hydroxy-phenyl]-2,4,6-trimethyl-benzenesulfonamide | C22H28ClN3O4S | 466.0 | 466.2 |
| 355 | 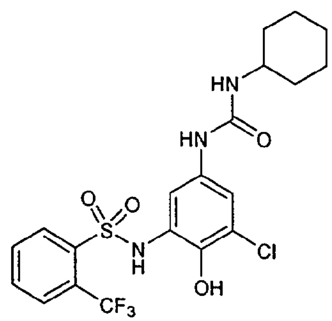 | N-[3-Chloro-5-(3-cyclohexyl-ureido)-2-hydroxy-phenyl]-2-trifluoromethyl-benzenesulfonamide | C20H21ClF3N3O4S | 491.9 | 492.2 |

| | | | | | |
|---|---|---|---|---|---|
| 356 | 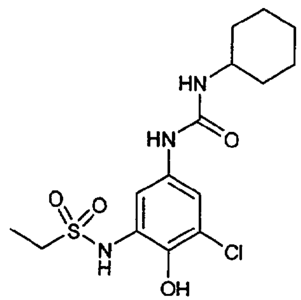 | Ethanesulfonic acid [3-chloro-5-(3-cyclohexyl-ureido)-2-hydroxy-phenyl]-amide | C15H22ClN3O4S | 375.9 | 376.1 |
| 357 | 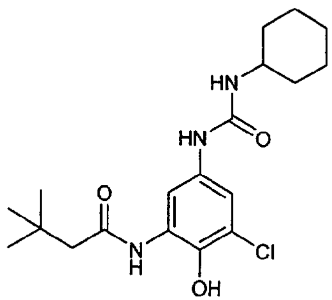 | N-[3-Chloro-5-(3-cyclohexyl-ureido)-2-hydroxy-phenyl]-3,3-dimethyl-butyramide | C19H28ClN3O3 | 381.90 | 382.2 |
| 358 | 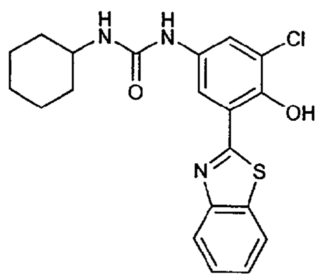 | 1-(3-Benzothiazol-2-yl-5-chloro-4-hydroxy-phenyl)-3-cyclohexyl-urea | C20H20ClN3O2S | 401.9 | 402.2 |

| | | | | | |
|---|---|---|---|---|---|
| 359 | 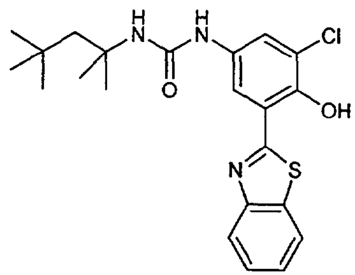 | 1-(3-Benzothiazol-2-yl-5-chloro-4-hydroxy-phenyl)-3-(1,1,3,3-tetramethyl-butyl)-urea | C22H26ClN3O2S | 432.0 | 432.3 |
| 360 | 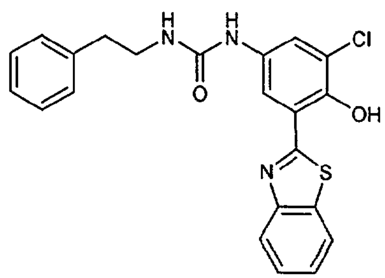 | 1-(3-Benzothiazol-2-yl-5-chloro-4-hydroxy-phenyl)-3-phenethyl-urea | C22H18ClN3O2S | 423.9 | 424.2 |
| 361 | 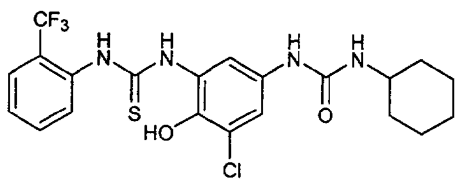 | 1-{3-Chloro-4-hydroxy-5-[3-(2-trifluoromethyl-phenyl)-thioureido]-phenyl}-3-cyclohexyl-urea | C21H22ClF3N4O2S | 486.9 | 487.1 |
| 362 | 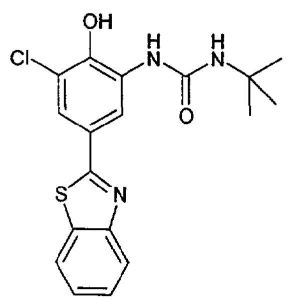 | 1-(5-Benzothiazol-2-yl-3-chloro-2-hydroxy-phenyl)-3-tert-butyl-urea | C18H18ClN3O2S | 375.9 | 376.1 |

| | | | | | |
|---|---|---|---|---|---|
| 363 | 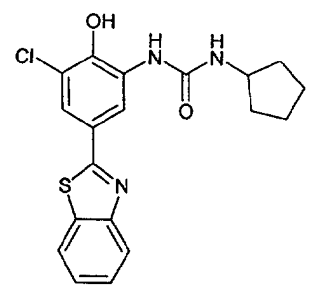 | 1-(5-Benzothiazol-2-yl-3-chloro-2-hydroxy-phenyl)-3-cyclopentyl-urea | C19H18ClN3O2S | 387.9 | 388.1 |
| 364 | 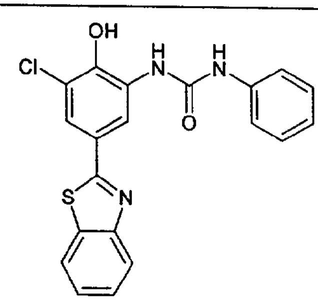 | 1-(5-Benzothiazol-2-yl-3-chloro-2-hydroxy-phenyl)-3-phenyl-urea | C20H14ClN3O2S | 395.9 | 396.2 |
| 365 | 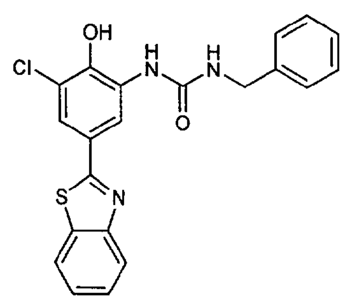 | 1-(5-Benzothiazol-2-yl-3-chloro-2-hydroxy-phenyl)-3-benzyl-urea | C21H16ClN3O2S | 409.9 | 410.1 |

| | | | | | |
|---|---|---|---|---|---|
| 366 | 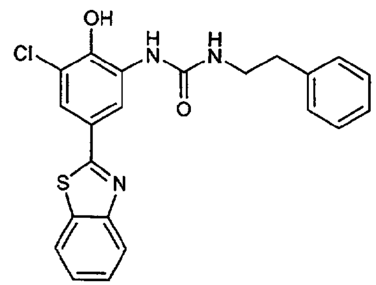 | 1-(5-Benzothiazol-2-yl-3-chloro-2-hydroxy-phenyl)-3-phenethyl-urea | C22H18ClN3O2S | 423.9 | 424.1 |
| 367 | 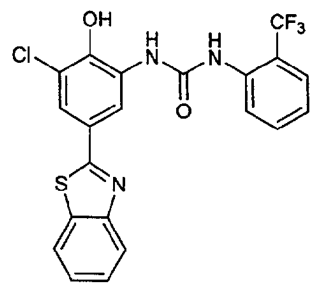 | 1-(5-Benzothiazol-2-yl-3-chloro-2-hydroxy-phenyl)-3-(2-trifluoromethyl-phenyl)-urea | C21H13ClF3N3O2S | 463.9 | 464.1 |
| 368 | 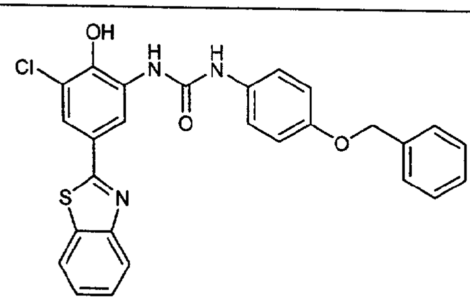 | 1-(5-Benzothiazol-2-yl-3-chloro-2-hydroxy-phenyl)-3-(4-benzyloxy-phenyl)-urea | C27H20ClN3O3S | 502.0 | 502.1 |

| | | | | | |
|---|---|---|---|---|---|
| 369 | 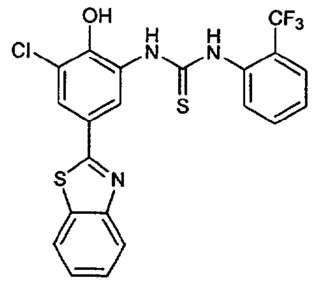 | 1-(5-Benzothiazol-2-yl-3-chloro-2-hydroxy-phenyl)-3-(2-trifluoromethyl-phenyl)-thiourea | C21H13ClF3N3OS2 | 479.9 | 480.0 |
| 370 | 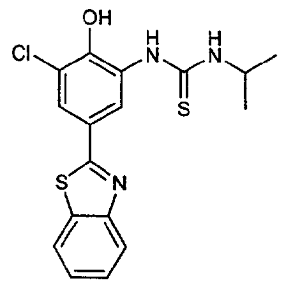 | 1-(5-Benzothiazol-2-yl-3-chloro-2-hydroxy-phenyl)-3-isopropyl-thiourea | C17H16ClN3OS2 | 377.9 | 378.1 |
| 371 | 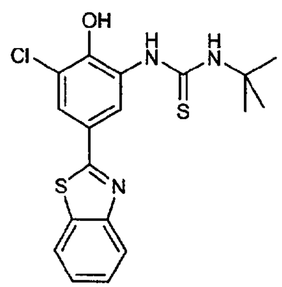 | 1-(5-Benzothiazol-2-yl-3-chloro-2-hydroxy-phenyl)-3-tert-butyl-thiourea | C18H18ClN3OS2 | 391.9 | 392.1 |

| | | | | | |
|---|---|---|---|---|---|
| 372 | 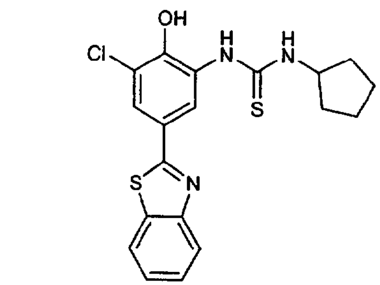 | 1-(5-Benzothiazol-2-yl-3-chloro-2-hydroxy-phenyl)-3-cyclopentyl-thiourea | C19H18ClN3OS2 | 403.9 | 404.1 |
| 373 | 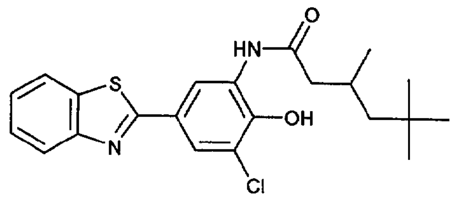 | 3,5,5-Trimethyl-hexanoic acid (5-benzothiazol-2-yl-3-chloro-2-hydroxy-phenyl)-amide | C22H25ClN2O2S | 417.0 | 417.2 |
| 374 | 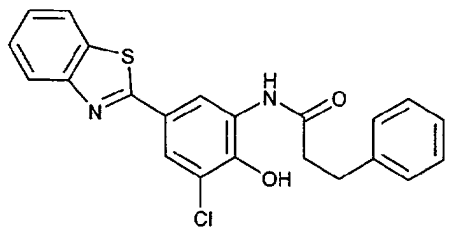 | N-(5-Benzothiazol-2-yl-3-chloro-2-hydroxy-phenyl)-3-phenyl-propionamide | C22H17ClN2O2S | 408.9 | 409.2 |
| 375 | 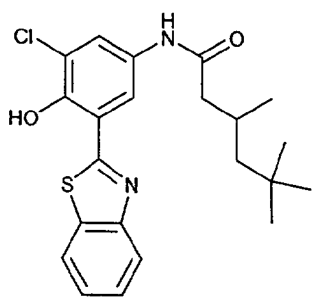 | 3,5,5-Trimethyl-hexanoic acid (3-benzothiazol-2-yl-5-chloro-4-hydroxy-phenyl)-amid | C22H25ClN2O2S | 417.0 | 417.7 |

| | | | | | |
|---|---|---|---|---|---|
| 376 | 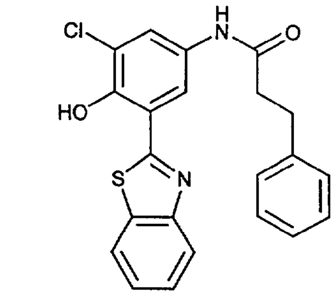 | N-(3-Benzothiazol-2-yl-5-chloro-4-hydroxy-phenyl)-3-phenyl-propionamide | C22H17ClN2O2S | 408.9 | 409.2 |
| 377 | 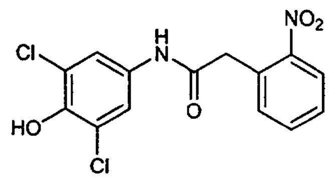 | N-(3,5-Dichloro-4-hydroxy-phenyl)-2-(2-nitro-phenyl)-acetamide | C14H10Cl2N2O4 | 341.15 | 341.5 |
| 378 | 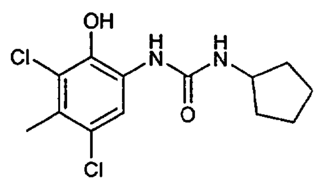 | 1-Cyclopentyl-3-(3,5-dichloro-2-hydroxy-4-methyl-phenyl)-urea | C13H16Cl2N2O2 | 303.18 | 303.5 |
| 379 | 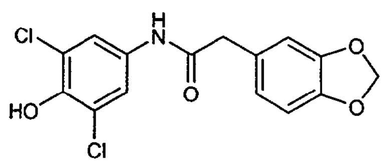 | 2-Benzo[1,3]dioxol-5-yl-N-(3,5-dichloro-4-hydroxy-phenyl)-acetamide | C15H11Cl2NO4 | 340.16 | 340.8 |

or a pharmaceutical acceptable salt or prodrug thereof.

Even more preferred compounds according to the present invention are those mentioned in any of the tables herein and those further disclosed and/or characterized in the examples.

As used herein, each of the following terms, used alone or in conjunction with other terms, are defined as follows (except where noted to the contrary):

The term "alkyl" refers to a saturated aliphatic radical containing from one to ten carbon atoms or a mono- or polyunsaturated aliphatic hydrocarbon radical containing from two to twelve carbon atoms, containing at least one double and triple bound, respectively. "Alkyl" refers to both branched and unbranched alkyl groups. Preferred alkyl groups are straight chain alkyl groups containing from one to eight carbon atoms. More preferred alkyl groups are straight chain alkyl groups containing from one to six carbon atoms and branched alkyl groups containing from three to six carbon atoms. It should be understood that any combination term using an "alk" or "alkyl" prefix refers to analogs according to the above definition of "alkyl". For example, terms such as "alkoxy", "alkylthio" refer to alkyl group linked to a second group via an oxygen or sulfur atom. "Alkanoyl" refers to an alkyl group linked to a carbonyl group (C=O).

The term "cycloalkyl" refers to the cyclic analog of an alkyl group, as defined above, optionally unsaturated and/or substituted. Preferred cycloalkyl groups are saturated cycloalkyl groups, more particularly those containing from three to eight carbon atoms, and even more preferably three to six carbon atoms.

The term "aryl" refers to aromatic groups having in the range of 6 to 14 carbon atoms and "substituted aryl" refers to aryl groups further bearing one or more substituents.

Each of the above defined "alkyl", "cycloalkyl", and "aryl" shall be understood to include their halogenated analogs, whereby the halogenated analogs may comprise one or several halogen atoms. The halogenated analogs thus comprise any halogen radical as defined in the following.

The term "halo" refers to a halogen radical selected from fluoro, chloro, bromo, iodo. Preferred halo groups are fluoro, chloro and bromo.

The term "heteroaryl" refers to a stable 5 to 8 membered, preferably 5 or 6 membered monocyclic or 8 to 11 membered bicyclic aromatic heterocycle radical. Each heterocycle consists of carbon atoms and from 1 to 4 heteroatoms selected from the group consisting of nitrogen, oxygen, sulfur. The heterocycle may be attached by any atom of the cycle, which results in the creation of a stable structure. Preferred heteroaryl radicals as used herein include, for example, furanyl, thienyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridinyl, pyridazinyl, pyrimidinyl, pyrazinyl, indolizinyl, indolyl, isoindolyl, benzofuranyl, benzothienyl, indazolyl, benzimidazolyl, benzthiazolyl, benzoxazolyl, purinyl, quinolizinyl, quinolinyl, isoquinolinyl, cinnolinyl, phthalazinyl, quinazolinyl, quinoxalinyl, naphthridinyl, pteridinyl, carbazolyl, acridinyl, phenazinyl, phenothiazinyl and phenoxazinyl.

The term "heterocyclyl" refers to a stable 5 to 8 membered, preferably 5 or 6 membered monocyclic or 8 to 11 membered bicyclic heterocycle radical which may be either saturated or unsaturated, and is non-aromatic. Each heterocycle consists of carbon atom(s) and from 1 to 4 heteroatoms selected from the group consisting of nitrogen, oxygen and sulfur. The heterocycle may be attached by any atom of the cycle, which results in the creation of a stable structure. Preferred heterocycle radicals as used herein include, for example, pyrrolinyl, pyrrolidinyl, pyrazolinyl, pyrazolidinyl, piperidinyl, morpholinyl, thiomorpholinyl, pyranyl, thiopyranyl, piperazinyl, indolinyl, azetidinyl, tetrahydropyranyl, tetrahydrothiopyranyl, tetrahydrofuranyl, hexahydropyrimidinyl, hexahydropyridazinyl, 1,4,5,6-tetrahydropyrimidin-2-ylamine, dihydro-oxazolyl, 1,2-thiazinanyl-1,1-dioxide, 1,2,6-thiadiazinanyl-1,1-dioxide, isothiazolidinyl-1,1-dioxide and imidazolidinyl-2,4-dione.

The terms "heterocyclyl", "heteroaryl" and "aryl", when associated with another moiety, unless otherwise specified, shall have the same meaning as given above. For example, "aroyl" refers to phenyl or naphthyl linked to a carbonyl group (C=O).

Each aryl or heteroaryl unless otherwise specified includes its partially or fully hydrogenated derivative. For example, quinolinyl may include decahydroquinolinyl and tetrahydroquinolinyl, naphthyl may include its hydrogenated derivatives such as tetrahydranaphthyl.

As used herein above and throughout this application, "nitrogen" and "sulfur" include any oxidized form of nitrogen and sulfur and the quatemized form of any basic nitrogen sulfoxide, sulfone, nitrone, N-oxide.

As used herein a wording defining the limits of a range of length such as e. g. "from 2 to 5" means any integer from 1 to 5, i. e. 1, 2, 3, 4 and 5. In other words, any range defined by two integers explicitly mentioned is meant to comprise any integer defining said limits and any integer comprised in said range.

As used herein the term substituted shall mean that one or more H atom of the group or compound which is substituted, is replaced by a different atom, a group of atoms, a molecule or a molecule moiety. Such atom, group of atoms, molecule or molecule moiety is also referred to herein as substituent.

The substituent can be selected from the group comprising hydroxy, alkoxy, mercapto, cycloalkyl, heterocyclic, aryl, heteroaryl, aryloxy, halogen, trifluoromethyl, difluoromethyl, cyano, nitrone, amino, amido, -C(O)H, acyl, oxyacyl, carboxyl, carbamate, sulfonyl, sulphonamide and sulfuryl. Any of the substituents may be substituted itself by any of the aforementioned substituents. This applies preferably to cycloalkyl, heterocylic, aryl, heteroaryl and aryloxy. It is

also preferred that alkoxy and mercapto are those of a lower alkyl group. It is to be acknowledged that any of the definition provided herein also applies to any substituent.

In a further aspect the present invention is related to the use of the compounds according to the present invention as a medicament and for the manufacture of a medicament, respectively.

In an embodiment the medicament is for the treatment or prevention of a disease, whereby the disease involves an undesired cell proliferation.

This use of the compounds according to the present invention is based on the fact that the compounds according to the present invention are suitable to inhibit undesired cell proliferation. Undesired cell proliferation comprises the undesired cell proliferation of procaryotic cells as well as undesired cell proliferation of eucaryotic cells. The term undesired cell proliferation also covers the phenomenon of abnormal cell proliferation, abnormal mitosis and undesired mitosis. Abnormal cell proliferation means any form of cell proliferation which occurs in a manner different from the normal cell proliferation. Normal cell proliferation is a cell proliferation observed under normal circumstances by the majority of cells and organisms, respectively. The same basic definition applies to abnormal mitosis.

More particularly, undesired cell proliferation and undesired mitosis mean a proliferation and a mitosis, respectively, which may be either a normal or an abnormal cell proliferation, however, in any case it is not a cell proliferation or mitosis which is desired. Desired may thus be defined by an individual such as a human being and in particular a physician, and defined within certain boundaries whereby the boundaries as such may reflect the extent of proliferation and mitosis, respectively, observed under usual conditions or in the majority of cells and organisms, respectively, or may be arbitrarily fixed or defined. Cell proliferation as used herein refers preferably to the proliferation of cells forming the organism to be treated or to which a compound according to the present invention shall be administered which is also referred to herein as the first organism. Cell proliferation as used herein also means the proliferation of cells which are different from the cells forming a first organism or species but are the cells forming a second organism or second species. Typically, the second organism enters in or has a relationship with the first organism. Preferably, the first organism is a human being or an animal or plant, also referred to herein as patient, and the second organism is a parasite and pathogen, respectively, to said first organism. Mitosis as used herein, preferably means the cell division of cells being subject to said cell proliferation whereby even more preferably mitosis is the process of cell division whereby a complete set of chromosomes is distributed to the daughter cells.

Without wishing to be bound by any theory, it seems that the compounds according to the present invention act on cells and thus influence their proliferation and mitosis, respectively, by being inhibitors to some enzymatic activity. Preferably, the inhibition is reversible. This activity is shown by the compounds according to the present invention with regard to bacteria, fungi, insect and mammalian cells.

Because of this, the compounds according to the present invention may be used for the treatment of a wide variety of disorders involving cell cycle regulation, both procaryotic and eucaryotic cell cycle regulation. The term "treatment" as used herein comprises both treatment and prevention of a disease. It also comprises follow-up treatment of a disease. Follow-up treatment is realized upon a treatment of a disease using compounds preferably different from the one according to the present invention. For example, after stimulating the growth of a cell, tissue or the like by the application of a respective compound such as, e. g., erythropoietin, it might be necessary to stop an overshooting reaction of cell proliferation which may be obtained using the compounds according to the present invention.

By "reversible" herein is meant that the inhibitor binds non-covalently to the respective enzyme, and is to be distinguished from irreversible inhibition. See Walsh, Enzymatic Reaction Mechanisms, Freeman & Co., N.Y., 1979. "Reversible" in this context is a term understood by those skilled in the art. Preferably the compounds according to the present invention are competitive inhibitors, that is, they compete with substrate in binding reversibly to the enzyme, with the binding of inhibitor and substrate being mutually exclusive.

In a preferred embodiment of the compounds according to the present invention the dissociation constant for inhibition of the enzyme(s) with the inhibitor, i. e. the compound according to the present invention, generally referred to and characterized by those in the art as $K_i$, is at most about 100 µM. By the term "binding constant" or "dissociation constant" or grammatical equivalents herein is meant the equilibrium dissociation constant for the reversible association of inhibitor with enzyme. The dissociation constants are defined and determined as described below. The determination of dissociation constants is known in the art. For example, for reversible inhibition reactions such as those of the present invention, the reaction scheme is as follows:

$$E+I \underset{k_2}{\overset{k_1}{\rightleftharpoons}} E{*}I \quad \text{(Equation 1)}$$

The enzyme (E) and the inhibitor (1) combine to give an enzyme-inhibitor complex (E*I). This step is assumed

to be rapid and reversible, with no chemical changes taking place; the enzyme and the inhibitor are held together by non-covalent forces. In this reaction, $k_1$ is the second order rate constant for the formation of the E*I reversible complex. $k_2$ is the first order rate constant for the dissociation of the reversible E*I complex. In this reaction, $Ki = k_2/k_1$.

The measurement of the equilibrium constant $K_i$ proceeds according to techniques well known in the art. For example, assays generally use synthetic chromogenic or fluorogenic substrates. The respective $K_i$ values may be estimated using the Dixon plot as described by Irwin Segel in Enzyme Kinetics: Behavior and analysis of rapid equilibrium and steady-state enzyme systems, 1975, Wiley-Interscience Publication, John Wiley & Sons, New York, or for competitive binding inhibitors from the following calculation:

$$1-(\nu_i/\nu_o)=[I]/[I]+K_i\ (1+([S]/K_m))) \qquad \text{(Equation 2)}$$

wherein $\nu_o$ is the rate of substrate hydrolysis in the absence of inhibitor, and $\nu_i$ is the rate in the presence of competitive inhibitor.

The compounds according to the present invention may be easily screened for their efficacy in relation to the various uses disclosed herein

By a "labelled compound according to the present invention" herein is meant a compound according to the present invention that has at least one element, isotope or chemical compound attached to enable the detection of the compound or the compound bound to a target such as an enzyme. In general, labels as used herein, fall into three classes: a) isotopic labels, which may be radioactive or heavy isotopes; b) immune labels, which may be antibodies or antigens; and c) colored or fluorescent dyes. The labels may be incorporated into the compound at any position. Examples of useful labels include $^{14}C$, $^{13}C$, $^{15}N$, $^{3}H$, biotin, and fluorescent labels as are well known in the art.

As used herein, the term "disease" describes any disease, diseased condition or pathological condition. Such disease may also be defined as abnormal condition. Also, in case of a pathogen, disease means a condition where a pathogen or an unwanted organism is present or present in a concentration or compartment where it is undesired and thus subject to reduction in numbers, removal, elimination and/or destruction by using the compounds according to the present invention.

The compounds according to the present invention may be used as a medicament and for the manufacture of a medicament, respectively, whereby the medicament is for the treatment of cell proliferative disorders. Cell proliferated disorders as used herein, typically involve an abnormal cell proliferation, an undesired cell proliferation, an abnormal mitosis and/or an undesired mitosis.

Cell proliferative disorders contemplated for treatment using the compounds according to the present invention and for the methods disclosed herein include also disorders characterized by unwanted or undesired, inappropriate or uncontrolled cell growth. Preferably, the disease is selected from the group comprising neurodegenerative diseases, stroke, inflammatory diseases, immune based disorders, infectious diseases, heart diseases, fibrotic disorders, cardiovascular diseases and cell proliferative diseases.

Preferably, the neurodegenerative disease is selected from the group comprising Alzheimer's disease, Huntington's disease, Parkinson's disease, peripheral neuropathy, progressive supranuclear palsy, corticobasal degeneration, frontotemporal dementia, synucleinopathies, multiple system atrophy, amyotrophic lateral atrophy, prion diseases and motor neuron diseases.

The compounds according to the present invention are additionally useful in inhibiting cell cycle (mitosis) or cell division in pathogenic organisms and are, therefore, useful for treating infectious diseases.

In a preferred embodiment the infectious is selected from the group comprising fungal, viral, bacterial and parasite infection.

Fungal infections contemplated for treatment using the compounds and methods according to the present invention include systemic fungal infections, dermatophytoses and fungal infections of the genito-urinary tract. Fungal infections, preferably systemic fungal infections, include those caused by *Histoplasma, Coccidioides, Cryptococcus, Blastomyces, Paracoccidioides, Aspergillus, Nocardia, Sporothrix, Rhizopus, Absidia, Mucor, Hormodendrum, Phialophora, Rhinosporidium,* and the like. Dermatophyte infections include those caused by *Microsporum, Trichophyton, Epidermophyton, Candida*, *Pityrosporum,* and the like. Fungal disorders of the genito-urinary tract include infections caused by *Candida, Cryptococcus, Aspergillus, Zygomycodoides*, and the like. Infection by such organisms causes a wide variety of disorders such as ringworm, thrush or candidiasis, San Joaquin fever or Valley fever or coccidiodomycosis, Gilchrist's disease or blastomycosis, aspergillosis, cryptococcosis, histioplasmosis, paracoccidiomycosis, zygomycosis, mycotic keratitis, nail hair and skin disease, Lobo's disease, lobomycosis, chromoblastomycosis, mycetoma, and the like. These infections can be particularly serious, and even fatal, in patients with a depressed immune system such as organ transplant recipients and persons with acquired immunodefficiency syndrome (AIDS). Insofar a patient group which can be treated using the inhibitors according to the present invention are persons with AIDS, particularly

those suffering from any of the infectious diseases described herein.

In a further embodiment the bacterial infection is selected from the group comprising infections caused by both Gram-positive and Gram-negative bacteria, including infections caused by *Staphylococcus, Clostridium, Streptococcus, Enterococcus, Diplococcus*, *Hemophilus, Neisseria, Erysipelothricosis, Listeria, Bacillus, Salmonella, Shigella, Escherichia, Klebsiella, Enterobacter, Serratia, Proteus, Morganella, Providencia, Yersinia, Camphylobacter, Mycobacteria, Helicobacter, Legionalla, Nocardia* and the like.

In a preferred embodiment the bacterial infection causes a wide variety of diseases. Said disorders are selected, among others, from the group comprising pneumonia, diarrhea, dysentery, anthrax, rheumatic fever, toxic shock syndrome, mastoiditis, meningitis, gonorrhea, typhoid fever, brucellis, Lyme disease, gastroenteritis, tuberculosis, cholera, tetanus and bubonic plague.

In another embodiment the disease is a viral infection, more particularly a viral infection caused by a virus selected from the group comprising retrovirus, HIV, Papilloma virus, Epstein-Barr, Herpes virus, Hepatitis virus, Papova virus, Influenza virus, Rabies, JC, encephalitis causing virus, hemorrhagic fever causing virus such as Ebola Virus and Marburg Virus.

In a further embodiment the parasite infection is selected from the group comprising infections caused by *Trypanosoma, Leishmania, Trichinella, Echinococcus, Nematodes, Classes Cestoda, Trematoda, Monogenea, Toxoplasma, Giardia, Balantidium, Paramecium, Plasmodium or Entamoeba.*

The disease may further be a cell proliferative disorder which preferably is selected from the group characterized by unwanted, inappropriate or uncontrolled cell growth. Particular examples include cancer, fibrotic disorders, non-neoplastic growths. The neoplastic cell proliferative disorder is preferably selected from the group comprising solid tumors, and hematopoeitic cancers such as lymphoma and leukemia.

More preferably, the solid tumor is selected from the group comprising carcinoma, sarcoma, osteoma, fibrosarcoma, and chondrosarcoma.

More preferably, the cell proliferative disorder is selected from the group comprising breast cancer, prostate cancer, colon cancer, brain cancer, lung cancer, pancreatic cancer, gastric cancer, bladder cancer, kidney cancer and head and neck cancer. Preferably, the lung cancer is non-small lung cancer and small lung cancer.

In case the disease is a non-neoplastic cell proliferative disorder, it is preferably selected from the group comprising fibrotic disorder. Preferably, the fibrotic disorder is fibrosis.

The disease may also be a non-neoplastic cell proliferative disorder which is selected from the group comprising prostatic hypertrophy, preferably benign prostatic hypertrophy, endometriosis, psoriasis, tissue repair and wound healing.

Fibrotic disorders which may be treated using the compounds according to the present invention are generally characterized by inappropriate overproliferation of non-cancerous fibroblasts. Examples thereof include fibromyalgia, fibrosis, more particularly cystic, hepatic, idopathic pulmonary, and pericardial fibrosis and the like, cardiac fibromas, fibromuscular hyperplasia, restenosis, atherosclerosis, fibromyositis, and the like.

In another embodiment the immune based and/or inflammatory disease is an autoimmune disease or autoimmune disorder. In a further embodiment, the immune based and/or inflammatory disease is selected from the group comprising rheumatoid arthritis, glomerulonephritis, systemic lupus erythematosus associated glomerulonephritis, irritable bowel syndrome, bronchial asthma, multiple sclerosis, pemphigus, pemphigoid, scleroderma, myasthenia gravis, autoimmune haemolytic and thrombocytopenic states, Goodpasture's syndrome, pulmonary hemorrhage, vasculitis, Crohn's disease, and dermatomyositis.

In a further preferred embodiment the immune based and/or inflammatory disease is an inflammatory condition.

In a still further embodiment the immune based and/or inflammatory disease is selected from the group comprising inflammation associated with bums, lung injury, myocardial infarction, coronary thrombosis, vascular occlusion, post-surgical vascular reocclusion, artherosclerosis, traumatic central nervous system injury, ischemic heart disease and ischemia-reperfusion injury, acute respiratory distress syndrome, systemic inflammatory response syndrome, multiple organ dysfunction syndrome, tissue graft rejection and hyperacute rejection of transplanted organs.

It is also within the present invention that the compounds according to the present invention may be used for the treatment of a patient suffering from a disease or diseased condition as defined above. Such treatment comprises the administration of one or several of the compounds according to the present invention or a medicament or pharmaceutical composition described herein.

Toxicity and therapeutic efficacy of a compound can be determined by standard pharmaceutical procedures in cell culture or experimental animals. Cell culture assays and animal studies can be used to determine the $LD_{50}$ (the dose lethal to 50% of a population) and the $ED_{50}$ (the dose therapeutically effective in 50% of a population). The dose ratio between toxic and therapeutic effects is the therapeutic index, which can be expressed as the ratio $LD_{50}/ED_{50}$. Compounds which exhibit large therapeutic indices are preferred. The data obtained from these cell culture assays and animal studies can be used in formulating a range of dosages suitable for use in humans. The dosage may vary

within this range depending upon a variety of factors, e.g., the dosage form employed, the route of administration utilized, the condition of the subject, and the like

For any compound according to the present invention, the therapeutically effective dose can be estimated initially from cell culture assays by determining an $IC_{50}$ (i.e., the concentration of the test substance which achieves a half-maximal inhibition of cell proliferation). A dose can then be formulated in animal models to achieve a circulating plasma concentration range that includes the $IC_{50}$ as determined in cell culture. Such information can be used to more accurately determine useful doses in humans. Levels in plasma may be measured, for example by HPLC.

It should be noted that the attending physician would know how to and when to terminate, interrupt, or adjust administration due to toxicity, to organ dysfunction, and the like. Conversely, the attending physician would also know to adjust treatment to higher levels if the clinical response were not adequate (precluding toxicity). The magnitude of an administered dose in the management of the disorder of interest will vary with the severity of the condition to be treated, with the route of administration, and the like. The severity of the condition may, for example, be evaluated, in part, by standard prognostic evaluation methods. Further, the dose and perhaps dose frequency will also vary according to the age, body weight, and response of the individual patient. Typically, the dose will be between about 1-10 mg/kg of body weight. About 1 mg to about 50 mg will preferably be administered to a child, and between 25 mg and about 1000 mg will preferably be administered to an adult.

A program comparable to that discussed above may be used in veterinary medicine. The exact dose will depend on the disorder to be treated and will be ascertainable by one skilled in the art using known techniques.

Depending on the specific conditions to be treated, such compounds may be formulated and administrated systemically or locally. Techniques for formulation and administration may be found in "Remington's Pharmaceutical Sciences", 1990, $18^{th}$ ed., Mack Publishing Co., Easton, PA. The administration of a compound according to the present invention can be done in a variety of ways, including, but not limited to, orally, subcutaneously, intravenously, intranasally, transdermally, intraperitoneally, intramuscularly, intrapulmonary, vaginally, rectally, or intraocularly, just to name a few. In some instances, for example, in the treatment of wounds and inflammation, the compound according to the present invention may be directly applied as a solution or spray.

In a further aspect the present invention is related to a medicament or a pharmaceutical composition comprising at least one active compound and at least one pharmaceutically acceptable carrier, excipient or diluent. As used herein, the active compound is a compound according to the present invention, a pharmaceutically salt or base thereof or a prodrug thereof, if not indicated to the contrary.

For injection, compounds of the invention may be formulated in aqueous solution, preferably in physiologically compatible buffers such as Hank's solution, Ringer's solution, or physiologically saline buffer. For transmucosal administration, penetrants appropriate to the barrier to be permeated are used in the formulation. Such penetrants are generally known in the art.

The use of pharmaceutical acceptable carriers to formulate the compounds according to the present invention into dosages or pharmaceutical compositions suitable for systemic administration is within the scope of the present invention. With proper choice of carrier and suitable manufacturing practice, the compositions of the present invention, in particular those formulated as solutions, may be administered parenterally, such as by intravenous injection. The compounds can be readily formulated using pharmaceutically acceptable carriers well known in the art into dosages suitable for oral administration. Such carriers enable the compounds according to the present invention to be formulated as tablets, pills, capsules, dragees, liquids, gels, syrups, slurries, suspensions and the like, for oral ingestion by a subject to be treated.

Compounds according to the present invention or medicaments comprising them, intended to be administered intracellularly may be administered using techniques well known to those of ordinary skill in the art. For example, such agents may be encapsulated into liposomes, then administered as described above. Liposomes are spherical lipid bilayers with aqueous interiors. All molecules present in an aqueous solution at the time of liposome formation are incorporated into the aqueous interior. The liposomal contents are both protected from the external microenvironment and, because liposomes fuse with cell membranes, are efficiently delivered into the cell cytoplasm. Delivery systems involving liposomes are disclosed in International Patent Publication No. WO 91/19501, as well as U.S. Patent No. 4,880,635 to Janoff et al. The publications and patents provide useful descriptions of techniques for liposome drug delivery and are incorporated by reference herein in their entirety.

Pharmaceutical compositions comprising a compound according to the present invention for parenteral administration include aqueous solutions of the active compound(s) in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injections suspensions may contain compounds which increase the viscosity of the suspension, such as sodium carboxymethyl cellulose, sorbitol, dextran, or the like. Optionally, the suspension may also contain suitable stabilizers or agents which increase the solubility of the compounds to allow for the preparation of highly concentrated solutions.

Pharmaceutical compositions comprising a compound according to the present invention for oral use can be obtained by combining the active compound(s) with solid excipient, optionally grinding the resulting mixture, and processing the mixture of granules, after adding suitable auxiliaries, if desired, to obtain tablets or dragee cores.

Suitable excipients are, in particular, fillers such as sugars, including lactose, sucrose, mannitol, sorbitol, and the like; cellulose preparations, such as, for example, maize starch wheat starch, rice starch, potato starch, gelatin, gum tragacanth, methyl cellulose, hydroxypropylmethyl cellulose, sodium carboxymethyl cellulose, polyvinylpyrrolidone (PVP) and the like, as well as mixtures of any two or more thereof. If desired, disintegrating agents may be added, such as cross-linked polyvinyl pyrrolidone, agar, alginic acid or a salt thereof such as sodium alginate, and the like.

Dragee cores as a pharmaceutical composition comprising a compound according to the present invention are provided with suitable coatings. For this purpose, concentrated sugar solutions may be used, which may optionally contain gum arabic, talc, polyvinyl pyrrolidone, carbopol gel, polyethylene glycol, titanium dioxide, lacquer solutions, suitable organic solvents or solvent mixtures, and the like. Dyestuffs or pigments may be added to the tablets or dragee coatings for identification or to characterize different combinations of active compound doses.

Pharmaceutical preparations comprising a compound according to the present invention which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added.

A "patient" for the purposes of the present invention, i. e. to whom a compound according to the present invention or a pharmaceutical composition according to the present invention is administered, includes both humans and other animals and organisms. Thus the compounds, pharmaceutical compositions and methods are applicable to or in connection with both human therapy and veterinary applications. For example, the veterinary applications include, but are not limited to, canine, bovine, feline, porcine, caprine, equine, and ovine animals, as well as other domesticated animals including reptiles, such as iguanas, turtles and snakes, birds such as finches and members of the parrot family, lagomorphs such as rabbits, rodents such as rats, mice, guinea pigs and hamsters, amphibians, fish, and arthropods. Valuable non-domesticated animals, such as zoo animals, may also be treated. In the preferred embodiment the patient is a mammal, and in the most preferred embodiment the patient is human.

The pharmaceutical composition according to the present invention comprises at least one compound according to the present invention in a form suitable for administration to a patient. Preferably, a compound according to the present application is in a water soluble form, such as being present as a pharmaceutically acceptable salt, which is meant to include both acid and base addition salts which are also generally referred to herein as pharmaceutically acceptable salts. "Acid addition salt", and more particularly "pharmaceutically acceptable acid addition salts" refers to those salts that retain the biological effectiveness of the free bases and that are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like. "Base addition salts" and more particularly "pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium, and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, and ethanolamine. The pharmaceutical compositions according to the present invention may also include one or more of the following: carrier proteins such as serum albumin; buffers; fillers such as microcrystalline cellulose, lactose, corn and other starches; binding agents; sweeteners and other flavoring agents; coloring agents; and polyethylene glycol. Additives are well known in the art, and are used in a variety of formulations.

The compounds according to the present invention are, in a further embodiment, administered to a subject either alone or in a pharmaceutical composition where the compound(s) is mixed with suitable carriers or excipient(s). In treating a subject, a therapeutically effective dose of compound (i.e. active ingredient) is administered. A therapeutically effective dose refers to that amount of the active ingredient that produces amelioration of symptoms or a prolongation of survival of a subject which can be determined by the one skilled in the art doing routine testing.

On the other hand, the compounds according to the present invention may as such or contained in a pharmaceutical composition according to the present invention be used in drug potential applications.

For example, therapeutic agents such as antibiotics or antitumor drugs can be inactivated through the catalytic action of endogenous enzymes, thus rendering the administered drug less effective or inactive. Accordingly, the compound(s) according to the present invention may be administered to a patient in conjunction with a therapeutic agent in order to potentiate or increase the activity of the drug. This co-administration may be by simultaneous administration, such as a mixture of the compound(s) according to the present invention and the drug, or by separate simultaneous or sequential administration.

According to the present invention the compounds disclosed herein, referred to as compounds according to the present invention, may be used as a medicament or for the manufacture of medicament or in a method of treatment of a patient in need thereof. Insofar any of these compounds constitute a pharmaceutical compound. The use of this kind of compound also comprises the use of pharmaceutically acceptable derivatives of such compounds.

In addition, the compounds according to the present invention may be transformed upon application to an organism such as a patient, into the pharmaceutically active compound. Insofar the compounds according to the present invention may be prodrugs which, however, are nevertheless used for the manufacture of the medicaments as disclosed herein given the fact that at least in the organism they are changed in a form which allows the desired

It is to be understood that any of the pharmaceutical compositions according to the present invention may be used for any of the diseases described herein.

The pharmaceutical compositions according to the present invention may be manufactured in a manner that is known as such, e.g., by means of conventional mixing, dissolving, granulating, dragee-mixing, levigating, emulsifying, encapsulating, entrapping, lyophilizing, processes, or the like.

In a further aspect of the present invention the compounds of the present invention may be used as insecticides as they may prevent cell cycle mitosis in insect cells and thus can be used to control the growth and proliferation of a variety of insect pests. This aspect of the present invention has important applications in agriculture, such as in the field, in the storage of agricultural products and the like. Additionally, the compounds according to the present invention are useful for controlling insect populations, preferably in places inhabited by men, such as homes, offices and the like.

Any of the compounds according to the present invention containing one or more asymmetric carbon atoms may occur as racemates and racemic mixtures, single enantiomers, diastereomeric mixtures and individual diastereomers. All such isomeric forms of these compounds are expressly included in the present invention. Each stereogenic carbon may be in the R or S configuration, or a combination of configurations.

It shall be understood by the one of ordinary skill in the art that all compounds of the invention are preferably those which are chemically stable. This applies to any of the various uses of the compounds according to the present invention disclosed herein.

In determining the suitability of any of the compounds according to the present applications for the various uses, besides the particular use-specific profile to be met by such a compound, also it has to be checked whether it is stable to proteolytic degradation. The resistance of the compound used as a pharmaceutical may be tested against a variety of non-commercially available proteases *in vitro* to determine its proteolytic stability. Promising candidates may then be routinely screened in animal models, for example using labelled inhibitors, to determine the *in vivo* stability and efficacy. In any of the aforementioned uses the compound may be present in a crude or purified form. Methods for purifying the compounds according to the present invention are known to the one skilled in the art.

The problem underlying the present invention is also solved by the technical teaching according to the attached independent claims. Preferred embodiments thereof may be taken from the dependent claims.

The invention is now further illustrated by reference to the following figure and examples from which further advantages, features and embodiments may be taken. It is understood that these examples are given for purpose of illustration only and not for purpose of limitation. All references cited herein are incorporated by reference.

Fig. 1 shows FACS results of compound 89.

<u>Example 1:</u> Material and Methods

In order that the invention herein described may be more fully understood, the following detailed description is set forth. As used herein, the following abbreviations are used:

Ar is argon;
D is doublet;
DCM is dichloromethane;
DIPEA is *N,N* diisopropylethylamine;
DMF is *N,N*-dimethylformamide;
DMSO is *N,N* dimethylsulfoxide;
eq is equivalent;
$Et_3N$ is triethylamine;
HCl is chlorhydric acid;
HPLC is high performance liquid chromatography;

h is hour;
Hz is hertz;
m is multiplet;
mL is milliliter;
$NaHCO_3$ is sodium hydrogencarbonate;
s is singulet;
THF is tetrahydrofuran.

**Method A: Urea formation in solution**

Amine salt and DIPEA (1 eq each) or amine (1 eq) was dissolved in dry dioxan and a solution of the isocyanate (1 eq) in DCM or DMSO was added under Ar in one portion. The solution was stirred for 3 h at room temperature. The solution was diluted with 2 ml DCM and scavenger resins (tris-(2-aminoethyl)-amine polystyrene (3 eq), methylisocyanate polystyrene (3 eq) and N-(2-mercaptoethyl)aminomethyl polystyrene (3 eq)) were added to remove unreacted isocyanate, amine and electrophilic impurities respectively. After 18 h at 40°C, the solution was filtered off and the solvent was removed under reduced pressure. The obtained crude ureas were purified by HPLC.

**Method B: Coupling of substituted anilines with sulfonyl chlorides**

Aniline (30 mg) and $NEt_3$ (1.2 eq, 2.2 eq. when HCl salt) were dissolved in dry acetonitrile (0.5 mL). Sulfonyl chloride (1 eq) was dissolved in dry acetonitrile (0.5 mL) and added to the solution. The reaction mixture was stirred under argon for 12 h at 40°C. Tris-(2-aminoethyl)-amine polystyrene (30 mg), (polystyrylmethyl) trimethylammonium bicarbonate (30 mg), *N*-(2-mercaptoethyl)amino methyl polystyrene (30 mg), and methylisocyanate polystyrene (30 mg) were given to the solution and stirred for additional 12 h at 40°C. After filtration the solvent was removed under reduced pressure. The crude reaction product was purified by preparative HPLC using acetonitrile and water as mobile phase.

**Method C: Coupling of substituted anilines with acid chlorides**

Aniline (30 mg) and $NEt_3$ (1 eq, 2 eq. when HCl salt) were dissolved at 0°C in dry DCM (0.5 mL) with 10% DMSO. Acid chloride (1 eq) was dissolved at 0°C in dry DCM (0.5 mL) and added to the solution. The reaction mixture was stirred under argon for 2 h while slowly warming up to room temperature. Work up was performed by pouring the reaction mixture with DCM over incubated HYDROMATRIX layers. 2 mL basic layer (saturated $NaHCO_3$ solution 2 ml/g HYDROMATRIX), 2 mL acidic layer (2M HCl 2 ml/g HYDROMATRIX), and 2 mL of dry HYDROMATRIX layer in a 10 ml syringe were used. The solvent was removed under reduced pressure. The crude reaction product was purified by preparative HPLC using acetonitrile and water as mobile phase.

**Method D: Reductive amination of aldehydes with hydroxyl anilines**

Amine hydrochloride (0.11 mmol, 1 eq), aldehyde (0.11 mmol, 1 eq), and DIPEA (0.11 mmol, 1 eq) were dissolved in anhydrous THF (1 mL), and molecular sieves 4Å (10 mg) was added to the solution. After shaking for 1.5 h at room temperature, (polystyrylmethyl)trimethylammonium cyanoborohydride (4.3 mmol/g, 0.22 mmol) was added to the reaction mixture. After shaking for 8 h at room temperature, 4-benzyloxybenzaldehyde polystyrene (3 mmol/g, 0.22 mmol), 3-(4-(hydrazinosufonyl)phenyl)propionyl AM resin (1.5 mmol/g, 0.22 mmol), and N-(2-mercaptoethyl)aminomethyl polystyrene (2.1 mmol/g, 0.22 mmol) were added, after which the reaction mixture was shaken at room for 18 h. Filtration, washing with DCM, and evaporation of the solvent in vacuo afforded the crude product, which was purified by reversed phase HPLC.

**Method E: Condensation of amines with hydroxyl carboxylic acids**

1-Ethyl-3(3'-dimethylaminophropyl)carbodiimide hydrochloride (0.15 mmol, 1.36 eq), hydroxyl carboxylic acid (0.15 mmol, 1.36 eq), and 1-hydroxy-7-azabenzotriazole (0.15 mmol, 1.36 eq) were dissolved in DMF (0.7 mL). After shaking for 30 min at room temperature, a solution of amine (0.11 mmol) in DMF (0.7 mL) was added to the reaction mixture. After shaking for 2 h at room temperature, amine (0.22 mmol) was added, after which the reaction mixture was shaken at 60 °C overnight. Then the solvent was evaporated in vacuo, and the residue was dissolved in DCM (7 mL). HYDROMATRIX™ (0.3 g) which was previously treated with HCl (2N, 0.6 mL) was added to the solution, and the mixture was shaken for 30 min. Filtration, washing with DCM, and evaporation of the solvent in vacuo afforded the crude product, which was purified by reversed phase HPLC.

**Method F: Carbamate formation in solution**

Amine or amine salt (1 eq) and sodium bicarbonate (1 or 2 eq) were dissolved in a mixture of MeOH / $H_2O$ (3:1). The mixture was treated with chloroformate (1 eq), which was added in three portions over 10 minutes. After 30 minutes at room temperature, the precipitating product was collected by filtration and washed with water. The obtained crude carbamates were purified by HPLC.

**Method G: Carbamate formation in solution**

To an ice cooled mixture of amine or amine salt (1 eq) and DIPEA (1.1 eq or 2.2 eq) in dry DCM was added an ice cooled solution of chloroformate (1.1 eq) in DCM in one portion. After 1.5-8 h at room temperature the solvent was removed under reduced pressure. The obtained crude carbamates were purified by HPLC.

**Method H: Carbamate formation in solution**

To a mixture of amine or amine salt (1 eq) and sodium bicarbonate (1 or 2.5 eq) in dry DCM was added chloroformate (1 eq) in one portion. After 4 hours at room temperature the sodium bicarbonate was filtered off and the solvent was removed under reduced pressure. The obtained crude carbamates were purified by HPLC.

**Method I: Thiourea formation in solution**

Amine salt and DIPEA (1 eq each) or amine (1 eq) was dissolved in dry dioxan and a solution of the thioisocyanate (1 eq) in DCM or DMSO was added under Ar in one portion. The solution was stirred for 3 h at room temperature. The solution was diluted with 2 ml DCM and scavenger resins (tris-(2-aminoethyl)-amine polystyrene (3 eq), methylisocyanate polystyrene (3 eq) and N-(2-mercaptoethyl)aminomethyl polystyrene (3 eq)) were added to remove unreacted isocyanate, amine and electrophilic impurities respectively. After 18 h at 40°C, the solution was filtered off and the solvent was removed under reduced pressure. The obtained crude thioureas were purified by HPLC.

**Method J: Synthesis of (3-amino-5-chloro-4-hydroxy-phenyl)-ureas.**

Isocyanate (5.0 mmol) was added to a stirred solution of 4-Amino-2-chloro-phenol (5.0 mmol) in anhydrous $CH_2Cl_2$ (23 mL) and THF (4 mL) at room temperature. After stirring for 12 h, the solvent was evaporated in vacuo. The residue was then dissolved in HOAc (95 mL) and added in one single portion to a stirred solution of $NaNO_2$ (1.17 g, 17.0 mmol) in $H_2O$ (8.4 mL). The flask was sealed with a stopper and the reaction mixture was stirred for 1.5 min at room temperature. The reaction was stopped by the addition of saturated aqueous $NaHCO_3$ (190 mL). After stirring for 10 min at room temperature, the yellow precipitate was filtered, washed with $H_2O$ (3 x 30 mL), and dried in vacuo. The yellow residue was then dissolved in a mixture of toluene (75 mL) and MeOH (90 mL). Raney nickel (0.5 g) was washed with MeOH (5 x 10 mL) and added to the reaction mixture. Then the reaction mixture was vigorously stirred under a hydrogen atmosphere at 1 bar at room temperature for 2 h. Filtration through a pad of Celite and evaporation of the solvent afforded the aniline, which was converted into the corresponding diureas by method A, the corresponding anilines by method D, the corresponding sulfonamides by method B, the corresponding amides by method C or E, or the corresponding thioureas by method I.

**Method K: Synthesis of (5-benzothiazol-2-yl-3-chloro-2-hydroxy-phenyl)-ureas, (5-benzothiazol-2-yl-3-chloro-2-hydroxy-phenyl)-thioureas, (3-benzothiazol-2-yl-5-chloro-4-hydroxy-phenyl)-ureas, (3-benzothiazol-2-yl-5-chloro-4-hydroxy-phenyl)-thioureas, (5-benzothiazol-2-yl-3-chloro-2-hydroxy-phenyl)-amides, and (3-benzothiazol-2-yl-5-chloro-4-hydroxy-phenyl)-amides.**

3-Chloro-2-hydroxy-5-nitro-benzoic acid or 3-chloro-4-hydroxy-5-nitro-benzoic acid (0.41 g, 1.9 mmol) was dissolved in polyphosphoric acid (12.3 g) at 110 °C. 2-Aminothiophenol (0.36 mg, 2.9 mmol) was added and the resulting solution was stirred at 110 °C for 5 h. After cooling, ammonia (35% in $H_2O$, 12 mL) was added to the reaction mixture. The precipitate was filtered, washed with $H_2O$ (3 x 10 mL) and dried in vacuo. The residue was then dissolved in a mixture of MeOH (30 mL) and THF (70 mL). Raney nickel (0.5 g) was washed with MeOH (5 x 10 mL) and added to the reaction mixture. Then the reaction mixture was vigorously stirred under a hydrogen atmosphere at 1 bar at room temperature for 1 h. Filtration through a pad of Celite and evaporation of the solvent afforded the aniline, which was converted into the corresponding ureas by method A, the corresponding amides by method C or E, or the corresponding thioureas by method I.

Example 2: 1-Adamantan-1-yl-3-(3,5-dichloro-2-hydroxy-4-methyl-phenyl)-urea

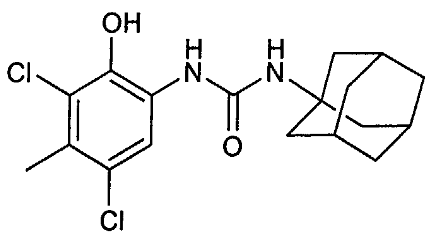

To a solution of 6-amino-2,4-dichloro-3-methyl-phenol hydrochloride (113.5 mg, 1 eq) and DIPEA (48 µL, 1 eq) in dioxan (1.1 mL) was added 1-adamantylisocyanate (88.5 mg, 1 eq) in 580 µL DMSO in one portion. The solution was stirred at room temperature for 3 h. The solution was diluted with 2 mL DCM and scavenger resins (tris-(2-aminoethyl)-amine polystyrene (3 eq), methylisocyanate polystyrene (3 eq) and *N*-(2-mercaptoethyl)aminomethyl polystyrene (3 eq)) were added. After 18 h at 40°C the solution was filtered off and the solvent was removed under reduced pressure. The crude product was purified by HPLC to obtain 118 mg (64 %) of the title compound as a white powder.
NMR-$^1$H (DMSO-$d_6$) $\delta$ = 1.62 ($s_b$, 6H), 1.92 ($s_b$, 6H), 2.05 ($s_b$, 3H), 2.29 (s, 3H), 6.79 (s, 1H), 7.95 (s, 1H), 8.15 (s, 1H), 9.82 (s, 1H).
NMR-$^{13}$C (DMSO-$d_6$) $\delta$ = 17.1, 29.3, 36.1, 41.5, 50.3, 116.3, 122.1, 123.8, 124.7, 129.9, 140.6, 154.3; MS (m/z): 369.2 [M+H$^+$].

Example 3: 1-(3,5-Dichloro-4-hydroxy-phenyl)-3-(1,1,3,3-tetramethyl-butyl)-urea

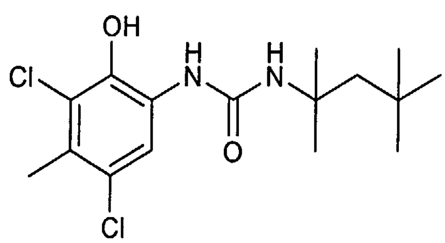

To a solution of 4-amino-2,6-dichloro-phenol (44.5 mg, 1 eq) in dioxan (2.0 mL) was added 2-isocyanato-2,4,4-trimethyl-pentane (38 mg, 1 eq) in one portion. The solution was stirred at room temperature for 3 h. The solution was diluted with 2 mL DCM and scavenger resins (tris-(2-aminoethyl)-amine polystyrene (3 eq), methylisocyanate polystyrene (3 eq) and N-(2-mercaptoethyl)aminomethyl polystyrene (3 eq)) were added. After 18 h at 35°C the solution was filtered off and the solvent was removed under reduced pressure. The crude material was purified by HPLC to obtain 67 mg (81 %) of the title compound as a white powder.
NMR-$^1$H (DMSO-$d_6$) $\delta$ = 0.95 (s, 9H), 1.28 (s, 6H), 1.69 (s, 2H), 5.90 (s, 1H), 7.33 (s, 2H), 8.22 (s, 1H), 9.50 ($s_b$, 1H).
NMR-$^{13}$C (DMSO-$d_6$) $\delta$ = 29.7, 31.2, 50.5, 53.2, 117.3, 1222.4, 134.0, 142.8, 153.9; MS (m/z): 333.2 [M+H$^+$].

Example 4: 2 *N*-(2-Hydroxy-4-methyl-phenyl)-C-phenyl-methanesulfonamide

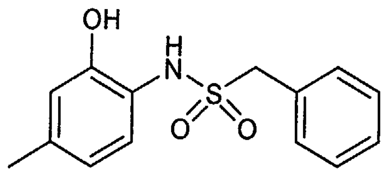

The compound was obtained in 32% yield (21.2 mg) using the protocol described in method A. NMR-$^1$H (DMSO-$d_6$) δ = 2.13 (s, 3H), 4.97 (s, 2H), 6.70 (d, 1H, *J*= 8.2 Hz), 6.78 (s, 1H), 6.83 (d, 1H, *J*= 8.2 Hz), 7.43 (m, 2H), 7.49 (m, 2H); MS (*m*/*z*): 278.1 [M$^+$].

Example 5: Propane-2-sulfonic acid (3,5-dichloro-2-hydroxy-4-methyl-phenyl)-amide

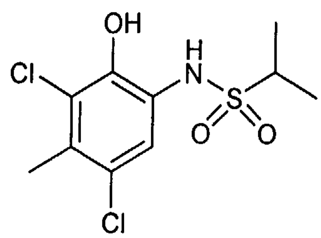

The compound was obtained in 47% yield (22.1 mg) using the protocol described in method B. NMR-$^1$H (DMSO-$d_6$) δ = 2.25 (d, 6H, *J*= 6.8), 2.27 (s, 3H), 3.97 (hep, 1H, *J*= 6.8), 5.39 (s, 1H), 6.91 (s, 1H); MS (*m*/*z*): 298.1 [MH$^+$].

Example 6: N-(3,5-Dichloro-4-hydroxy-phenyl)-2-phenyl-acetamide

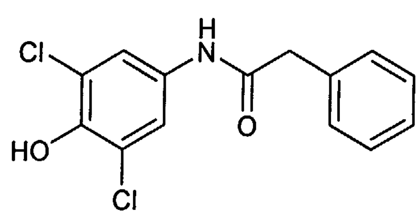

The compound was obtained in 22% yield (11.0 mg) using the protocol described in method C. NMR-$^1$H (DMSO-$d_6$) δ = 3.06 (s, 2H), 7.25 (m, 1H), 7.31 (m, 2H), 7.32 (m, 2H), 7.61 (m, 2H), 9.87 (m, 1H); MS (*m*/*z*): 296.2 [MH$^+$].

Example 7: N-(3,5-Dichloro-2-hydroxy-4-methyl-phenyl)-N-methyl-2-trifluoromethylbenzamide

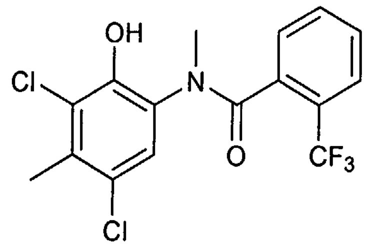

Aniline HCL salt (300 mg, 1.3 mmol) and $K_2CO_3$ (500 mg) were dissolved in DMSO (5 mL). $CH_3I$ (187 mg, 1.3 mmol) was added and the suspension was stirred for 48 h at room temperature. After filtration, the solvent was removed under reduced pressure. The crude reaction product was purified by preparative HPLC using acetonitrile and water as mobile phase to give the *N*-methyl amino phenol derivative (130 mg, 31 %).

The amide was obtained in 56% yield (19.7 mg) using the protocol described in method C.

NMR-$^1$H (DMSO-$d_6$) $\delta$ = 2.41 (s, 3H), 3.75 (s, 3H), 7.70-7.85 (m, 4H), 8.05 (d, 1H, *J*= 5.0); MS (*m*/*z*): 377.9 [$MH^+$].

Example 8: 2,4-Dichloro-3-methyl-6-(3-methyl-benzylamino)-phenol

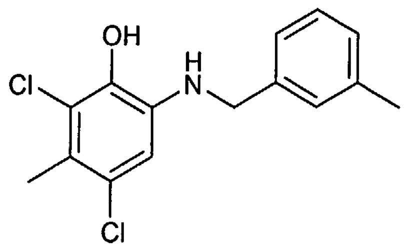

According to method D 6-amino-2,4-dichloro-3-methyl-phenol hydrochloride (25 mg, 0.11 mmol) and 3-methyl-benzaldehyde (13 mg, 0.11 mmol) gave 2,4-dichloro-3-methyl-6-(3-methyl-benzylamino)-phenol (16 mg, 49%). NMR-$^1$H (DMSO-$d_6$) $\delta$ 2.21 (s, 3 H), 2.27 (s, 3 H), 4.27 (s, 2 H), 5.80 (br s, 1 H), 6.35 (s, 1 H), 7.02-7.24 (m, 4 H), 9.18 (br s, 1 H); MS (*m*/*z*): 296.2 [$M+H^+$].

Example 9: *N*-Benzyl-4-bromo-3,5-dihydroxy-benzamide

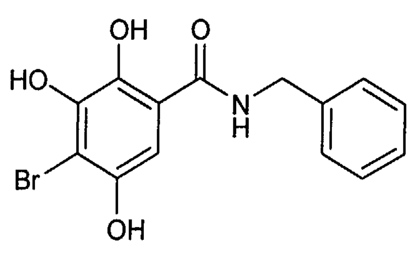

According to method **E** 4-bromo-3,5-dihydroxy-benzoic acid (35 mg, 0.15 mmol) and benzylamine (48 mg, 0.45 mmol) gave *N*-Benzyl-4-bromo-3,5-dihydroxy-benzamide (19 mg, 39%).

NMR-$^1$H (DMSO-$d_6$) $\delta$ 4.40 (d, *J* = 6.0 Hz, 2H), 6.86 (s, 2H), 7.20-7.36 (m, 5H), 8.91 (t, *J*= 6.2 Hz, 1H), 10.24 (s, 2H); MS (*m*/*z*): 322.1 [$M+H^+$].

Example 10: 2-(3-Chloro-4-hydroxy-phenyl)-1-piperidin-1-yl-ethanone

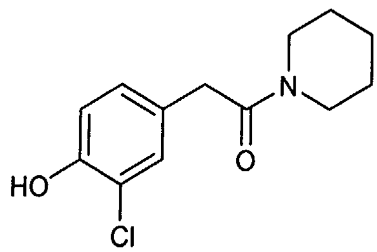

According to method E (3-chloro-4-hydroxy-phenyl)-acetic acid (28 mg, 0.15 mmol) and piperidine (38 mg, 0.45 mmol) gave 2-(3-chloro-4-hydroxy-phenyl)-1-piperidin-1-yl-ethanone (12 mg, 32%).
NMR-$^1$H (DMSO-$d_6$) δ 1.29-1.42 (m, 4 H), 1.48-1.59 (m, 2 H), 3.34-3.45 (m, 4 H), 3.57 (s, 2 H), 6.88 (d, *J* = 8.3 Hz, 1 H), 6.97 (dd, *J* = 8.3, 2.0 Hz, 1 H), 7.17 (d, *J* = 2.0 Hz, 1H), 10.00 (s, 1 H); MS (*m*/*z*): 254.3 [M+H$^+$].

Example 11: (3,5-Dichloro-4-hydroxy-phenyl)-carbamic acid phenyl ester

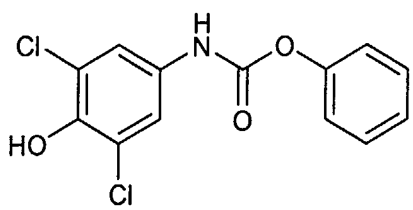

According to method F 4-amino-2,6-dichloro-phenol (67.5 mg, 1 eq) and phenylchloroformate (47 µL, 1 eq) gave 55.7 mg (49 %) of the title compound as a white solid.
NMR-$^1$H (DMSO-$d_6$) δ = 7.25 (m, 2H), 7.43 (m, 2 H), 7.50 (s, 2H)
NMR-$^{13}$C (DMSO-$d_6$) δ = 118.5, 121.9, 122.5, 125.5, 129.4, 131.7, 144.7, 150.3, 151.6

Example 12: (3,5-Dichloro-2-hydroxy-4-methyl-phenyl)-carbamic acid phenyl ester

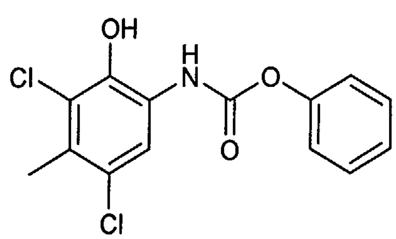

According to method F 6-amino-2,4-dichloro-3-methylphenol hydrochloride (57 mg, 1 eq) and phenylchloroformate (31.3 µl, 1 eq) gave 24.3 mg (31 %) of the title compound as a white solid.
NMR-$^1$H (DMSO-$d_6$) δ = 2.38 (s, 3 H), 6.76 (m, 3H), 7.15 (m, 3H)
NMR-$^{13}$C (DMSO-$d_6$) δ = 16.5, 109.1, 115.2, 118.8, 126.7, 128.5, 129.5, 129.7, 139.4, 153.4, 157.4

Example 13: (3,5-Dichloro-2-hydroxy-phenyl)-carbamic acid phenyl ester

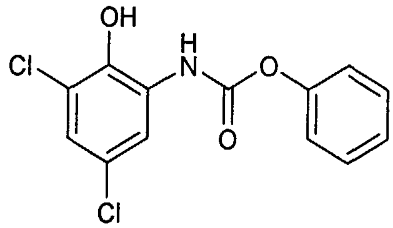

According to method F 2-amino-4,6-dichloro-phenol (25 mg, 1 eq) and phenylchloroformate (23 µL, 1 eq) gave 10.9 mg (26 %) of the title compound as a white solid.
NMR-$^1H$ (DMSO-$d_6$) δ = 7.43 (m, 2 H), 7.25 (m, 2H), 7.5 (s, 2H)
NMR-$^{13}C$ (DMSO-$d_6$) δ = 109.1, 118.8, 121.4, 128.2, 129.4, 132.6, 153.2, 157.3

Example 14: (3,5-Dichloro-2-hydroxy-4-methyl-phenyl)-carbamic acid 2-isopropyl-5-methyl-cyclohexyl ester

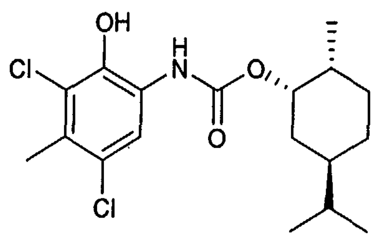

According to method G 6-amino-2,4-dichloro-3-methyl-phenol hydrochloride (35 mg, 1 eq) and (-)-menthylchloroformate (36.1 µL, 1.1 eq) in DCM gave 22 mg (39 %) of the title compound as a white solid.
NMR-$^1H$ (DMSO-$d_6$) δ = 0.84-0.90 (m, 3H), 1.22-1.41 (m, 6H), 2.35 (s, 3H), 3.28-3.33 (m, 2H), 4.06 (t, 2H), 7.57 (s, 1H), 8.74 (s, 1H).

Example 15: (3,5-Dichloro-4-hydroxy-phenyl)-carbamic acid 2-isopropyl-5-methyl-cyclohexyl ester

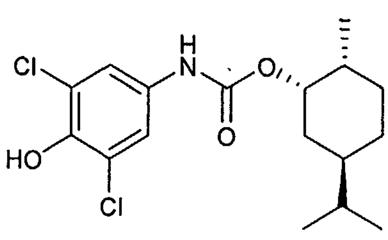

According to method G 4-amino-2,6-dichloro-phenol (40 mg, 1 eq) and (-)-menthylchloroformate (54 µl, 1.1 eq) in DCM gave 44.1 mg (54 %) of the title compound as a white solid.
NMR-$^1H$ (DMSO-$d_6$) δ = 0.75 (d, 3H), 0.82-093 (m, 7H), 0.96-1.14 (m, 2H), 1.29-1.53 (m, 2H), 1.59-1.71 (m, 2H), 1.88-2.02 (m, 2H), 4.54 (ddd, 1H), 7.47 (s, 2H), 9.67 (s, 1H), 9.73 (s, 1H)

Example 16: (3,5-Dichloro-2-hydroxy-4-methyl-phenyl)-carbamic acid hexyl ester

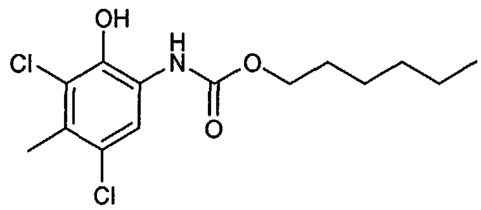

According to method G 6-amino-2,4-dichloro-3-methyl-phenol hydrochloride (35 mg, 1 eq) and hexylchloroformate (27.0 µL, 1.1 eq) gave 10.4 mg (21 %) of the title compound as a white solid.
NMR-$^1$H (DMSO-$d_6$) δ = 0.87 (t, 3H), 1.25-1.40 (m, 6H), 1.55-1.66 (m, 2H), 2.35 (s, 3H), 4.06 (t, 2H), 7.56 (s, 1H), 8.74 (s, 1H), 9.73 (s, 1H).

Example 17: (3,5-Dichloro-4-hydroxy-phenyl)-carbamic acid hexyl ester

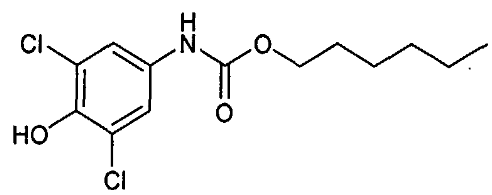

According to method G 4-amino-2,6-dichloro-phenol (40 mg, 1 eq) and hexylchloroformate (41 µL, 1.1 eq) gave 30 mg (43 %) of the title compound as a white solid.
NMR-$^1$H (DMSO-$d_6$) δ = 0.87 (t, 3H), 1.21-1.40 (m, 6H), 1.54-1.66 (m, 2H), 4.06 (t, 2H), 7.46 (s, 2H), 9.67 (s, 1H), 9.73 (s, 1H).

Example 18: 1-[3-Chloro-5-(3-cyclohexyl-ureido)-2-hydroxy-phenyl]-3-(2-trifluoromethyl-phenyl)-urea

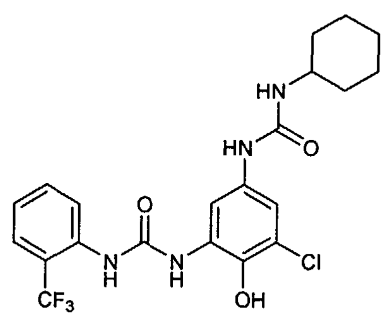

According to method J 1-(3-amino-5-chloro-4-hydroxy-phenyl)-3-cyclohexyl-urea (30 mg, 0.11 mmol) and 1-isocyanato-2-trifluoromethyl-benzene (21 mg, 0.11 mmol) gave 1-[3-chloro-5-(3-cyclohexyl-ureido)-2-hydroxy-phenyl]-3-(2-trifluoromethyl-phenyl)-urea (16 mg, 31%).
NMR-$^1$H (DMSO-$d_6$) δ 0.98-1.28 (m, 5 H), 1.39-1.76 (m, 5 H), 3.30-3.42 (m, 1 H), 5.84 (d, *J* = 7.8 Hz, 1 H), 7.24 (d, *J*= 7.8 Hz, 1 H), 7.31 (d, *J*= 2.4 Hz, 1 H), 7.52-7.67 (m, 2 H), 7.71-7.75 (m, 2 H), 8.20 (br. s, 1 H), 8.80-8.82 (m, 1 H), 8.97-9.04 (m, 1 H), 9.11 (s, 1 H); MS: *m*/*z*: 471.1 [M+H$^+$].

Example 19: *N*-[3-Chloro-5-(3-cyclohexyl-ureido)-2-hydroxy-phenyl]-2-nitro-benzenesulfonamide

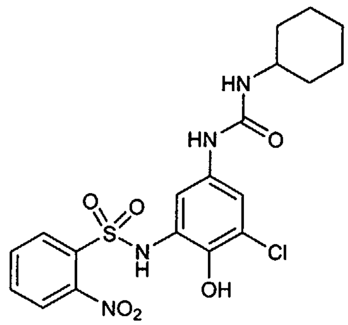

According to method J 1-(3-amino-5-chloro-4-hydroxy-phenyl)-3-cyclohexyl-urea (19 mg, 67 µmol) and 2-nitro-benzenesulfonyl chloride (15 mg, 67 µmol) gave *N*-[3-Chloro-5-(3-cyclohexyl-ureido)-2-hydroxy-phenyl]-2-nitro-benzenesulfonamide (15 mg, 48%).
NMR-$^1$H (DMSO-$d_6$) δ 1.05-1.37 (m, 5 H), 1.49-1.83 (m, 5 H), 3.42-3.47 (m, 1 H), 5.94 (d, *J* = 7.9 Hz, 1 H), 6.99 (d, *J* = 2.4 Hz, 1 H), 7.46 (d, *J* = 2.4 Hz, 1 H), 7.78-7.90 (m 2 H), 7.93-8.03 (m, 2 H), 8.27 (s, 1 H), 9.19 (br s, 1 H), 9.70 (s, 1 H); MS: *m*/*z*: 469.1 [M+H$^+$].

Example 20: 1-(3-Benzothiazol-2-yl-5-chloro-4-hydroxy-phenyl)-3-cyclohexyl-urea

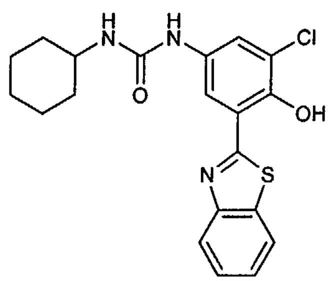

According to method K 4-amino-2-benzothiazol-2-yl-6-chloro-phenol (21 mg, 76 µmol) and isocyanato-cyclohexane (9.5 mg, 76 µmol) gave 1-(3-Benzothiazol-2-yl-5-chloro-4-hydroxy-phenyl)-3-cyclohexyl-urea (3.0 mg, 10%).
NMR-$^1$H (DMSO-$d_6$) δ 1.09-1.41 (m, 5 H), 1.49-1.87 (m, 5 H), 3.41-3.47 (m, 1H), 6.12 (d, *J* = 7.8 Hz, 1 H), 7.51 (t, *J* = 7.8 Hz, 1 H), 7.60 (t, *J* = 7.1 Hz, 1 H), 7.68 (d, *J* = 2.4 Hz, 1 H), 8.06 (d, *J* = 2.4 Hz, 1 H), 8.11 (d, *J* = 8.3 Hz, 1 H), 8.21 (d, *J* = 7.3 Hz, 1 H), 8.52 (s, 1 H), 11.92 (s, 1 H); MS: *m*/*z*: 402.2 [M+H$^+$].

Example 21: 1-(5-Benzothiazol-2-yl-3-chloro-2-hydroxy-phenyl)-3-(2-trifluoromethyl-phenyl)-thiourea

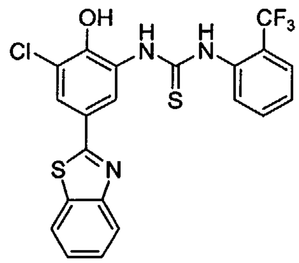

According to method K 2-Amino-4-benzothiazol-2-yl-6-chloro-phenol (20 mg, 72 µmol) and 1-isothiocyanato-2-trifluoromethyl-benzene (15 mg, 72 µmol) gave 1-(5-benzothiazol-2-yl-3-chloro-2-hydroxy-phenyl)-3-(2-trifluoromethyl-phenyl)-thiourea (12 mg, 35%).
NMR-$^1$H (DMSO-$d_6$) δ 7.40-7.56 (m, 3 H), 7.66-7.79 (m, 3 H), 7.92 (d, *J*= 2.4 Hz, 1 H), 8.03 (d, *J* = 7.8 Hz, 1 H), 8.12 (d, *J* = 8.8 Hz, 1 H), 8.68 (br s, 1 H), 9.65 (s, 1 H), 9.94 (s, 1 H), 10.65 (s, 1 H); MS: *m*/*z*: 480.0 [M+H$^+$].

Example 22: Specificity of inhibition of proteases

In order to investigate the impact of some of the inventive compounds on the activity of key proteases the following assay was performed: Protease activities were measured spectrophotometrically at 30°C according to Schomburg and Salzmann (Schomburg, B.; Salzmann M. GBF: Enzyme Handbook. Springer Verlag, Berlin Heidelberg, 1991) and Bergmeyer et al. (Bergmeyer, H. U.; Bergmeyer, J.; Graßl, M. Methods of Enzymatic Analysis, Vol. V Enzymes 3: Peptides, Proteinases and Their Inhibitors. pp 55 - 371, VCH, Weinheim, 1988). The release of 4-nitroaniline was determined at 390 nm with a Spectramax Plus UV/Vis spectrophotometer (Molecular Devices). The cathepsin B assay was performed in a reaction mixture containing 0.2 µg/ml cathepsin B, 2 mM Z-Arg-Arg-pNA in 88 mM $KH_2PO_4$, 12 mM $Na_2HPO_4$, 1.33 mM EDTA, 0.03% Brij 35 (pH 5.8). The trypsin assay was carried out in a reaction mixture containing 0.1 µg/ml trypsin and 120 µM Ac-Ala-Ala-Ser($PO_3H_2$) -Pro-Arg-pNA in 35 mM HEPES (pH 7.8) and the papain assay in a mixture consisting of 16 µg/ml papain and 2 mM Bz-DL-Arg-pNA in 10 mM $Na_2HPO_4$, 2 mM L-Cys, 5 mM EDTA (pH 6.5). In general, reactions were started by addition of peptide substrate after a 30 min incubation of 1-100 µM effector with given concentrations of enzyme.
The key proteases used were the following:

T-6: Papain
T-7: Trypsin
T-8: Cathepsin

In order to cluster the various compounds the following classes of activity were defined.

A: IC50 < 1 µM
B: 1 µM < IC50 < 10 µM
C: 10 µM < IC50 < 50 µM
D: 50 µM < IC50 < 100 µM
E: IC50 > 100 µM

Table 3

Specificity of the inhibition of some proteases

| Compound | N° | Target | | |
|---|---|---|---|---|
| | | T-6 | T-7 | T-8 |
| 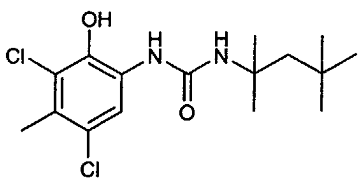 | 33 | D | C | D |
| 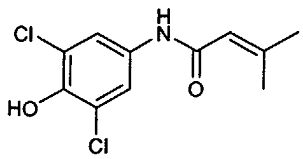 | 238 | E | E | E |

| Compound | N° | Target | | |
|---|---|---|---|---|
| | | T-6 | T-7 | T-8 |
| 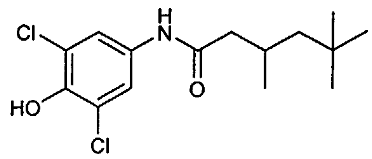 | 170 | E | E | E |
| 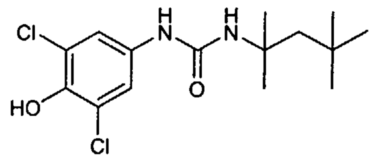 | 89 | E | D | E |
| 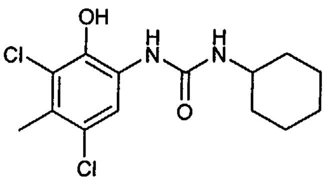 | 24 | E | | |
| 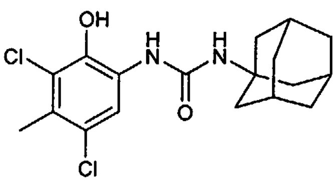 | 88 | E | | |
| 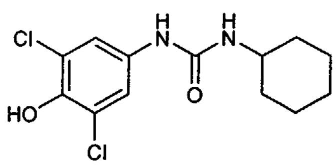 | 110 | E | | |
| 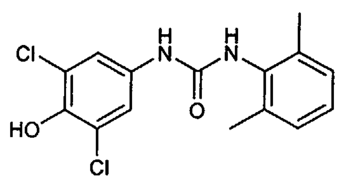 | 114 | E | | |

As may be taken from table 3 none of the tested compound is a strong inhibitor of any of the key proteases tested.

Example 23: Cytotoxic effects on tumor cell lines

In order to show that the compounds according to the present invention are actually useful in the treatment of tumors, the cytotoxic effects of some of said compounds on tumor cell lines were determined.

For this cytotoxic evaluation of the compounds the commercial available WST-1 assay (Roche) was used according to the manufacturer's instructions. The assay is based on the cleavage of the tetrazolium salt WST-1 by mitochondrial dehydrogenases found in viable cells. In general compounds were added to cells cultured in 96-well plates at 37°C. After 48 h of incubation 10 µl of WST-1 solution was added. The formazan dye was analyzed with an ELISA plate reader at (450 vs. 620) nm.

The following tumor cell lines were used in this assay:

CL-1: human acute myeloid leukemia, HL-60
CL-2: human cervix carcinoma, HeLa
CL-3: human prostate carcinoma, PC-3
CL-4: human colon adenocarcinoma, Caco-2
CL-5: human breast adenocarcinoma, MCF-7

In order to cluster the efficacy of the various compounds the following classes in terms of EC50 were defined.

A: EC50 < 10 μM
B: 10 μM < EC50 < 50 μM
C: 50 μM < EC50 < 100 μM
D: 100 μM < EC50 < 200 μM
E: EC50 > 200 μM

Table 4

Cytotoxic effects on tumor cell lines

| Compound | N° | Target | | | | |
|---|---|---|---|---|---|---|
| | | CL-1 | CL-2 | CL-3 | CL-4 | CL-5 |
| 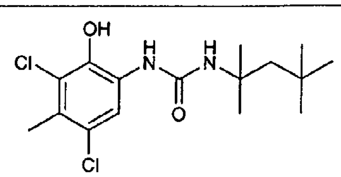 | 33 | A | A | A | A | |
| 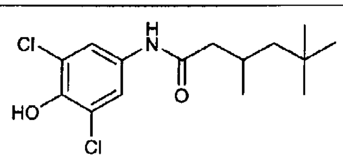 | 170 | B | C | B | B | |
| 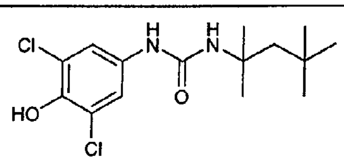 | 89 | B | B | B | B | |
| 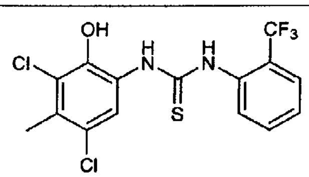 | 342 | A | B | B | B | |

As may be taken from table 4 all of the tested compounds are highly efficient in exhibiting a cytotoxic effect on at least one of the various tumor cell lines tested. Of particular relevance are compounds 33, 342.

<u>Example 24</u>: FACS measurements and TUNEL assay of Compound 89

In order to show that the compounds according to the present invention are actually useful for inducing apoptosis in tumor cells, FACS measurements and TUNEL assay were performed as, e. g., Enari M. Sakahira H. Yokoyama H. Okawa K. Iwamatsu A. Nagata S. A caspase-activated DNase that degrades DNA during apoptosis, and its inhibitor ICAD [erratum appears in Nature 1998 May 28;393(6683):396.]. *Nature. 391:43-50, 1998;* Darzynkiewicz Z. Juan G. Li X. Gorczyca W. Murakami T. Traganos F. Cytometry in cell necrobiology: analysis of apoptosis and accidental cell death (necrosis). *Cytometry. 27:1-20, 1997;* Apoptotic HL-60 cells were detected by FACS analysis of FITC-dUTP-labelled DNA breaks using the Apo-Direct kit (BD-Pharmingen) according to the manufacturer's protocol.

As may be taken from Fig. 1 compound 89 induce apoptosis in tumor cells.

The features of the present invention disclosed in the specification, the claims and/or the drawing may both separately and in any combination thereof be material for realizing the invention in various forms thereof.

## Claims

1. Use of a compound for the manufacture of a medicament for the treatment of a disease, whereby the disease involves an abnormal cell proliferation, an undesired cell proliferation, an abnormal mitosis and/or an undesired mitosis.
whereby the compound has the structure:

$$A\text{-}X\text{-}Y \qquad (I)$$

wherein A is cycloalkyl, substituted cycloalkyl, heterocyclyl, substituted heterocyclyl, aryl, substituted aryl, heteroaryl or substituted heteroaryl;
X is a spacer and is selected from $X_1$ or $X_2$,
wherein $X_1$ is selected from the group comprising
$-[(CR^aR^b)_n\text{-}NR^c\text{-}CO\text{-}NR^d\text{-}(CR^aR^b)_m]_t\text{-}$, $-[(CR^aR^b)_n\text{-}NR^c\text{-}CS\text{-}NR^d\text{-}(CR^aR^b)_m]_t\text{-}$, $-[(CR^aR^b)_n\text{-}NR^c\text{-}C(N\text{-}CN)\text{-}NR^d\text{-}(CR^aR^b)_m]_t\text{-}$, $-[(CR^aR^b)_n\text{-}NR^c\text{-}C(N\text{-}R^e)\text{-}NR^d\text{-}(CR^aR^b)_m]_t\text{-}$, $-[(CR^aR^b)_n\text{-}CO\text{-}NR^c\text{-}(CR^aR^b)_m]_t\text{-}$, $-[(CR^aR^b)_n\text{-}NR^c\text{-}(CR^aR^b)_m]_t\text{-}$, $-[(CR^aR^b)_n\text{-}NR^c\text{-}CO\text{-}O\text{-}(CR^aR^b)_m]_t\text{-}$, $-[(CR^aR^b)_n\text{-}O\text{-}CO\text{-}NR^c\text{-}(CR^aR^b)_m]_t\text{-}$, $-[(CR^aR^b)_n\text{-}O\text{-}(CR^aR^b)_m]_t\text{-}$, $-[(CR^aR^b)_n\text{-}SR^c\text{-}(CR^aR^b)_m]_t\text{-}$;
wherein $X_2$ is selected from the group comprising
$-[(CR^aR^b)_n\text{-}CO\text{-}(CR^aR^b)_m]_t\text{-}$, $-[(CR^aR^b)_n\text{-}CS\text{-}(CR^aR^b)_m]_t\text{-}$, $-[(CR^aR^b)_n\text{-}NR^c\text{-}CO\text{-}(CR^aR^b)m]_t\text{-}$, $-[(CR^aR^b)_n\text{-}CO\text{-}NR^c\text{-}(CR^aR^b)_m]_t\text{-}$, $-[(CR^aR^b)_n\text{-}NR^c\text{-}CS\text{-}(CR^aR^b)_m]_t\text{-}$, $-[(CR^aR^b)_n\text{-}CS\text{-}NR^c\text{-}(CR^aR^b)_m]_t\text{-}$, $-[(CR^aR^b)_n\text{-}NR^c\text{-}SO_2\text{-}(CR^aR^b)_m]_t\text{-}$, $-[(CR^aR^b)_n\text{-}SO_2\text{-}NR^c\text{-}(CR^aR^b)_m]_t\text{-}$, $-[(CR^aR^b)_n\text{-}SO\text{-}(CR^aR^b)_m]_t\text{-}$, $-[(CR^aR^b)_n\text{-}SO_2\text{-}(CR^aR^b)_m]_t\text{-}$, $-[(CR^aR^b)_n]_t\text{-}$;
wherein n and m are independently selected from each other and are any integer between 0 and 10 provided that if n is 0, m is different from 0, and if m is 0, n is different from 0;
wherein t is independently selected from n and/or m and is any integer between 0 and 10;
wherein
$R^a$, $R^b$, $R^c$, $R^d$ and $R^e$ are independently from each other selected from the group comprising H, alkyl, cycloalkyl, aryl, heteroaryl and heterocyclyl;
and wherein Y is selected from the group comprising alkyl, substituted alkyl, cycloalkyl, substituted cycloalkyl, cycloalkenyl, substituted cycloalkenyl, heterocyclyl, substituted heterocyclyl, mono-unsaturated heterocyclyl, poly-unsaturated heterocyclyl, mono-substituted poly-unsaturated heterocyclyl, poly-substituted poly-unsaturated heterocyclyl, aryl, substituted aryl, heteroaryl and substituted heteroaryl, wherein Y is different from a peptide.

2. The use according to claim 1, wherein A is

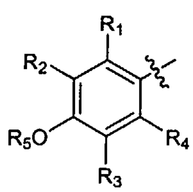

or

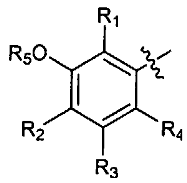

or

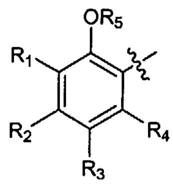

wherein $R_1$, $R_2$, $R_3$ and $R_4$ are each independently selected from the group comprising H, O, S, $NR^e$, halogen, alkyl, cycloalkyl, aryl, heterocyclyl and heteroaryl; and
$R_5$ is selected from selected from the group comprising H, alkyl, cycloalkyl, aryl, heterocyclyl and heteroaryl; and
$R^e$ is selected from the group comprising H, alkyl, aryl, alkoxy, aryloxy, alkylamino and arylamino.

**3.** The use according to claim 1 or 2, wherein
$R_1$, $R_2$, $R_3$, $R_4$ and/or $R_5$ have independently from each other one or more groups of the formula $R^f$; whereby $R^f$ is selected from the group comprising alkyl, cycloalkyl, aryl, heterocyclyl, heteroaryl, alkoxy, aryloxy, arylalkoxy, alkoxycarbonyl, aryloxycarbonyl, alkanoyl, aroyl, alkanoyloxy, aroyloxy, carbamoyl, alkanoylamino, aroylamino, alkylthio, arylthio, ureido and amine.

**4.** The use according to claim 3, wherein
the alkylthio group is derivatized, preferably the sulfur atom is oxidized to a sulfoxide or sulfone;
the arylthio group is derivatized, preferably the sulfur atom is oxidized to a sulfoxide or sulfone,
the ureido group is derivatized, preferably the nitrogen atom is independently mono- or di-substituted, more preferably the substitution is selected from the group comprising alkyl, aryl, heterocyclyl, heteroaryl, alkoxycarbonylamino, aryloxycarbonylamino, alkylcarbamoyloxy, arylcarbamoyloxy, alkylsulfonylamino, arylsulfonylamino, alkylaminosulfonyl, and arylaminosulfonyl; and/or
the amino group is derivatized, preferably the nitrogen atom is independently mono- or di-substituted by alkly, aryl, heterocyclyl, heteroalyl, halogen, hydroxy, oxo, carboxy, cyano, nitro, amidino and guanidino.

**5.** The use according to claim 3 or 4, whereby $R^f$ is further substituted by one ore more groups $R^g$, whereby $R^g$ is selected from the group comprising alkyl, cycloalkyl, aryl, arylalkyl, alkoxy, aryloxy, arylalkoxy, alkanoyl, aroyl, amino, halogen, hydroxy, oxo, carboxy, cyano, nitro, amidino and guanidino.

**6.** Use of a compound for the manufacture of a medicament for the treatment of a disease whereby the disease involves an abnormal cell proliferation, an undesired cell proliferation, an abnormal mitosis and/or an undesired mitosis, whereby the compound is selected from the group comprising
3-[3-(5-Chloro-2-hydroxy-phenyl)-ureido]-propionic acid ethyl ester
1-(5-Chloro-2-hydroxy-phenyl)-3-pentyl-urea
1-(5-Chloro-2-hydroxy-phenyl)-3-(3,5-dichloro-phenyl)-urea
1-(5-Chloro-2-hydroxy-phenyl)-3-(4-chloro-phenyl)-urea
1-(5-Chloro-2-hydroxy-phenyl)-3-cyclohexyl-urea
1-(5-Chloro-2-hydroxy-phenyl)-3-(4-trifluoromethoxy-phenyl)-urea
1-(5-Chloro-2-hydroxy-phenyl)-3-(4-cyano-phenyl)-urea
1-Benzo[1,3]dioxol-5-yl-3-(5-chloro-2-hydroxy-phenyl)-urea
1-Benzyl-3-(5-chloro-2-hydroxy-phenyl)-urea
1-(5-Chloro-2-hydroxy-phenyl)-3-0-tolyl-urea

1-(5-Chloro-2-hydroxy-phenyl)-3-(3-methoxy-phenyl)-urea
1-(5-Chloro-2-hydroxy-phenyl)-3-(2,6-dimethyl-phenyl)-urea
1-(5-Chloro-2-hydroxy-phenyl)-3-phenethyl-urea
1-(5-Chloro-2-hydroxy-phenyl)-3-(1,1,3,3-tetramethyl-butyl)-urea
1-(5-Chloro-2-hydroxy-phenyl)-3-(3,4,5-trimethoxy-phenyl)-urea
1-(5-Chloro-2-hydroxy-phenyl)-3-naphthalen-1-yl-urea
1-Adamantan-1-yl-3-(5-chloro-2-hydroxy-phenyl)-urea
1-(5-Chloro-2-hydroxy-phenyl)-3-(4-phenoxy-phenyl)-urea
1-(5-Chloro-2-hydroxy-phenyl)-3-phenyl-urea
3-[3-(3,5-Dichloro-2-hydroxy-4-methyl-phenyl)-ureido]-propionic acid ethyl ester
1-(3,5-Dichloro-2-hydroxy-4-methyl-phenyl)-3-pentyl-urea
1-(3,5-Dichloro-2-hydroxy-4-methyl-phenyl)-3-(3,5-dichloro-phenyl)-urea
1-(4-Chloro-phenyl)-3-(3,5-dichloro-2-hydroxy-4-methyl-phenyl)-urea
1-Cyclohexyl-3-(3,5-dichloro-2-hydroxy-4-methyl-phenyl)-urea
1-(3,5-Dichloro-2-hydroxy-4-methyl-phenyl)-3-(4-trifluoromethoxy-phenyl)-urea
1-(4-Cyano-phenyl)-3-(3, 5-dichloro-2-hydroxy-4-methyl-phenyl)-urea
1-Benzo[1,3]dioxol-5-yl-3-(3,5-dichloro-2-hydroxy-4-methyl-phenyl)-urea
1-Benzyl-3-(3,5-dichloro-2-hydroxy-4-methyl-phenyl)-urea
1-(3,5-Dichloro-2-hydroxy-4-methyl-phenyl)-3-o-tolyl-urea
1-(3,5-Dichloro-2-hydroxy-4-methyl-phenyl)-3-(3-methoxy-phenyl)-urea
1-(3,5-Dichloro-2-hydroxy-4-methyl-phenyl)-3-(2,6-dimethyl-phenyl)-urea
1-(3,5-Dichloro-2-hydroxy-4-methyl-phenyl)-3-phenethyl-urea
1-(3,5-Dichloro-2-hydroxy-4-methyl-phenyl)-3-(1,1,3,3-tetramethyl-butyl)-urea
1-(3,5-Dichloro-2-hydroxy-4-methyl-phenyl)-3-(3,4,5-trimethoxy-phenyl)-urea
1-(3,5-Dichloro-2-hydroxy-4-methyl-phenyl)-3-naphthalen-1-yl-urea
1-Adamantan-1-yl-3-(3,5-dichloro-2-hydroxy-4-methyl-phenyl)-urea
1-(3,5-Dichloro-2-hydroxy-4-methyl-phenyl)-3-(4-phenoxy-phenyl)-urea
1-(3,5-Dichloro-2-hydroxy-4-methyl-phenyl)-3-phenyl-urea
3-[3-(2-Hydroxy-4-methyl-phenyl)-ureido]-propionic acid ethyl ester
1-(2-Hydroxy-4-methyl-phenyl)-3-pentyl-urea
1-(3,5-Dichloro-phenyl)-3-(2-hydroxy-4-methyl-phenyl)-urea
1-(4-Chloro-phenyl)-3-(2-hydroxy-4-methyl-phenyl)-urea
1-Cyclohexyl-3-(2-hydroxy-4-methyl-phenyl)-urea
1-(2-Hydroxy-4-methyl-phenyl)-3-(4-trifluoromethoxy-phenyl)-urea
1-(4-Cyano-phenyl)-3-(2-hydroxy-4-methyl-phenyl)-urea
1-Benzo[1,3]dioxol-5-yl-3-(2-hydroxy-4-methyl-phenyl)-urea
1-Benzyl-3-(2-hydroxy-4-methyl-phenyl)-urea
1-(2-Hydroxy-4-methyl-phenyl)-3-o-tolyl-urea
1-(2-Hydroxy-4-methyl-phenyl)-3-(3-methoxy-phenyl)-urea
1-(2,6-Dimethyl-phenyl)-3-(2-hydroxy-4-methyl-phenyl)-urea
1-(2-Hydroxy-4-methyl-phenyl)-3-phenethyl-urea
1-(2-Hydroxy-4-methyl-phenyl)-3-(1,1,3,3-tetramethyl-butyl)-urea
1-(2-Hydroxy-4-methyl-phenyl)-3-(3,4,5-trimethoxy-phenyl)-urea
1-(2-Hydroxy-4-methyl-phenyl)-3-naphthalen-1-yl-urea
1-Adamantan-1-yl-3-(2-hydroxy-4-methyl-phenyl)-urea
1-(2-Hydroxy-4-methyl-phenyl)-3-(4-phenoxy-phenyl)-urea
1-(2-Hydroxy-4-methyl-phenyl)-3-phenyl-urea
N-(2-Hydroxy-phenyl)-C-phenyl-methanesulfonamide
N-(4-Hydroxy-phenyl)-C-phenyl-methanesulfonamide
N-(5-Chloro-2-hydroxy-phenyl)-C-phenyl-methanesulfonamide
N-(2-Hydroxy-4-methyl-phenyl)-C-phenyl-methanesulfonamide
N-(3,5-Dichloro-2-hydroxy-4-methyl-phenyl)-C-phenyl-methanesulfonamide
Butane-1-sulfonic acid (3,5-dichloro-2-hydroxy-4-methyl-phenyl)-amide
Octane-1-sulfonic acid (3,5-dichloro-2-hydroxy-4-methyl-phenyl)-amide
Propane-2-sulfonic acid (3,5-dichloro-2-hydroxy-4-methyl-phenyl)-amide
1-(3,5-Dichloro-2-hydroxy-phenyl)-3-(1,1,3,3-tetramethyl-butyl)-urea
1-(3-Chloro-2-hydroxy-phenyl)-3-(1,1,3,3-tetramethyl-butyl)-urea
1-Adamantan-1-yl-3-(3,5-dichloro-2-hydroxy-phenyl)-urea

1-Adamantan-1-yl-3-(3-chloro-2-hydroxy-phenyl)-urea
6-Benzylamino-2,4-dichloro-3-methyl-phenol
2,4-Dichloro-6-(2-chloro-6-fluoro-benzylamino)-3-methyl-phenol
2,4-Dichloro-3-methyl-6-(3-methyl-benzylamino)-phenol
2,4-Dichloro-6-(3,4-dimethoxy-benzylamino)-3-methyl-phenol
N-(3-Chloro-2-hydroxy-phenyl)-2-trifluoromethyl-benzenesulfonamide
4-Acetyl-N-(3-chloro-2-hydroxy-phenyl)-benzenesulfonamide
2,4-Dichloro-6-[2-(4-chloro-phenylsulfanyl)-benzylamino]-3-methyl-phenol
6-[(Biphenyl-2-ylmethyl)-amino]-2,4-dichloro-3-methyl-phenol
4-[(3,5-Dichloro-2-hydroxy-4-methyl-phenylamino)-methyl]-benzonitrile
4-[(3,5-Dichloro-2-hydroxy-4-methyl-phenylamino)-methyl]-benzoic acid methyl ester
6-[(5-Bromo-thiophen-2-ylmethyl)-amino]-2,4-dichloro-3-methyl-phenol
2,4-Dichloro-3-methyl-6-[(3-methyl-thiophen-2-ylmethyl)-amino]-phenol
2,4-Dichloro-3-methyl-6-[(5-methyl-furan-2-ylmethyl)-amino]-phenol
1-Adamantan-1-yl-3-(2,6-dibromo-3-chloro-4-methyl-phenyl)-urea
1-(2,6-Dibromo-3-chloro-4-methyl-phenyl)-3-(1,1,3,3-tetramethyl-butyl)-urea
1-Adamantan-1-yl-3-(3-chloro-2-cyano-phenyl)-urea
1-(3-Chloro-2-cyano-phenyl)-3-(1,1,3,3-tetramethyl-butyl)-urea
1-Adamantan-1-yl-3-(3-chloro-4-hydroxy-phenyl)-urea
1 -(3-Chloro-4-hydroxy-phenyl)-3-(1,1,3,3-tetramethyl-butyl)-urea
1-Adamantan-1-yl-3-(3,5-dichloro-4-hydroxy-phenyl)-urea
1-(3,5-Dichloro-4-hydroxy-phenyl)-3-(1,1,3,3-tetramethyl-butyl)-urea
1-Piperidin-1-yl-2-(2,4,6-tribromo-3-hydroxy-phenyl)-ethanone
2-(3-Chloro-4-hydroxy-phenyl)-1-piperidm-1-yl-ethanone
1-Pyrrolidin-1-yl-2-(2,4,6-tribromo-3-hydroxy-phenyl)-ethanone
2-(3-Chloro-4-hydroxy-phenyl)-1-pyrrolidin-1-yl-ethanone
N-(3,5-Dichloro-2-hydroxy-4-methyl-phenyl)-N-methyl-2-trifluoromethyl-benzamide
3,5,5-Trimethyl-hexanoic acid (3,5-dichloro-2-hydroxy-4-methyl-phenyl)-amide
2-Chloro-6-[3-(1,1,3,3-tetramethyl-butyl)-ureido]-benzoic acid
3-Adamantan-1-yl-1-(3, 5-dichloro-2-hydroxy-4-methyl-phenyl)-1-methyl-urea
1-(3,5-Dichloro-2-hydroxy-4-methyl-phenyl)-1-methyl-3-(1,1,3,3-tetramethyl-butyl)-urea
1-Adamantan-1-yl-3-(5-bromo-3-fluoro-2-hydroxy-phenyl)-urea
1-(5-Bromo-3-fluoro-2-hydroxy-phenyl)-3-(1,1,3,3-tetramethyl-butyl)-urea
1-(5-Bromo-3-fluoro-2-hydroxy-phenyl)-3-tert-butyl-urea
1-Adamantan-1-yl-3-(3,4-difluoro-2-hydroxy-phenyl)-urea
1-(3,4-Difluoro-2-hydroxy-phenyl)-3-(1,1,3,3-tetramethyl-butyl)-urea
1-tert-Butyl-3-(3,4-difluoro-2-hydroxy-phenyl)-urea
1-Adamantan-1-yl-3-(3-fluoro-4-hydroxy-phenyl)-urea
1-(3-Fluoro-4-hydroxy-phenyl)-3-(1,1,3,3-tetramethyl-butyl)-urea
1-tert-Butyl-3-(3-fluoro-4-hydroxy-phenyl)-urea
I -tert-Butyl-3-(3,5-dichloro-4-hydroxy-phenyl)-urea
1-(3,5-Dichloro-4-hydroxy-phenyl)-3-(2-phenyl-cyclopropyl)-urea
1-Cyclohexyl-3-(3,5-dichloro-4-hydroxy-phenyl)-urea
1-Cyclopentyl-3-(3,5-dichloro-4-hydroxy-phenyl)-urea
1-(3,5-Dichloro-4-hydroxy-phenyl)-3-pentyl-urea
1-(2-Chloro-6-trifluoromethyl-phenyl)-3-(3,5-dichloro-4-hydroxy-phenyl)-urea
1-(3,5-Dichloro-4-hydroxy-phenyl)-3-(2,6-dimethyl-phenyl)-urea
1-(3,5-Dichloro-4-hydroxy-phenyl)-3-phenethyl-urea
1-(3,5-Dichloro-4-hydroxy-phenyl)-3-naphthalen-2-yl-urea
1-Biphenyl-2-yl-3-(3,5-dichloro-4-hydroxy-phenyl)-urea
1-tert-Butyl-3-(3,5-dichloro-2-hydroxy-4-methyl-phenyl)-urea
1-tert-Butyl-3-(3,5-dichloro-2-hydroxy-phenyl)-urea
1-tert-Butyl-3-(3-chloro-4-hydroxy-phenyl)-urea
1-Adamantan-1-yl-3-(3,5-dibromo-4-hydroxy-phenyl)-urea
1-(3,5-Dibromo-4-hydroxy-phenyl)-3-(1,1,3,3-tetramethyl-butyl)-urea
1-tert-Butyl-3-(3,5-dibromo-4-hydroxy-phenyl)-urea
1-(3,5-Dibromo-4-hydroxy-phenyl)-3-(2-phenyl-cyclopropyl)-urea
1-(3,5-Dibromo-4-hydroxy-phenyl)-3-pentyl-urea

1-Cyclohexyl-3-(3,5-dibromo-4-hydroxy-phenyl)-urea
1-Adamantan-1-yl-3-(3-hydroxy-4-methoxy-phenyl)-urea
1-(3-Hydroxy-4-methoxy-phenyl)-3-(1,1,3,3-tetramethyl-butyl)-urea
1-tert-Butyl-3-(3-hydroxy-4-methoxy-phenyl)-urea
1-(3-Hydroxy-4-methoxy-phenyl)-3-(2-phenyl-cyclopropyl)-urea
1-(3-Hydroxy-4-methoxy-phenyl)-3-pentyl-urea
1-Cyclohexyl-3-(3-hydroxy-4-methoxy-phenyl)-urea
1-Adamantan-1-yl-3-(2,4-dihydroxy-phenyl)-urea
1-(2,4-Dihydroxy-phenyl)-3-( 1,1, 3,3-tetramethyl-butyl)-urea
1-tert-Butyl-3-(2,4-dihydroxy-phenyl)-urea
1-(2,4-Dihydroxy-phenyl)-3-(2-phenyl-cyclopropyl)-urea
1-(2,4-Dihydroxy-phenyl)-3-pentyl-urea
1-Cyclohexyl-3-(2,4-dihydroxy-phenyl)-urea
1-Adamantan-1-yl-3-(2'-hydroxy-[1,1';3',1"]terphenyl-5'-yl)-urea
1-(2'-Hydroxy-[1,1';3',1"]terphenyl-5'-yl)-3-(1,1,3,3-tetramethyl-butyl)-urea
1-tert-Butyl-3-(2'-hydroxy-[1,1';3',1"]terphenyl-5'-yl)-urea
1-(2'-Hydroxy-[1,1';3',1"]terphenyl-5'-yl)-3-(2-phenyl-cyclopropyl)-urea
1-(2'-Hydroxy-[1,1';3',1"]terphenyl-5'-yl)-3-pentyl-urea
1-Cyclohexyl-3-(2'-hydroxy-[1,1';3',1"]terphenyl-5'-yl)-urea
1-Adamantan-1-yl-3-(3-chloro-4-methoxy-phenyl)-urea
1-(3-Chloro-4-methoxy-phenyl)-3-(1,1,3,3-tetramethyl-butyl)-urea
1-tert-Butyl-3-(3-chloro-4-methoxy-phenyl)-urea
1-(3-Chloro-4-methoxy-phenyl)-3-(2-phenyl-cyclopropyl)-urea
1-(3-Chloro-4-methoxy-phenyl)-3-pentyl-urea
1-(3-Chloro-4-methoxy-phenyl)-3-cyclohexyl-urea
1-Adamantan-1-yl-3-(4-hydroxy-3-methoxy-benzyl)-urea
1-(4-Hydroxy-3-methoxy-benzyl)-3-(1,1,3,3-tetramethyl-butyl)-urea
1-tert-Butyl-3-(4-hydroxy-3-methoxy-benzyl)-urea
1-(4-Hydroxy-3-methoxy-benzyl)-3-(2-phenyl-cyclopropyl)-urea
1-(4-Hydroxy-3-methoxy-benzyl)-3-pentyl-urea
1-Cyclohexyl-3-(4-hydroxy-3-methoxy-benzyl)-urea
1-Adamantan-1-yl-3-(4-hydroxy-naphthalen-1-yl)-urea
1-(4-Hydroxy-naphthalen-1-yl)-3-(1,1,3,3-tetramethyl-butyl)-urea
1-tert-Butyl-3-(4-hydroxy-naphthalen-1-yl)-urea
1-(4-Hydroxy-naphthalen-1-yl)-3-(2-phenyl-cyclopropyl)-urea
1-(4-Hydroxy-naphthalen-1-yl)-3-pentyl-urea
1-Cyclohexyl-3-(4-hydroxy-naphthalen-1-yl)-urea
1-Adamantan-1-yl-3-[4-(2-hydroxy-ethyl)-phenyl]-urea
1-[4-(2-Hydroxy-ethyl)-phenyl]-3-(1,1,3,3-tetramethyl-butyl)-urea
1-tert-Butyl-3-[4-(2-hydroxy-ethyl)-phenyl]-urea
1-[4-(2-Hydroxy-ethyl)-phenyl]-3-(2-phenyl-cyclopropyl)-urea
1-[4-(2-Hydroxy-ethyl)-phenyl]-3-pentyl-urea
1-Cyclohexyl-3-[4-(2-hydroxy-ethyl)-phenyl]-urea
N-(3,5-Dichloro-4-hydroxy-phenyl)-2-phenyl-acetamide
3,5,5-Trimethyl-hexanoic acid (3,5-dichloro-4-hydroxy-phenyl)-amide
Octane-1-sulfonic acid (3,5-dichloro-4-hydroxy-phenyl)-amide
(3,5-Dichloro-2-hydroxy-4-methyl-phenyl)-carbamic acid 2-isopropyl-5-methyl-cyclohexyl ester
(3,5-Dichloro-2-hydroxy-4-methyl-phenyl)-carbamic acid hexyl ester
(3,5-Dichloro-4-hydroxy-phenyl)-carbamic acid phenyl ester
3-[3-(5-Chloro-2-hydroxy-phenyl)-ureido]-propionic acid ethyl ester
4-Bromo-3,5-dihydroxy-N-(1,1,3,3-tetramethyl-butyl)-benzamide
N-Benzyl-4-chloro-3-hydroxy-benzamide
N-Benzyl-3,5-dihydroxy-benzamide
2,6-Dichloro-4-(2-methyl-thiazol-4-yl)-phenol
2,6-Dichloro-4-(2-phenyl-thiazol-4-yl)-phenol
2,6-Dichloro-4-piperidin-1-ylmethyl-phenol
2,6-Dichloro-4-[2-(4-chloro-phenylsulfanyl)-benzylamino]-phenol
3,5-Dichloro-4-hydroxy-N-(1,1,3,3-tetramethyl-butyl)-benzamide

2-(3-Chloro-4-hydroxy-phenyl)-N-(1,1,3,3-tetramethyl-butyl)-acetamide
4-(2-Benzyl-thiazol-4-yl)-2,6-dichloro-phenol
N-Adamantan-1-yl-2-(3,5-dichloro-4-hydroxy-phenyl)-acetamide
2-(3,5-Dichloro-4-hydroxy-phenyl)-N-phenethyl-acetamide
N-Cyclohexyl-2-(3,5-dichloro-4-hydroxy-phenyl)-acetamide
2-(3,5-Dichloro-4-hydroxy-phenyl)-N-indan-1-yl-acetamide
2-(3,5-Dichloro-4-hydroxy-phenyl)-N-(2-hydroxy-2-phenyl-ethyl)-acetamide
2-(3,5-Dichloro-4-hydroxy-phenyl)-N-( 1,5-dimethyl-hexyl)-acetamide
2-(3,5-Dichloro-4-hydroxy-phenyl)-N-(3-methyl-butyl)-acetamide
2-(3,5-Dichloro-4-hydroxy-phenyl)-N-(3,3-diphenyl-propyl)-acetamide
1-[2-(3,5-Dichloro-4-hydroxy-phenyl)-acetyl]-3-(3-dimethylamino-propyl)-1-ethyl-urea
2-(3-Fluoro-4-hydroxy-phenyl)-N-(1,1,3,3-tetramethyl-butyl)-acetamide
2-(3-Fluoro-4-hydroxy-phenyl)-1-piperidin-1-yl-ethanone
2,6-Dichloro-4-(2-phenethyl-thiazol-4-yl)-phenol
2,6-Dichloro-4-[2-(2,2-dimethyl-propyl)-thiazol-4-yl]-phenol
2-(3,5-Dichloro-4-hydroxy-phenyl)-1-piperidin-1-yl-ethanone
2-(3,5-Dichloro-4-hydroxy-phenyl)-N-[2-(4-ftuoro-phenyl)-1,1-dimethyl-ethyl]-acetamide
2-Cyano-N-cyclohexyl-2-(3,5-dichloro-4-hydroxy-phenyl)-acetamide
2,6-Dichloro-4-[2-(2,4,4-trimethyl-pentyl)-thiazol-4-yl]-phenol
2,6-Dichloro-4-[2-(1-methyl-butyl)-thiazol-4-yl]-phenol
2,6-Dichloro-4-(2-cyclopentylmethyl-thiazol-4-yl)-phenol
2,6-Dichloro-4-[2-(2-cyclopentyl-ethyl)-thiazol-4-yl]-phenol
4-(2-tert-Butyl-thiazol-4-yl)-2,6-dichloro-phenol
2,6-Dichloro-4-(2-thiophen-2-ylmethyl-thiazol-4-yl)-phenol
2,6-Dichloro-4-(2-phenoxymethyl-thiazol-4-yl)-phenol
2-Cyano-2-(3,5-dichloro-4-hydroxy-phenyl)-N-phenethyl-acetamide
1-tert-Butyl-3-[3-chloro-2-hydroxy-5-(2-oxo-2-piperidin-1-yl-ethyl)-phenyl]-urea
2-[3-(3-tert-Butyl-ureido)-5-chloro-4-hydroxy-phenyl]-N-cyclohexyl-acetamide
2-[3-(3-tert-Butyl-ureido)-5-chloro-4-hydroxy-phenyl]-N-(1,1,3,3-tetramethyl-butyl)-acetamide
2-[3-(3-tert-Butyl-ureido)-5-chloro-4-hydroxy-phenyl]-N-phenethyl-acetamide
2-(3-Acetyl-2,2-dimethyl-cyclobutyl)-N-(3,5-dichloro-4-hydroxy-phenyl)-acetamide
2-Bicyclo[2.2.1]hept-2-yl-N-(3,5-dichloro-4-hydroxy-phenyl)-acetamide
2-Propyl-pentanoic acid (3,5-dichloro-4-hydroxy-phenyl)-amide
2-Cyano-N-(3,5-dichloro-4-hydroxy-phenyl)-acetamide
N-(3,5-Dichloro-4-hydroxy-phenyl)-2-(3-hydroxy-4,7,7-trimethyl-bicyclo[2.2.1]hept-2-yl)-acetamide
2,2-Dicyclohexyl-N-(3,5-dichloro-4-hydroxy-phenyl)-acetamide
4-[2,4-Bis-(1,1-dimethyl-propyl)-phenoxy]-N-(3,5-dichloro-4-hydroxy-phenyl)-butyramide
N-(3,5-Dichloro-4-hydroxy-phenyl)-3-phenyl-acrylamide
2-Methyl-pentanoic acid (3,5-dichloro-4-hydroxy-phenyl)-amide
N-(3,5-Dichloro-4-hydroxy-phenyl)-2-phenoxy-acetamide
N-(3,5-Dichloro-4-hydroxy-phenyl)-malonamic acid ethyl ester
N-(3,5-Dichloro-4-hydroxy-phenyl)-succinamic acid ethyl ester
N-(3,5-Dichloro-4-hydroxy-phenyl)-3-(3-trifluoromethyl-phenyl)-acrylamide
3-Cyclopentyl-N-(3,5-dichloro-4-hydroxy-phenyl)-propionamide
N-(3,5-Dichloro-4-hydroxy-phenyl)-2,2-diphenyl-acetamide
Acetic acid 1-(3,5-dichloro-4-hydroxy-phenylcarbamoyl)-ethyl ester
N-(3,5-Dichloro-4-hydroxy-phenyl)-2-(2-isopropyl-5-methyl-cyclohexyloxy)-acetamide
N-(3,5-Dichloro-4-hydroxy-phenyl)-3,3-dimethyl-butyramide
3,5,5-Trimethyl-hexanoic acid (3-chloro-4-hydroxy-phenyl)-amide
3,5,5-Trimethyl-hexanoic acid (5-bromo-3-fluoro-2-hydroxy-phenyl)-amide
3,5,5-Trimethyl-hexanoic acid (3,5-dibromo-4-hydroxy-phenyl)-amide
3,5,5-Trimethyl-hexanoic acid (2,4-dihydroxy-phenyl)-amide
3,5,5-Trimethyl-hexanoic acid 4-hydroxy-3-methoxy-benzylamide
Adamantane-1-carboxylic acid 4-hydroxy-3-methoxy-benzylamide
N-(4-Hydroxy-3-methoxy-benzyl)-2-trifluoromethyl-benzamide
N-(3,5-Dichloro-4-hydroxy-phenyl)-2-thiophen-2-yl-acetamide
N-(3,5-Dichloro-4-hydroxy-phenyl)-acetamide
2-Cyclopentyl-N-(3,5-dichloro-4-hydroxy-phenyl)-acetamide

3-Methyl-but-2-enoic acid (3,5-dichloro-4-hydroxy-phenyl)-amide
N-(3,5-Dichloro-4-hydroxy-phenyl)-2-phenyl-acetamide
N-(3,5-Dichloro-4-hydroxy-phenyl)-3 -phenyl-propionamide
3-Bromo-2-hydroxy-5-(3,5,5-trimethyl-hexanoylamino)-benzoic acid
4-Methyl-pentanoic acid (3,5-dichloro-4-hydroxy-phenyl)-amide
3,7-Dimethyl-oct-6-enoic acid (3,5-dichloro-4-hydroxy-phenyl)-amide
2-Adamantan-1-yl-N-(3,5-dichloro-4-hydroxy-phenyl)-acetamide
3-Cyclohexyl-N-(3,5-dichloro-4-hydroxy-phenyl)-propionamide
N-(3,5-Dichloro-4-hydroxy-phenyl)-2-(2-fluoro-phenyl)-acetamide
N-(3,5-Dichloro-4-hydroxy-phenyl)-3-hydroxy-3-phenyl-propionamide
N-[(3,5-Dichloro-4-hydroxy-phenylcarbamoyl)-methyl]-benzamide
3-(3,5-Dichloro-4-hydroxy-phenyl)-1,1-diethyl-urea
3-(3,5-Dichloro-4-hydroxy-phenyl)-1,1-diisopropyl-urea
1-(3,5-Dichloro-4-hydroxy-phenyl)-3-(3-hydroxy-propyl)-urea
1-(3,5-Dichloro-4-hydroxy-phenyl)-3-(2-morpholin-4-yl-ethyl)-urea
1-(3,5-Dichloro-4-hydroxy-phenyl)-3-(3-isopropoxy-propyl)-urea
1-(1-Aza-bicyclo[2.2.2]oct-3-yl)-3-(3,5-dichloro-4-hydroxy-phenyl)-urea
1-(3,5-Dichloro-4-hydroxy-phenyl)-3-(2-piperidin-1-yl-ethyl)-urea
1-Adamantan-2-yl-3-(3,5-dichloro-4-hydroxy-phenyl)-urea
1-Adamantan-1-yl-3-(4-hydroxy-3-morpholin-4-ylmethyl-phenyl)-urea
1-(4-Hydroxy-3-morpholin-4-ylmethyl-phenyl)-3-(1,1,3,3-tetramethyl-butyl)-urea
1-(4-Hydroxy-3-morpholin-4-ylmethyl-phenyl)-3-(2-trifluoromethyl-phenyl)-urea
1-(3,5-Dichloro-4-hydroxy-phenyl)-3-(2-hydroxy-2-phenyl-ethyl)-urea
1-(3,5-Dichloro-4-hydroxy-phenyl)-3-[2-hydroxy-1-hydroxymethyl-2-(4-nitro-phenyl)-ethyl]-urea
1-(3,5-Dichloro-4-hydroxy-phenyl)-3-[2-(3,4-dimethoxy-phenyl)-ethyl]-urea
2-[3-(3,5-Dichloro-4-hydroxy-phenyl)-ureido]-3-phenyl-propionamide
1-(3,5-Dichloro-4-hydroxy-phenyl)-3-[2-(4-hydroxy-phenyl)-ethyl]-urea
1-(3,5-Dichloro-4-hydroxy-phenyl)-3-indan-2-yl-urea
1-(3,5-Dichloro-4-hydroxy-phenyl)-3-(3-phenyl-propyl)-urea
1-(3,5-Dichloro-4-hydroxy-phenyl)-3-indan-1-yl-urea
1-(3,5-Dichloro-4-hydroxy-phenyl)-3-(2-pyridin-2-yl-ethyl)-urea
1-(3,5-Dichloro-4-hydroxy-phenyl)-3-(2-pyridin-3-yl-ethyl)-urea
2-[3-(3,5-Dichloro-4-hydroxy-phenyl)-ureido]-4-methyl-pentanoic acid ethyl ester
2-[3-(3,5-Dichloro-4-hydroxy-phenyl)-ureido]-3-phenyl-propionic acid ethyl ester
2-[3-(3,5-Dichloro-2-hydroxy-4-methyl-phenyl)-ureido]-4-methyl-pentanoic acid ethyl ester
2-[3-(3,5-Dichloro-2-hydroxy-4-methyl-phenyl)-ureido]-3-phenyl-propionic acid ethyl ester
1-Adamantan-1-yl-3-(3,5-difluoro-4-hydroxy-phenyl)-urea
1-(3,5-Difluoro-4-hydroxy-phenyl)-3-(1,1,3,3-tetramethyl-butyl)-urea
1-(3,5-Difluoro-4-hydroxy-phenyl)-3-(2-trifluoromethyl-phenyl)-urea
1-tert-Butyl-3-(3,5-difluoro-4-hydroxy-phenyl)-urea
1-Adamantan-1-yl-3-(2-hydroxy-phenyl)-urea
1-(2-Hydroxy-phenyl)-3-( 1,1,3,3-tetramethyl-butyl)-urea
1-(2-Hydroxy-phenyl)-3-(2-trifluoromethyl-phenyl)-urea
1-Adamantan-1-yl-3-(3-hydroxy-phenyl)-urea
1-(3-Hydroxy-phenyl)-3-(1,1,3,3-tetramethyl-butyl)-urea
1-(3-Hydroxy-phenyl)-3-(2-trifluoromethyl-phenyl)-urea
1-Adamantan-1-yl-3-(3-hydroxy-phenyl)-urea
1-(3-Hydroxy-phenyl)-3-( 1,1,3,3-tetramethyl-butyl)-urea
1-(3-Hydroxy-phenyl)-3-(2-trifluoromethyl-phenyl)-urea
1,3-Bis-(3,5-dichloro-4-hydroxy-phenyl)-urea
1,3-Di-adamantan-1-yl-urea
1,3-Bis-(1,1,3,3-tetramethyl-butyl)-urea
3-Adamantan-1-yl-1-(3,5-dibromo-4-hydroxy-benzyl)-1-(3,5-dichloro-2-hydroxy-4-methyl-phenyl)-urea
1-(3,5-Dibromo-4-hydroxy-benzyl)-1-(3,5-dichloro-2-hydroxy-4-methyl-phenyl)-3-(1,1,3,3-tetramethyl-butyl)-urea
1-(3,5-Dibromo-4-hydroxy-benzyl)-1-(3,5-dichloro-2-hydroxy-4-methyl-phenyl)-3-(2-trifluoromethyl-phenyl)-urea
3-tert-Butyl-1-(3,5-dibromo-4-hydroxy-benzyl)-1-(3,5-dichloro-2-hydroxy-4-methyl-phenyl)-urea
1-(3,5-Dichloro-4-hydroxy-phenyl)-3-(1,2,2-trimethyl-propyl)-urea
1-(3,5-Dichloro-4-hydroxy-phenyl)-3-[2-(4-fluoro-phenyl)-1,1-dimethyl-ethyl]-urea

1-tert-Butyl-3-[3-chloro-5-(3-cyclohexyl-ureido)-2-hydroxy-phenyl]-urea
1-[3-Chloro-5-(3-cyclohexyl-ureido)-2-hydroxy-phenyl]-3-(1,1,3,3-tetramethyl-butyl)-urea
1-[3-Chloro-5-(3-cyclohexyl-ureido)-2-hydroxy-phenyl]-3-(2-trifluoromethyl-phenyl)-urea
1-{3-Chloro-5-[2-(4-chloro-phenylsulfanyl)-benzylamino]-4-hydroxy-phenyl}-3-cyclohexyl-urea
1-{3-[(Biphenyl-2-ylmethyl)-amino]-5-chloro-4-hydroxy-phenyl}-3-cyclohexyl-urea
1-[3-Chloro-5-(2-chloro-6-fluoro-benzylamino)-4-hydroxy-phenyl]-3-cyclohexyl-urea
N-[3-Chloro-5-(3-cyclohexyl-ureido)-2-hydroxy-phenyl]-2-nitro-benzenesulfonamide
1-(3-Chloro-5-formyl-4-hydroxy-phenyl)-3-cyclohexyl-urea
1-tert-Butyl-3-[3-chloro-2-hydroxy-5-(3-phenethyl-ureido)-phenyl]-urea
1-(3-Benzothiazol-2-yl-5-chloro-4-hydroxy-phenyl)-3-phenethyl-urea
1-[3-Chloro-2-hydroxy-5-(3-phenethyl-ureido)-phenyl]-3-phenyl-urea
[3-Chloro-2-hydroxy-5-(3-phenethyl-ureido)-phenyl]-carbamic acid isobutyl ester
[3-Chloro-2-hydroxy-5-(3-phenethyl-ureido)-phenyl]-carbamic acid see-butyl ester
Cyclopropanecarboxylic acid [3-chloro-2-hydroxy-5-(3-phenethyl-ureido)-phenyl]-amide
Cyclobutanecarboxylic acid [3-chloro-2-hydroxy-5-(3-phenethyl-ureido)-phenyl]-amide
Cyclopentanecarboxylic acid [3-chloro-2-hydroxy-5-(3-phenethyl-ureido)-phenyl]-amide
Cyclohexanecarboxylic acid [3-chloro-2-hydroxy-5-(3-phenethyl-ureido)-phenyl]-amide
1-tert-Butyl-3-{3-chloro-2-hydroxy-5-[3-(1,1,3,3-tetramethyl-butyl)-ureido]-phenyl}-urea
1-(3-Benzothiazol-2-yl-5-chloro-4-hydroxy-phenyl)-3-(1,1,3,3-tetramethyl-butyl)-urea
1-{3-Chloro-2-hydroxy-5-[3-(1,1,3,3-tetramethyl-butyl)-ureido]-phenyl}-3-phenyl-urea
{3-Chloro-2-hydroxy-5-[3-(1,1,3,3-tetramethyl-butyl)-ureido]-phenyl}-carbamic acid isobutyl ester
{3-Chloro-2-hydroxy-5-[3-(1,1,3,3-tetramethyl-butyl)-ureido]-phenyl}-carbamic acid sec-butyl ester
Cyclopropanecarboxylic acid {3-chloro-2-hydroxy-5-[3-(1,1,3,3-tetramethyl-butyl)-ureido]-phenyl}-amide
Cyclobutanecarboxylic acid {3-chloro-2-hydroxy-5-[3-(1,1,3,3-tetramethyl-butyl)-ureido]-phenyl}-amide
Cyclopentanecarboxylic acid {3-chloro-2-hydroxy-5-[3-(1,1,3,3-tetramethyl-butyl)-ureido]-phenyl}-amide
Cyclohexanecarboxylic acid {3-chloro-2-hydroxy-5-[3-(1,1,3,3-tetramethyl-butyl)-ureido]-phenyl}-amide
N-[3-(3-tert-Butyl-ureido)-5-chloro-4-hydroxy-phenyl]-3-phenyl-propionamide
N-(3-Benzothiazol-2-yl-5-chloro-4-hydroxy-phenyl)-3-phenyl-propionamide
N-[3-Chloro-4-hydroxy-5-(3-phenyl-ureido)-phenyl]-3-phenyl-propionamide
[3-Chloro-2-hydroxy-5-(3-phenyl-propionylamino)-phenyl]-carbamic acid isobutyl ester
[3-Chloro-2-hydroxy-5-(3-phenyl-propionylamino)-phenyl]-carbamic acid sec-butyl ester
Cyclopropanecarboxylic acid [3-chloro-2-hydroxy-5-(3-phenyl-propionylamino)-phenyl]-amide
Cyclobutanecarboxylic acid [3-chloro-2-hydroxy-5-(3-phenyl-propionylamino)-phenyl]-amide
Cyclopentanecarboxylic acid [3-chloro-2-hydroxy-5-(3-phenyl-propionylamino)-phenyl]-amide
Cyclohexanecarboxylic acid [3-chloro-2-hydroxy-5-(3-phenyl-propionylamino)-phenyl]-amide
1-(3,5-Dichloro-2-hydroxy-4-methyl-phenyl)-3-phenyl-thiourea
1-(3,5-Dichloro-4-hydroxy-phenyl)-3-phenyl-thiourea
1-tert-Butyl-3-(3,5-dichloro-2-hydroxy-4-methyl-phenyl)-thiourea
1-Cyclohexyl-3-(3,5-dichloro-2-hydroxy-4-methyl-phenyl)-thiourea
1-Cyclopentyl-3-(3,5-dichloro-2-hydroxy-4-methyl-phenyl)-thiourea
1-Cyclohexyl-3-(3,5-dichloro-4-hydroxy-phenyl)-thiourea
1-Cyclopentyl-3-(3,5-dichloro-4-hydroxy-phenyl)-thiourea
1-Benzyl-3-(3,5-dichloro-2-hydroxy-4-methyl-phenyl)-thiourea
1-Benzyl-3-(3,5-dichloro-4-hydroxy-phenyl)-thiourea
1-(3,5-Dichloro-2-hydroxy-4-methyl-phenyl)-3-(2-triftuoromethyl-phenyl)-thiourea
1-(3,5-Dichloro-2-hydroxy-phenyl)-3-(2-trifluoromethyl-phenyl)-thiourea
1-(3,5-Dichloro-4-hydroxy-phenyl)-3-(2-trifluoromethyl-phenyl)-thiourea
1-(3,5-Dichloro-2-hydroxy-4-methyl-phenyl)-3-(4-trifluoromethyl-phenyl)-thiourea
1-(3,5-Dichloro-4-hydroxy-phenyl)-3-(4-trifluoromethyl-phenyl)-thiourea
1-tert-Butyl-3-[3-chloro-5-(3-cyclohexyl-ureido)-2-hydroxy-phenyl]-urea
1-[3-Chloro-5-(3-cyclohexyl-ureido)-2-hydroxy-phenyl]-3-(1,1,3,3-tetramethyl-butyl)-urea
1-[3-Chloro-5-(3-cyclohexyl-ureido)-2-hydroxy-phenyl]-3-(2-trifluoromethyl-phenyl)-urea
1-{3-Chloro-5-[2-(4-chloro-phenylsulfanyl)-benzylamino]-4-hydroxy-phenyl}-3-cyclohexyl-urea
1-{3-[(Biphenyl-2-ylmethyl)-amino]-5-chloro-4-hydroxy-phenyl} -3-cyclohexyl-urea
1-[3-Chloro-5-(2-chloro-6-fluoro-benzylamino)-4-hydroxy-phenyl]-3-cyclohexyl-urea
N-[3-Chloro-5-(3-cyclohexyl-ureido)-2-hydroxy-phenyl]-2-nitro-benzenesulfonamide
N-[3-Chloro-5-(3-cyclohexyl-ureido)-2-hydroxy-phenyl]-2,4,6-trimethyl-benzenesulfonamide
N-[3-Chloro-5-(3-cyclohexyl-ureido)-2-hydroxy-phenyl]-2-trifluoromethyl-benzenesulfonamide

Ethanesulfonic acid [3-chloro-5-(3-cyclohexyl-ureido)-2-hydroxy-phenyl]-amide
N-[3-Chloro-5-(3-cyclohexyl-ureido)-2-hydroxy-phenyl]-3,3-dimethyl-butyramide
1-(3-Benzothiazol-2-yl-5-chloro-4-hydroxy-phenyl)-3-cyclohexyl-urea
1-(3-Benzothiazol-2-yl-5-chloro-4-hydroxy-phenyl)-3-(1,1,3,3-tetramethyl-butyl)-urea
1-(3-Benzothiazol-2-yl-5-chloro-4-hydroxy-phenyl)-3-phenethyl-urea
1- {3-Chloro-4-hydroxy-5-[3-(2-trifluoromethyl-phenyl)-thioureido]-phenyl}-3-cyclohexyl-urea
1-(5-Benzothiazol-2-yl-3-chloro-2-hydroxy-phenyl)-3-tert-butyl-urea
1-(5-Benzothiazol-2-yl-3-chloro-2-hydroxy-phenyl)-3-cyclopentyl-urea
1-(5-Benzothiazol-2-yl-3-chloro-2-hydroxy-phenyl)-3-phenyl-urea
1-(5-Benzothiazol-2-yl-3-chloro-2-hydroxy-phenyl)-3-benzyl-urea
1-(5-Benzothiazol-2-yl-3-chloro-2-hydroxy-phenyl)-3-phenethyl-urea
1-(5-Benzothiazol-2-yl-3-chloro-2-hydroxy-phenyl)-3-(2-trifluoromethyl-phenyl)-urea
1-(5-Benzothiazol-2-yl-3-chloro-2-hydroxy-phenyl)-3-(4-benzyloxy-phenyl)-urea
1-(5-Benzothiazol-2-yl-3-chloro-2-hydroxy-phenyl)-3-(2-trifluoromethyl-phenyl)-thiourea
1-(5-Benzothiazol-2-yl-3-chloro-2-hydroxy-phenyl)-3-isopropyl-thiourea
1-(5-Benzothiazol-2-yl-3-chloro-2-hydroxy-phenyl)-3-tert-butyl-thiourea
1-(5-Benzothiazol-2-yl-3-chloro-2-hydroxy-phenyl)-3-cyclopentyl-thiourea
3,5,5-Trimethyl-hexanoic acid (5-benzothiazol-2-yl-3-chloro-2-hydroxy-phenyl)-amide
N-(5-Benzothiazol-2-yl-3-chloro-2-hydroxy-phenyl)-3-phenyl-propionamide
3,5,5-Trimethyl-hexanoic acid (3-benzothiazol-2-yl-5-chloro-4-hydroxy-phenyl)-amid
N-(3-Benzothiazol-2-yl-5-chloro-4-hydroxy-phenyl)-3-phenyl-propionamide
N-(3,5-Dichloro-4-hydroxy-phenyl)-2-(2-nitro-phenyl)-acetamide
1-Cyclopentyl-3-(3,5-dichloro-2-hydroxy-4-methyl-phenyl)-urea
2-Benzo[1,3]dioxol-5-yl-N-(3,5-dichloro-4-hydroxy-phenyl)-acetamide
or a pharmaceutical acceptable salt or prodrug thereof.

**7.** The use according to any of claims 1 to 6, wherein the compound is acting on an enzymatic activity involved in the regulation of cell division and/or cell cycle or part thereof, preferably the part of the cell cycle is mitosis.

**8.** The use according to any of claims 1 to 7, wherein the disease is selected from the group comprising neurodegenerative diseases, stroke, inflammatory diseases, immune based disorders, infectious diseases, heart diseases, cardiovascular diseases and cell proliferative diseases.

**9.** The use according to claim 8, wherein the neurodegenerative disease is selected from the group comprising Alzheimer's disease, Huntington's disease, Parkinson's disease, peripheral neuropathy, progressive supranuclear palsy, corticobasal degeneration, frontotemporal dementia, synucleinopathies, multiple system atrophy, amyotrophic lateral atrophy, prion diseases and motor neuron diseases.

**10.** The use according to claim 8, wherein the infectious disease is selected from the group comprising fungal, viral, bacterial and parasite infection.

**11.** The use according to claim 10, wherein the fungal infection is selected from the group comprising gynaecological and dermatological infection.

**12.** The use according to claim 10, wherein the fungal infection is caused by *Histoplasma, Coccidioides, Cryptococcus, Blastomyces, Paracoccidioides, Aspergillus, Sporothrix, Rhizopus, Absidia, Mucor, Hormodendrum, Phialophora* Microsporum, Epidermophyton, *Rhinosporidum or by a yeast, preferably Candida or Cryptococcus.*

**13.** The use according to claim 10, wherein the fungal infection causes a disorder selected from the group comprising ringworm, candidiasis, coccidioidomycosis, blastomycosis, aspergillosis, cryptococcosis, histioplasmosis, paracoccidiomycosis, zygomycosis, sporotrichiosis, mycotic keratitis, nail hair and skin disease, lobomycosis, chromoblastomycosis, mycetoma.

**14.** The use according to claim 10, wherein the bacterial infection is selected from the group comprising infections caused by Gram-positive and by Gram-negative bacteria.

**15.** The use according to claim 14, wherein the bacterial infection is caused by *Staphylococcus, Clostridium, Streptococcus, Listeria, Salmonella, Bacillus, Escherichia, Mycobacteria, Serratia, Enterobacter, Enterococcus,* Nocardia,

*Hemophilus, Neisseria, Proteus, Yersinia, Helicobacter or Legionella..*

**16.** The use according to claim 10, wherein the bacterial infection causes a disorder selected from the group comprising pneumonia, diarrhea, dysentery, anthrax, rheumatic fever, toxic shock syndrome, mastoiditis, meningitis, gonorrhea, typhoid fever, brucellis, Lyme disease, gastroenteritis, tuberculosis, cholera, tetanus and bubonic plague.

**17.** The use according to claim 10, wherein the viral infection is selected from the group comprising infections caused by retrovirus, HIV, Papilloma virus, Polio virus, Epstein-Barr, Herpes virus, Hepatitis virus, Papova virus, Influenza virus, Rabies, JC, encephalitis causing virus or hemorrhagic fever causing virus.

**18.** The use according to claim 10, wherein the parasite infection is selected from the group comprising infections caused by *Trypanosoma, Leishmania, Trichinella, Echinococcus, Nematodes, Classes Cestoda Trematoda, Monogenea, Toxoplasma, Giardia, Balantidium, Paramecium, Plasmodium, or Entamoeba.*

**19.** The use according to claim 8, wherein the cell proliferative disorder is selected from the group comprising neoplastic and non-neoplastic disorders.

**20.** The use according to claim 19, wherein the neoplastic cell proliferative disorder is selected from the group comprising solid tumor, lymphoma and leukemia.

**21.** The use according to claim 20, wherein the solid tumor is selected from the group comprising carcinoma, sarcoma, osteoma, fibrosarcoma, and chondrosarcoma.

**22.** The use according to claim 19, wherein the neoplastic cell proliferative disorder is selected from the group comprising breast cancer, prostate cancer, colon cancer, brain cancer, lung cancer, pancreatic cancer, gastric cancer, bladder cancer and kidney cancer.

**23.** The use according to claim 19, wherein the non-neoplastic cell proliferative disorder is a fibrotic disorder, preferably the fibrotic disorder is fibrosis.

**24.** The use according to claim 19, wherein the non-neoplastic cell proliferative disorder is selected from the group comprising prostatic hypertrophy, endometriosis, psoriasis, tissue repair and wound healing.

**25.** The use according to claim 8, wherein the immune based/inflammatory disease is an autoimmune disease or disorder.

**26.** The use according to claim 8, wherein the immune based/inflammatory disease is selected from the group comprising rheumatoid arthritis, glomerulonephritis, systemic lupus erythematosus associated glomerulonephritis, irritable bowel syndrome, bronchial asthma, multiple sclerosis, pemphigus, pemphigoid, scleroderma, myasthenia gravis, autoimmune haemolytic and thrombocytopenic states, Goodpasture's syndrome, pulmonary hemorrhage, vasculitis, Crohn's disease and dermatomyositis.

**27.** The use according to claim 8, wherein the immune based and/or inflammatory disease is an inflammatory condition.

**28.** The use according to claim 11, wherein the immune based and/or inflammatory disease is selected from the group comprising inflammation associated with bums, lung injury, myocardial infarction, coronary thrombosis, vascular occlusion, post-surgical vascular reocclusion, artherosclerosis, traumatic central nervous system injury, ischemic heart disease and ischemia-reperfusion injury, acute respiratory distress syndrome, systemic inflammatory response syndrome, multiple organ dysfunction syndrome, tissue graft rejection and hyperacute rejection of transplanted organs.

**29.** The use according to any of claims 1 to 28, wherein the medicament is for administration via an administration route which is selected from the group comprising oral, subcutaneous, intravenous, intranasal, transdermal, intraperitoneal, intramuscular, intrapulmonar, vaginal, rectal, and intraocular administration.

**30.** The use according to any of claims 1 to 29, wherein the medicament is for the administration to a mammal, preferably to a human being.

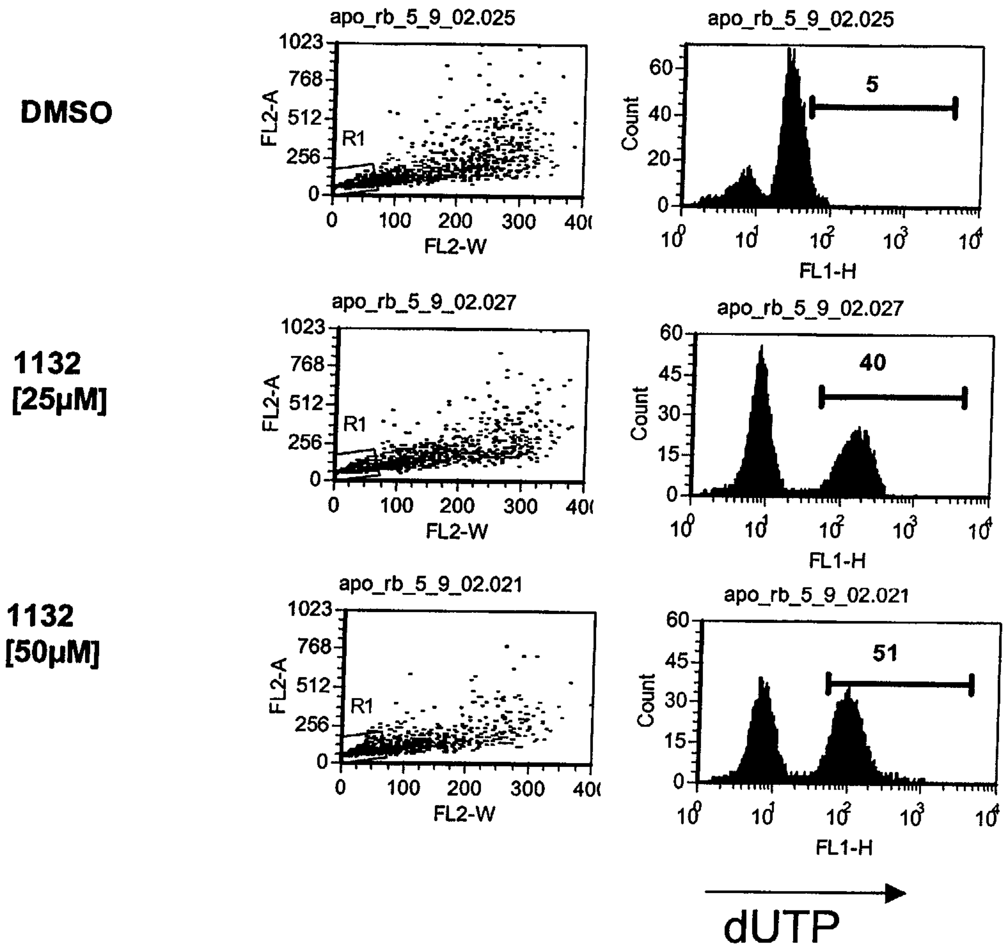
DMSO
1132
[25µM]
1132
[50µM]
apo_rb_5_9_02.025
apo_rb_5_9_02.027
apo_rb_5_9_02.021
FL2-A
FL2-W
R1
Count
FL1-H
5
40
51
dUTP

Fig. 1

European Patent Office

## PARTIAL EUROPEAN SEARCH REPORT

which under Rule 45 of the European Patent Convention shall be considered, for the purposes of subsequent proceedings, as the European search report

Application Number: EP 02 02 0922

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 96 25157 A (HERTZBERG ROBERT PHILIP ;JUREWICZ ANTHONY JOSEPH (US); RUTLEDGE ME) 22 August 1996 (1996-08-22)<br>* abstract *<br>* claims 1-32 *<br>--- | 8-30 | A61K31/17<br>A61P43/00 |
| A | WO 97 40028 A (VERTEX PHARMA) 30 October 1997 (1997-10-30)<br>* abstract *<br>* claims 1-53 *<br>--- | 8-30 | |
| A | WO 00 34269 A (AMERICAN HOME PROD) 15 June 2000 (2000-06-15)<br>* abstract *<br>* claims 1-28 *<br>--- | 8-30 | |
| A | US 5 593 993 A (NOREEN ROLF ET AL) 14 January 1997 (1997-01-14)<br>* abstract *<br>* claims 1-9 *<br>---<br>-/-- | 8-30 | |

TECHNICAL FIELDS SEARCHED (Int.Cl.7)

A61K

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 17 February 2003 | Taylor, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

## INCOMPLETE SEARCH SHEET C

Application Number
EP 02 02 0922

Claim(s) searched incompletely:
8-30

Claim(s) not searched:
1-7

Reason for the limitation of the search:

Present claims 1-6 relate to an extremely large number of possible compounds. Support within the meaning of Art. 84 EPC and/or disclosure within the meaning of Art. 83 EPC is to be found, however, for only a very small proportion of the compounds claimed. In the present case, the claims so lack support, and the application so lacks disclosure, that a meaningful search over the whole of the claimed scope is impossible.

Moreover, a therapeutical effect has only been shown for an even smaller number of compounds, namely those in Tables 3 and 4. Even amongst these compounds, it is noted that only a sub-set have (limited) in vitro activity.

Consequently, the search has been carried out for those parts of the application which do appear to be clear (and concise), namely those compounds as disclosed in Tables 3 and 4.

Present claims 1 and 7 relate to a disease defined by reference to a desirable characteristic or property, namely

"... whereby the disease involves an abnormal cell proliferation ... and/or undesired mitosis" (claim 1); and
"... wherein the compound is acting on ... cell cycle is mitosis" (claim 7).

The claims covers all diseases acting my these mechanisms, whereas the application provides support within the meaning of Art. 84 EPC and/or disclosure within the meaning of Art. 83 EPC for only a very limited number of such diseases. As there is no test provided to distinguish whether any given disease acts by these mechanisms or not, the claim so lacks support, and the application so lacks disclosure, that a meaningful search of this claim is impossible.

Independent of the above reasoning, the claim also lacks clarity (Art. 84 EPC). An attempt is made to define the medical use by reference to a result to be achieved. Again, this lack of clarity in the present case is such as to render a meaningful search over the whole of the claimed scope impossible.

European Patent Office

# PARTIAL EUROPEAN SEARCH REPORT

Application Number
EP 02 02 0922

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| A | US 5 886 044 A (JUREWICZ ANTHONY JOSEPH ET AL) 23 March 1999 (1999-03-23)<br>* abstract *<br>* claims 1-20 *<br>--- | 8-30 | |
| A | US 5 780 483 A (HERTZBERG ROBERT PHILIP ET AL) 14 July 1998 (1998-07-14)<br>* abstract *<br>* claims 1-12 *<br>----- | 8-30 | |
| | | | TECHNICAL FIELDS SEARCHED (Int.Cl.7) |

EPO FORM 1503 03.82 (P04C10)

European Patent Office

Application Number
EP 02 02 0922

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

8 (part), 9, 29 (part), 30 (part)

European Patent Office

## LACK OF UNITY OF INVENTION SHEET B

Application Number
EP 02 02 0922

The Search Division considers that the present European patent application does not comply with the requirements of unity of invention and relates to several inventions or groups of inventions, namely:

1. Claims: 8(part), 9, 29(part), 30(part)

   The use of the compounds in Tables 3 and 4 for the treatment of neurodegenerative diseases including stroke.

2. Claims: 8(part), 25-28 (all part), 29(part), 30(part)

   The use of the compounds in Tables 3 and 4 for the treatment of inflammatory diseases.

3. Claims: 8(part), 25-28 (all part), 29(part), 30(part)

   The use of the compounds in Tables 3 and 4 for the treatment of immune-based disorders.

4. Claims: 8(part), 10-18, 29(part), 30(part)

   The use of the compounds in Tables 3 and 4 for the treatment of infectious diseases.

5. Claims: 8(part), 29(part), 30(part)

   The use of the compounds in Tables 3 and 4 for the treatment of heart diseases and cardiovascular diseases.

6. Claims: 8(part), 19-24, 29(part), 30(part)

   The use of the compounds in Tables 3 and 4 for the treatment of cell proliferative diseases.

## ANNEX TO THE EUROPEAN SEARCH REPORT ON EUROPEAN PATENT APPLICATION NO. EP 02 02 0922

This annex lists the patent family members relating to the patent documents cited in the above-mentioned European search report.
The members are as contained in the European Patent Office EDP file on
The European Patent Office is in no way liable for these particulars which are merely given for the purpose of information.

17-02-2003

| Patent document cited in search report | Publication date | Patent family member(s) | Publication date |
|---|---|---|---|
| WO 9625157 A | 22-08-1996 | EP 0809492 A1 | 03-12-1997 |
| | | JP 11503110 T | 23-03-1999 |
| | | NO 983737 A | 14-10-1998 |
| | | WO 9625157 A1 | 22-08-1996 |
| | | US 6180675 B1 | 30-01-2001 |
| | | US 5886044 A | 23-03-1999 |
| | | US 5780483 A | 14-07-1998 |
| WO 9740028 A | 30-10-1997 | US 5807876 A | 15-09-1998 |
| | | US 6344465 B1 | 05-02-2002 |
| | | US 6054472 A | 25-04-2000 |
| | | AP 813 A | 28-02-2000 |
| | | AU 723730 B2 | 07-09-2000 |
| | | AU 2678597 A | 12-11-1997 |
| | | BG 102945 A | 31-08-1999 |
| | | BR 9708735 A | 03-08-1999 |
| | | CN 1219929 A | 16-06-1999 |
| | | CZ 9803380 A3 | 17-02-1999 |
| | | EP 0902782 A1 | 24-03-1999 |
| | | HU 0004421 A2 | 28-04-2001 |
| | | JP 2001509132 T | 10-07-2001 |
| | | KR 2000010580 A | 15-02-2000 |
| | | NO 984917 A | 23-12-1998 |
| | | NZ 332405 A | 23-06-2000 |
| | | PL 329639 A1 | 12-04-1999 |
| | | SK 146198 A3 | 12-07-1999 |
| | | TR 9802136 T2 | 21-06-2001 |
| | | WO 9740028 A1 | 30-10-1997 |
| | | US 6541496 B1 | 01-04-2003 |
| | | ZA 9703397 A | 14-11-1997 |
| WO 0034269 A | 15-06-2000 | US 6166028 A | 26-12-2000 |
| | | AU 3112200 A | 26-06-2000 |
| | | BG 105580 A | 31-01-2002 |
| | | BR 9916042 A | 04-12-2001 |
| | | CA 2351390 A1 | 15-06-2000 |
| | | CN 1335843 T | 13-02-2002 |
| | | CZ 20011958 A3 | 17-10-2001 |
| | | EP 1140913 A1 | 10-10-2001 |
| | | HU 0104758 A2 | 29-04-2002 |
| | | JP 2002531558 T | 24-09-2002 |
| | | NO 20012836 A | 08-08-2001 |
| | | PL 348177 A1 | 06-05-2002 |
| | | SK 7692001 A3 | 10-09-2002 |
| | | TR 200101598 T2 | 22-10-2001 |
| | | WO 0034269 A1 | 15-06-2000 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 02 02 0922

This annex lists the patent family members relating to the patent documents cited in the above-mentioned European search report.
The members are as contained in the European Patent Office EDP file on
The European Patent Office is in no way liable for these particulars which are merely given for the purpose of information.

17-02-2003

| Patent document cited in search report | Publication date | Patent family member(s) | Publication date |
|---|---|---|---|
| WO 0034269 A | | US 6201013 B1 | 13-03-2001 |
| | | US 6262082 B1 | 17-07-2001 |
| | | US 6403617 B1 | 11-06-2002 |
| | | US 6271236 B1 | 07-08-2001 |
| | | US 6407123 B1 | 18-06-2002 |
| | | US 6426355 B1 | 30-07-2002 |
| | | US 6407249 B1 | 18-06-2002 |
| | | US 6410571 B1 | 25-06-2002 |
| US 5593993 A | 14-01-1997 | US 5714503 A | 03-02-1998 |
| | | US 5658907 A | 19-08-1997 |
| | | AP 384 A | 15-05-1995 |
| | | AP 388 A | 15-05-1995 |
| | | AT 232095 T | 15-02-2003 |
| | | AU 657978 B2 | 30-03-1995 |
| | | AU 2045192 A | 11-03-1993 |
| | | AU 2407492 A | 02-03-1993 |
| | | BG 98623 A | 31-05-1995 |
| | | CA 2075173 A1 | 03-02-1993 |
| | | CN 1069882 A | 17-03-1993 |
| | | CZ 9202288 A3 | 12-05-1993 |
| | | DE 69232919 D1 | 13-03-2003 |
| | | EP 0540143 A2 | 05-05-1993 |
| | | ES 2051641 A1 | 16-06-1994 |
| | | FI 923443 A | 03-02-1993 |
| | | HU 9500395 A3 | 28-09-1995 |
| | | IL 102548 A | 16-08-1998 |
| | | JP 3383328 B2 | 04-03-2003 |
| | | JP 5320138 A | 03-12-1993 |
| | | KR 252453 B1 | 01-07-2000 |
| | | MX 9204454 A1 | 30-06-1994 |
| | | NO 922949 A | 03-02-1993 |
| | | NO 931337 A | 03-02-1993 |
| | | NO 931338 A | 03-02-1993 |
| | | NO 931339 A | 03-02-1993 |
| | | NO 931340 A | 03-02-1993 |
| | | NZ 260293 A | 26-05-1997 |
| | | OA 9914 A | 15-08-1994 |
| | | WO 9303022 A1 | 18-02-1993 |
| | | RU 2106341 C1 | 10-03-1998 |
| | | ZA 9205663 A | 28-01-1994 |
| US 5886044 A | 23-03-1999 | US 6211373 B1 | 03-04-2001 |
| | | US 6262113 B1 | 17-07-2001 |
| | | US 6180675 B1 | 30-01-2001 |
| | | US 5780483 A | 14-07-1998 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 02 02 0922

This annex lists the patent family members relating to the patent documents cited in the above-mentioned European search report.
The members are as contained in the European Patent Office EDP file on
The European Patent Office is in no way liable for these particulars which are merely given for the purpose of information.

17-02-2003

| Patent document cited in search report | Publication date | Patent family member(s) | Publication date |
|---|---|---|---|
| US 5886044 A | | EP 0809492 A1 | 03-12-1997 |
| | | JP 11503110 T | 23-03-1999 |
| | | NO 983737 A | 14-10-1998 |
| | | WO 9625157 A1 | 22-08-1996 |
| US 5780483 A | 14-07-1998 | US 5886044 A | 23-03-1999 |
| | | US 6211373 B1 | 03-04-2001 |
| | | US 6262113 B1 | 17-07-2001 |
| | | US 6180675 B1 | 30-01-2001 |
| | | EP 0809492 A1 | 03-12-1997 |
| | | JP 11503110 T | 23-03-1999 |
| | | NO 983737 A | 14-10-1998 |
| | | WO 9625157 A1 | 22-08-1996 |

EPO FORM P0459

For more details about this annex : see Official Journal of the European Patent Office, No. 12/82